# NOUVEAU FASCICULE DE BREVET EUROPEEN

(11) **EP 1 047 388 B2**
(45) Date de publication et mention de la décision concernant l'opposition: **19.08.2015**
(45) Mention de la délivrance du brevet: 22.09.2004
(21) Numéro de dépôt: 99934801.4
(22) Date de dépôt: 28.07.1999
(51) Int. Cl.: A61K 8/40, A61K 8/46, A61K 8/49

(54) **COMPOSITION DE TEINTURE POUR FIBRES KERATINIQUES AVEC UN COLORANT DIRECT CATIONIQUE ET UN AGENT TENSIO-ACTIF ANIONIQUE**
FÄRBEMITTEL FÜR KERATINFASERN, DAS EIN KATIONISCHER FARBSTOFFE UND EIN ANIONISCHES TENSID ENTHÄLT
DYEING COMPOSITION FOR KERATINOUS FIBRES WITH A DIRECT CATIONIC COLOURING AGENT AND AN ANIONIC SURFACTANT

(30) Priorité: 19.08.1998 FR 9810546
(43) Date de publication de la demande: 02.11.2000
(73) Titulaire: L'Oréal, 75008 Paris (FR)
(72) Inventeur: LANG, Gérard, F-95390 Saint Prix (FR); COTTERET, Jean, F-78480 Veneuil sur Seine (FR)
(74) Mandataire: Miszputen, Laurent
(86) Numéro de dépôt international: PCT/FR1999/001866
(87) Numéro de publication internationale: WO 2000/010518

(56) Documents cités:
- EP-A- 0 531 943
- EP-A- 0 850 637
- EP-A- 0 850 638
- WO-A-95/01772
- WO-A-95/15144
- WO-A-99/20234
- WO-A-99/20235
- DE-A1- 4 421 031
- DE-U- 29 504 690
- FR-A- 2 140 205
- FR-A- 2 189 006
- FR-A- 2 282 860
- FR-A- 2 285 851

## Description

L'invention concerne une composition de teinture pour fibres kératiniques, en particulier pour fibres kératiniques humaines telles que les cheveux, comprenant, dans un milieu approprié pour la teinture, au moins un colorant direct cationique de formule donnée, et au moins un agent tensio-actif anionique particulier.

L'invention a également pour objets les procédés et dispositifs de teinture mettant en oeuvre ladite composition.

Dans le domaine capillaire, on peut distinguer deux types de coloration.

Le premier est la coloration semi-permanente ou temporaire, ou coloration directe, qui fait appel à des colorants capables d'apporter à la coloration naturelle des cheveux, une modification de couleur plus ou moins marquée résistant éventuellement à plusieurs shampooings. Ces colorants sont appelés colorants directs; ils peuvent être mis en oeuvre avec ou sans agent oxydant. En présence d'oxydant, le but est d'obtenir une coloration éclaircissante. La coloration éclaircissante est mise en oeuvre en appliquant sur les cheveux le mélange extemporané d'un colorant direct et d'un oxydant et permet notamment d'obtenir, par éclaircissement de la mélanine des cheveux, un effet avantageux tel qu'une couleur unie dans le cas des cheveux gris ou de faire ressortir la couleur dans le cas de cheveux naturellement pigmentés.
Le deuxième est la coloration permanente ou coloration d'oxydation Celle-ci est réalisée avec des colorants dits "d'oxydation" comprenant les précurseurs de coloration d'oxydation et les coupleurs. Les précurseurs de coloration d'oxydation, appelés couramment "bases d'oxydation", sont des composés initialement incolores ou faiblement colorés qui développent leur pouvoir tinctorial au sein du cheveu en présence d'agents oxydants ajoutés au moment de l'emploi, en conduisant à la formation de composés colorés et colorants. La formation de ces composés colorés et colorants résulte, soit d'une condensation oxydative des "bases d'oxydation" sur elles-mêmes, soit d'une condensation oxydative des "bases d'oxydation" sur des composés modificateurs de coloration appelés couramment "coupleurs" et généralement présents dans les compositions tinctoriales utilisées en teinture d'oxydation.
Pour varier les nuances obtenues avec lesdits colorants d'oxydation, ou les enrichir de reflets, II arrive qu'on leur ajoute des colorants directs.

Parmi les colorants directs cationiques disponibles dans le domaine de la teinture des fibres kératiniques notamment humaines, on connaît déjà les composés dont la structure est développée dans le texte qui va suivre; néanmoins, ces colorants conduisent à des colorations qui présentent des caractéristiques encore insuffisantes sur le plan de la puissance, de l'homogénéité de la couleur répartie le long de la fibre, on dit alors que la coloration est trop sélective, et sur le plan de la tenacité, en terme de résistance aux diverses agressions que peuvent subir les cheveux (lumière, intempéries, shampooings). EP-A-0 850 638 et EP-A-0 850 637 décrivent des compositions comprenant un colorant direct cationique et au moins un agent oxydant. Un agent tensio-actif amphotère est utilisé dans les exemples.

Or, apres d'importantes recherches menées sur la question, la demanderesse vient maintenant de découvrir qu'il est possible d'obtenir de nouvelles compositions pour la teinture des fibres kératiniques capables de conduire à des colorations puissantes et peu sélectives et résistant bien néanmoins aux diverses agressions que peuvent subir les cheveux, en associant au moins un agent tensio-actif anionique particulier à au moins un colorant direct cationique connu de l'art antérieur et de formules respectivement définies ci-après.

Cette découverte est à la base de la présente invention.

La présente invention a donc pour premier objet une composition pour la teinture des fibres kératiniques et en particulier des fibres kératiniques humaines telles que les cheveux, renfermant dans un milieu approprié pour la teinture, (i)au moins un colorant direct cationique dont la structure répond aux formules (I) à (IV) définies ci-après, caractérisée par le fait qu'elle contient en outre (ii)au moins un agent tensio-actif anionique particulier.

Les colorants directs cationiques de formules (I), (II), (III) et (III') utilisables dans les compositions tinctoriales conformes à l'invention, sont des composés connus et sont décrits par exemple dans les demandes de brevets WO 95/01772, WO 95/15144 et EP-A-0 714 954. Ceux de formule (IV) utilisables dans les compositions tinctoriales conformes à l'invention, sont des composés connus et sont décrits par exemple dans les demandes de brevets FR-2189006, FR-2285851 et FR-2140205 et ses certificats d'addition.

Parmi les colorants directs cationiques de formule (I) utilisables dans les compositions tinctoriales conformes à l'invention, on peut plus particulièrement citer les composés répondant aux structures (I1) à (I54) suivantes : 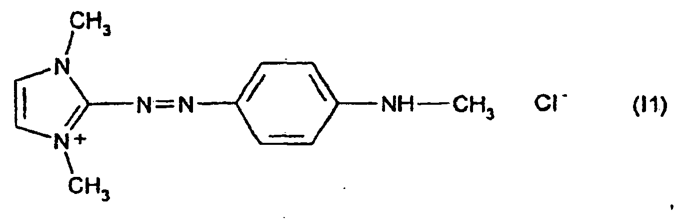 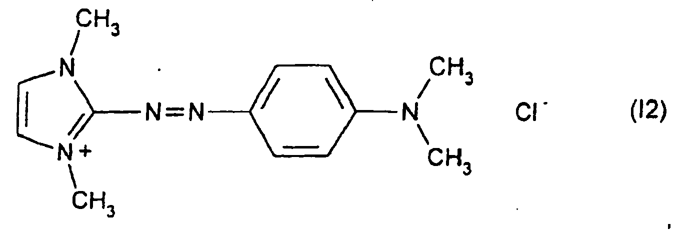 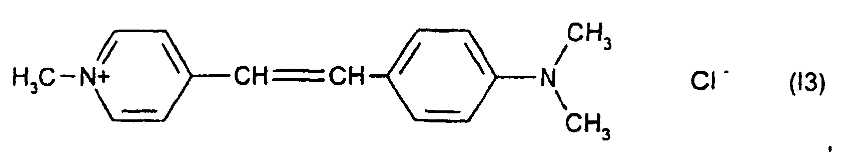 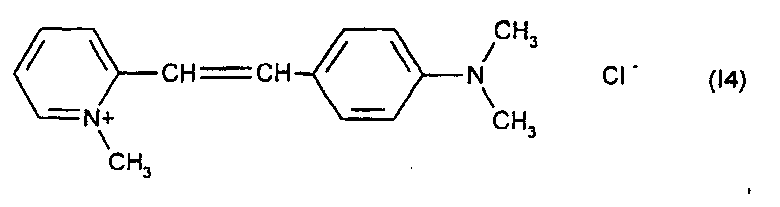 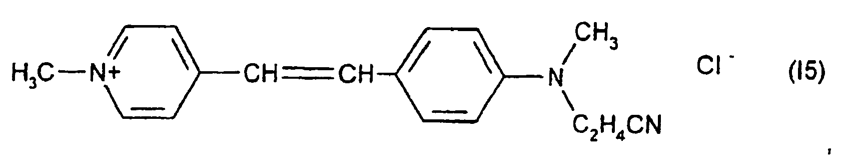 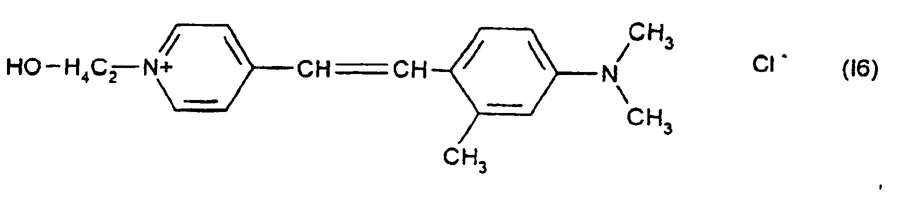 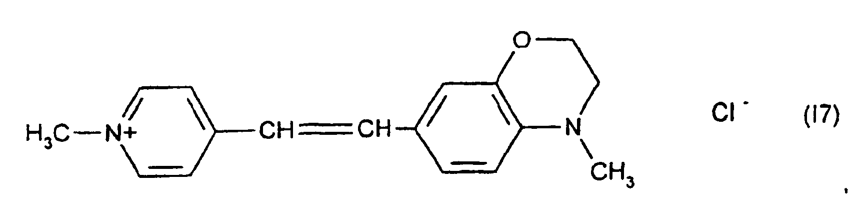 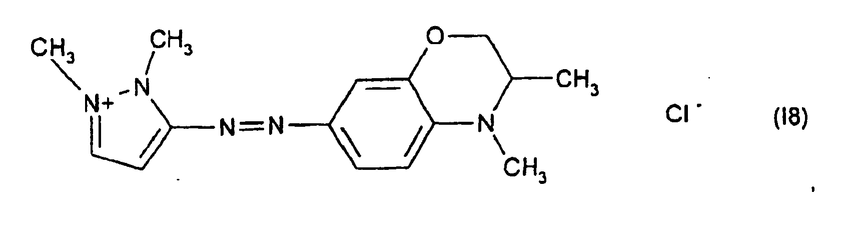 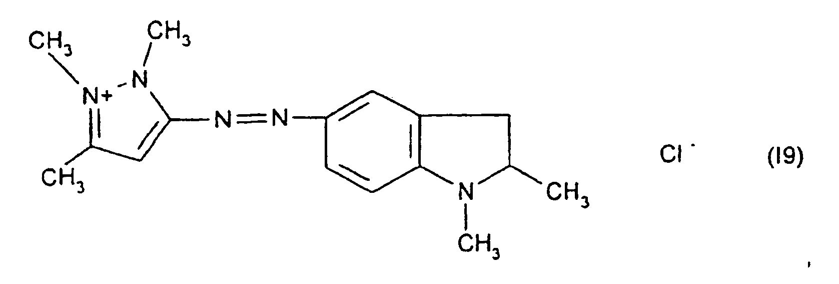 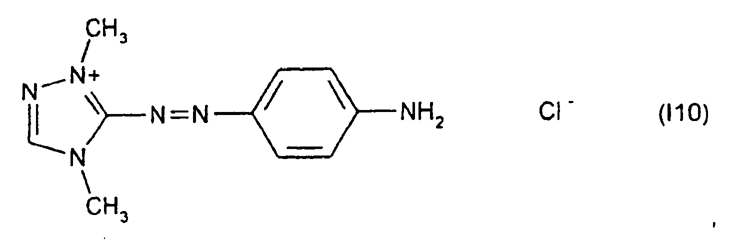 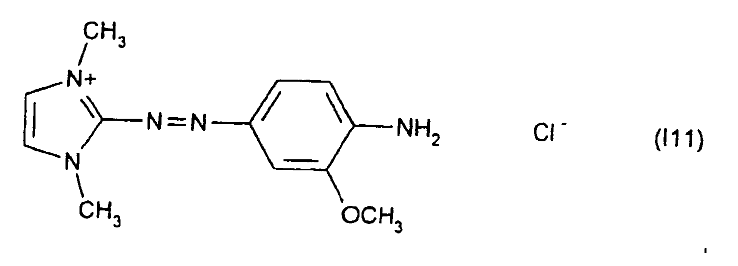 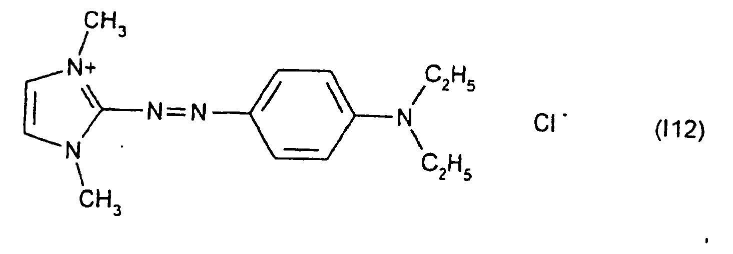 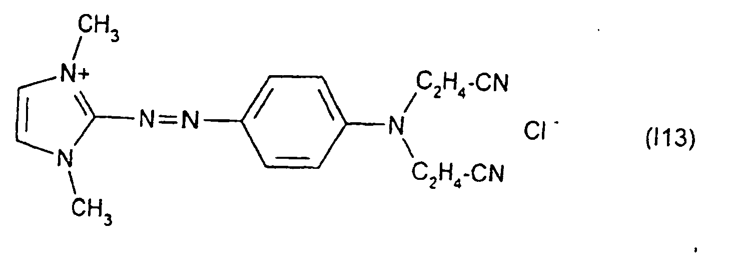 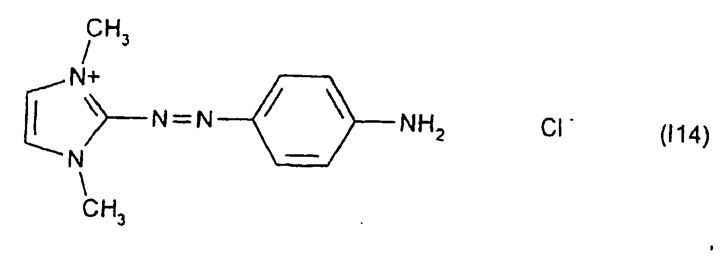 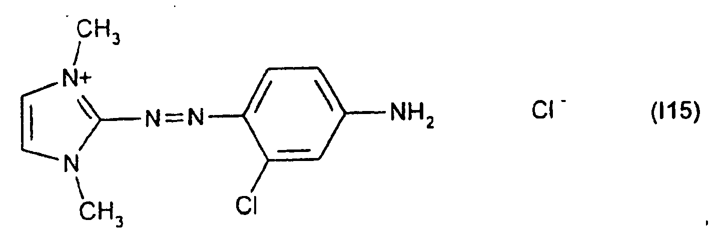 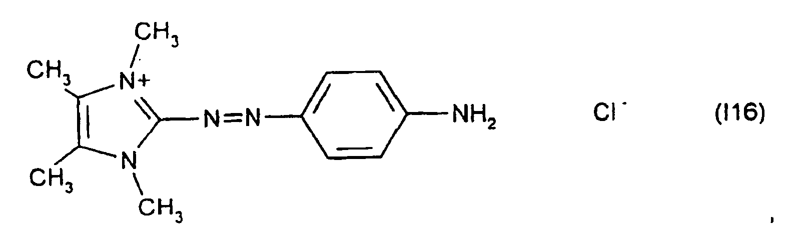 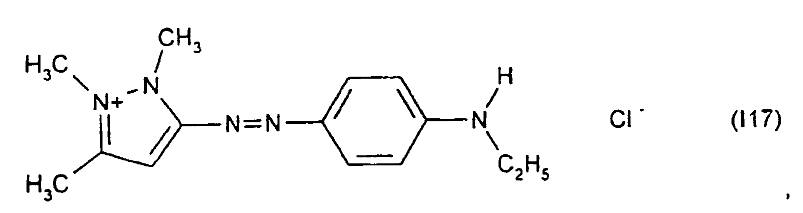 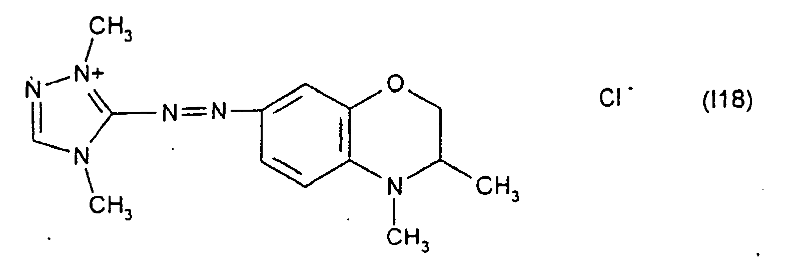 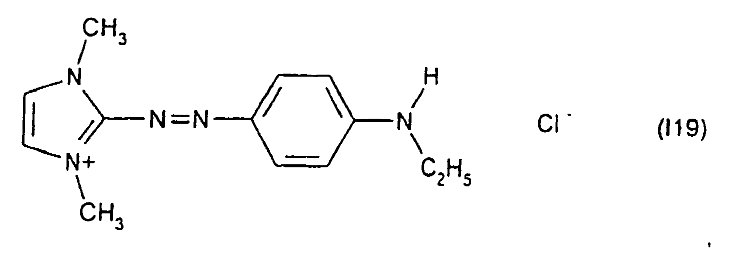 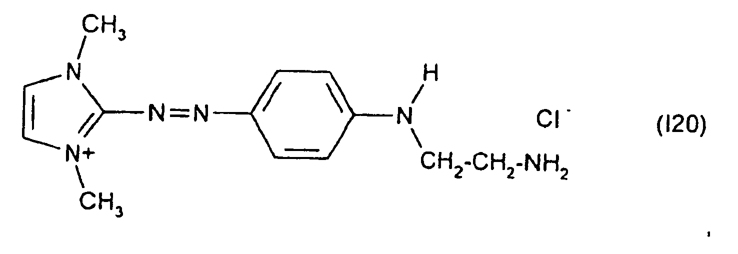 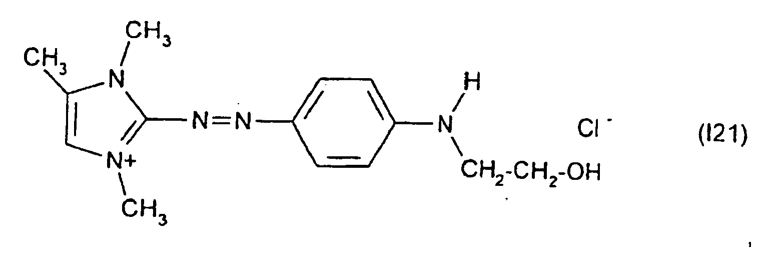 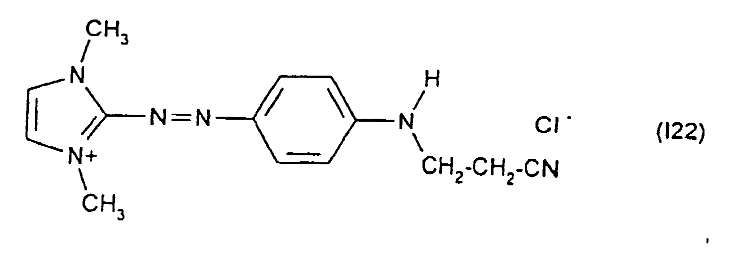 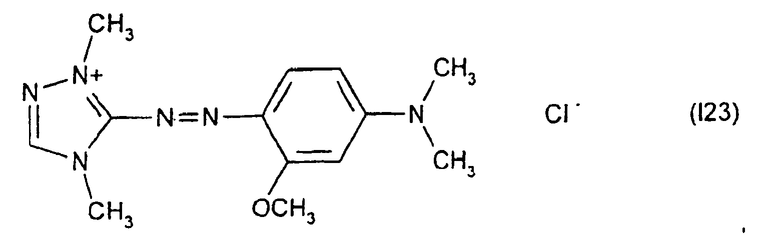 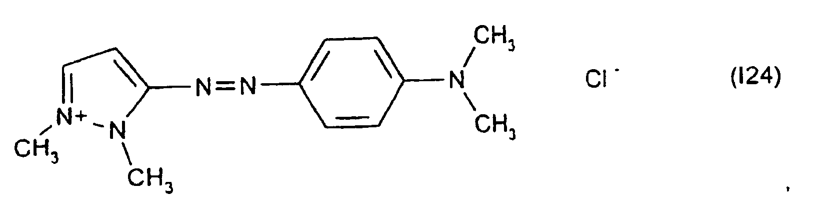 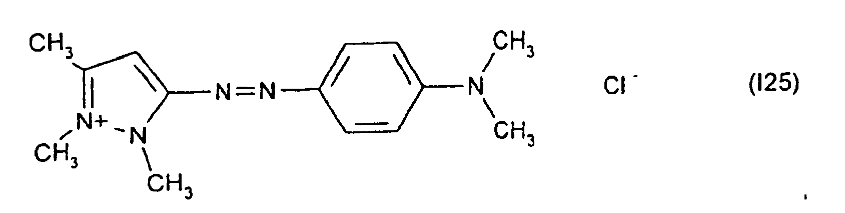 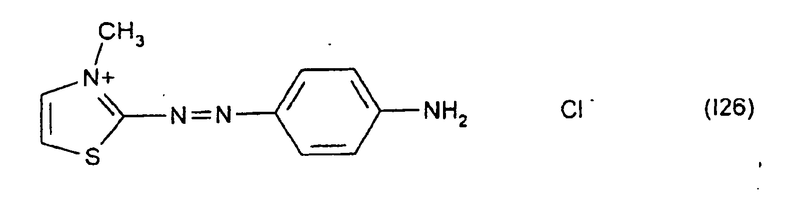 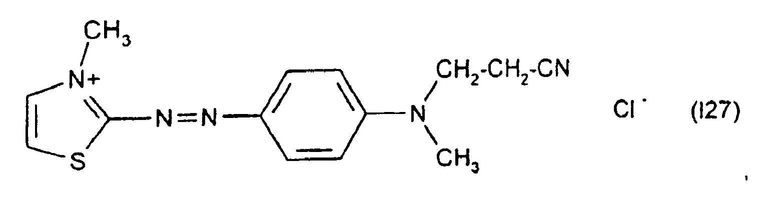 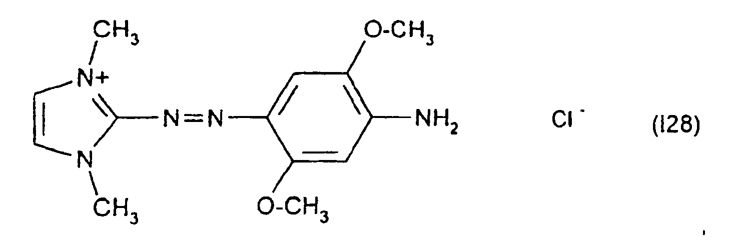 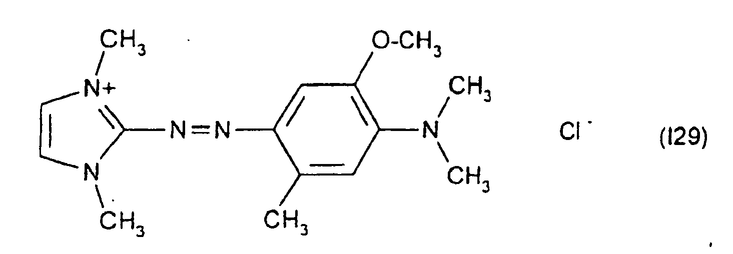 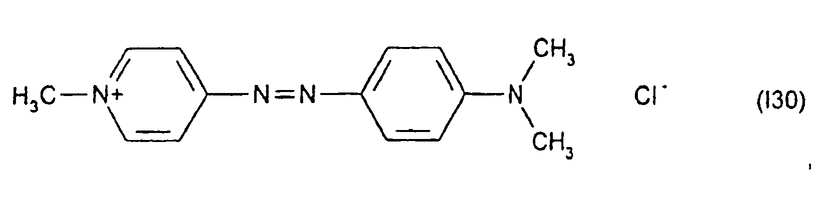 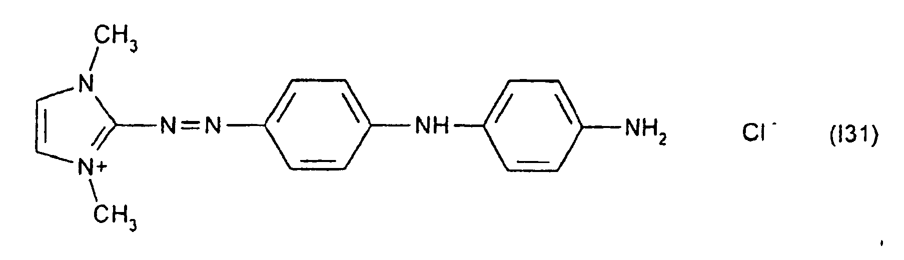 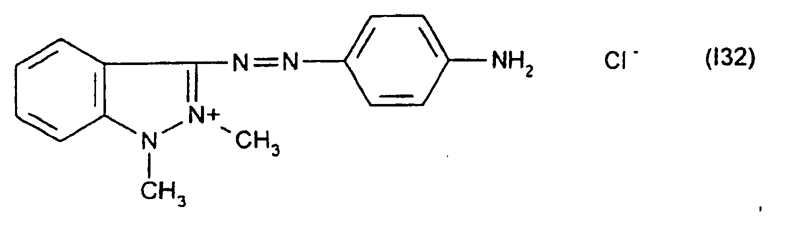 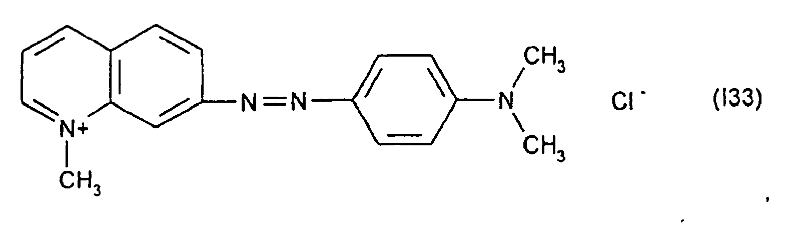 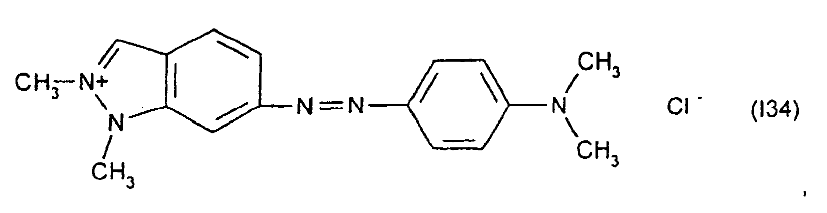 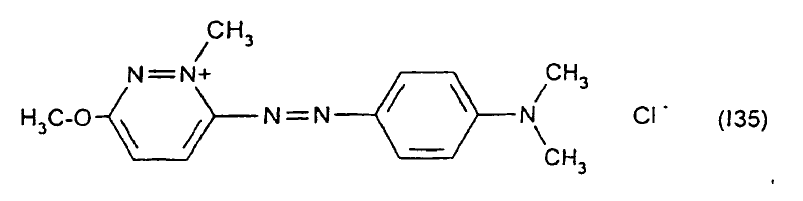 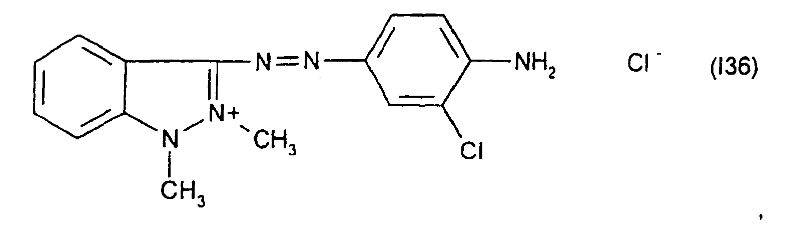 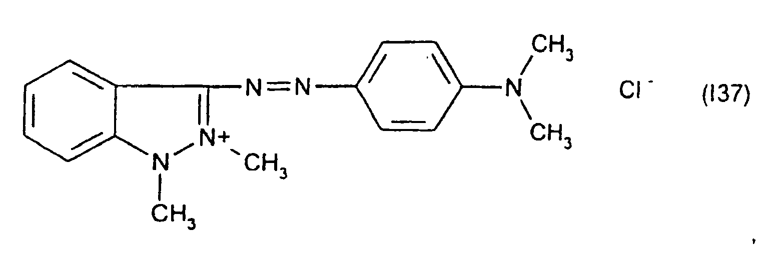 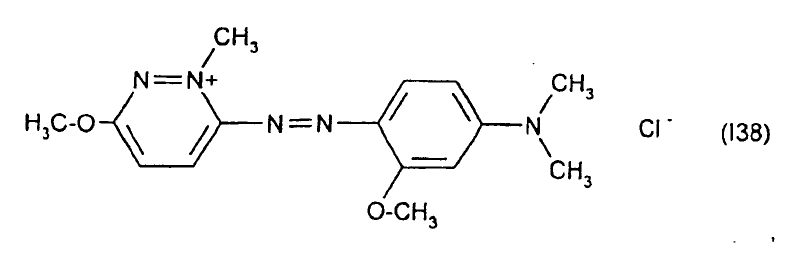 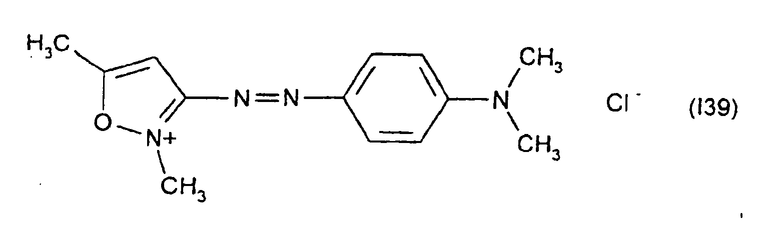 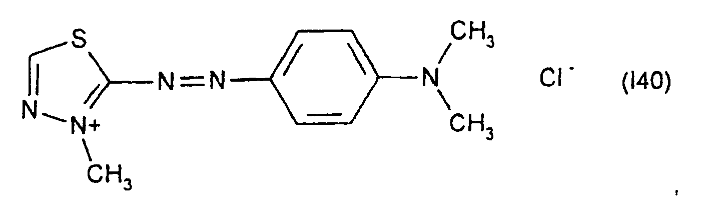 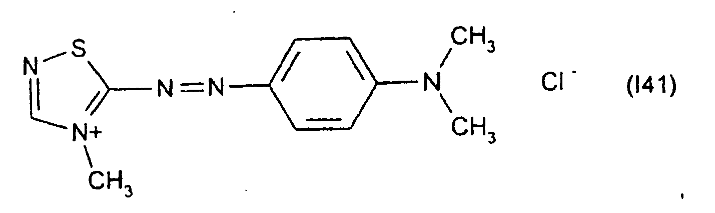 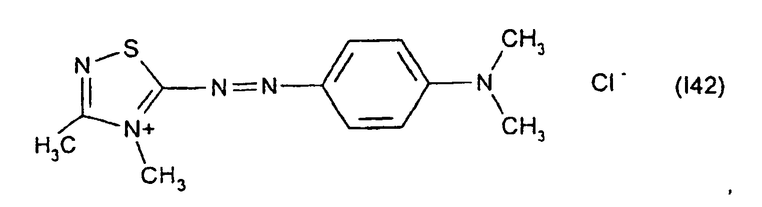 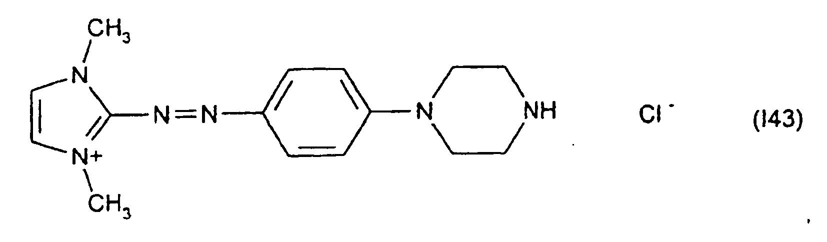 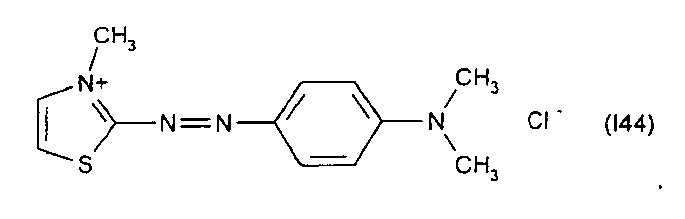 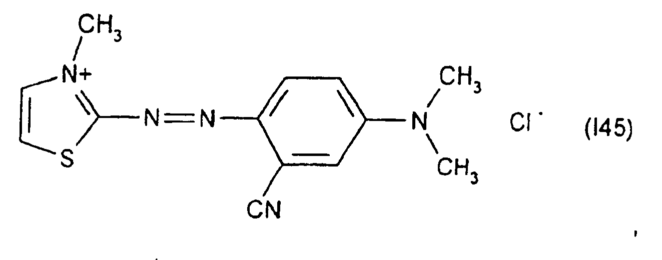 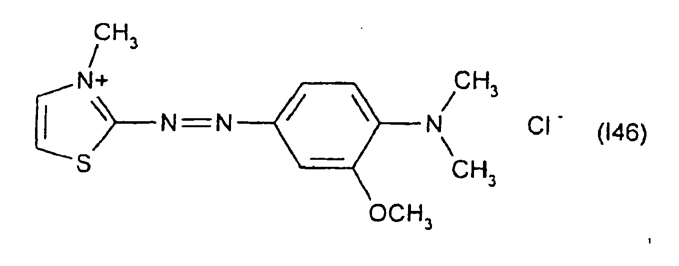 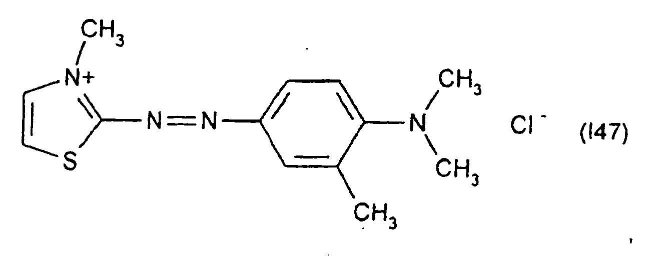 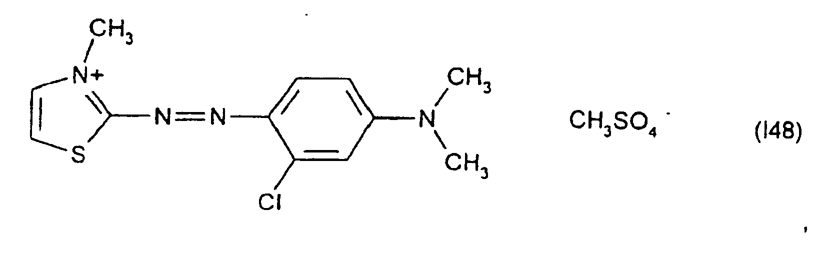 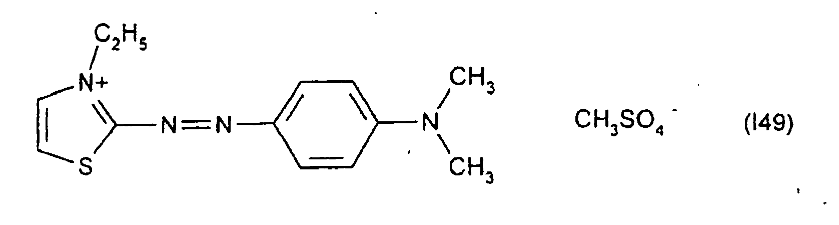 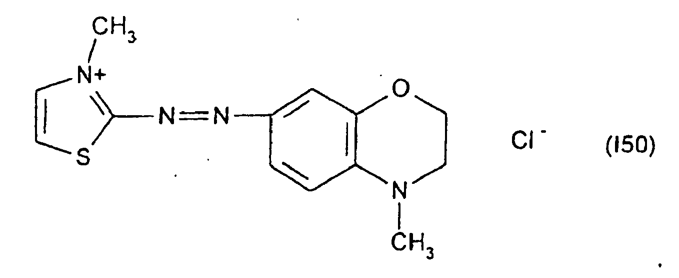 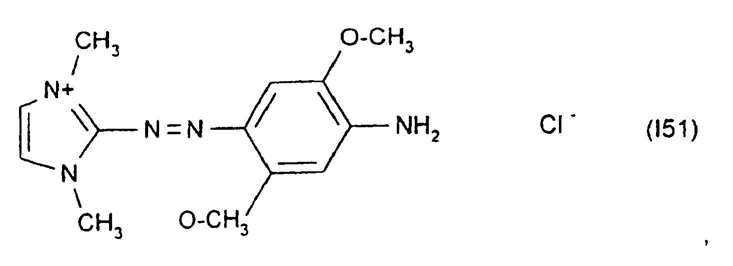 et

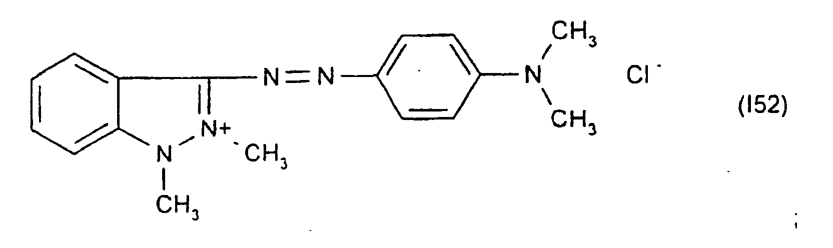

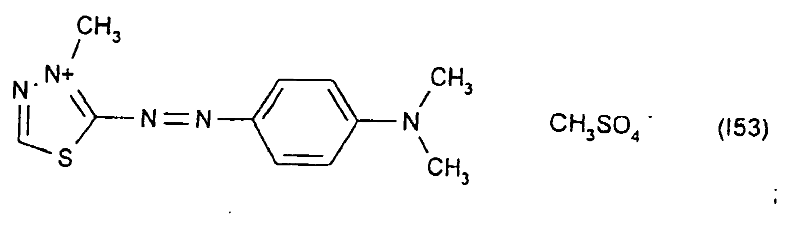

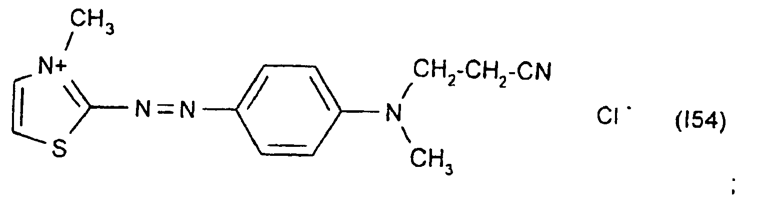

Parmi les composés de structures (I1) à (I54) décrits ci-dessus, on préfère tout particulièrement les composés répondant aux structures (I1), (I2), (I14) et (I31).

Parmi les colorants directs cationiques de formule (II) utilisables dans les compositions tinctoriales conformes à l'invention, on peut plus particulièrement citer les composés répondant aux structures (II1) à (II9) suivantes : 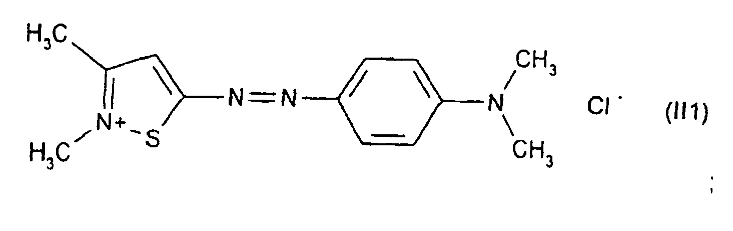 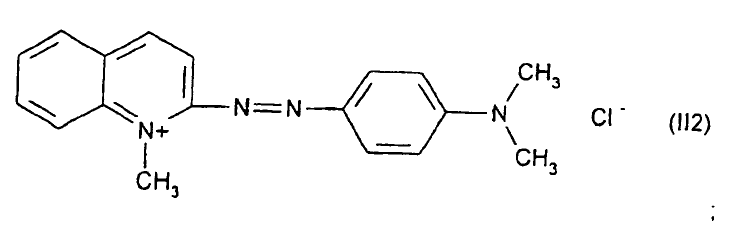 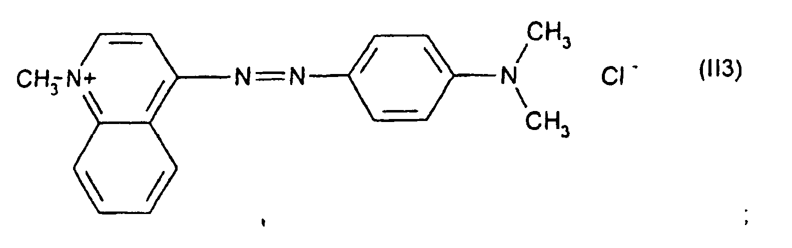 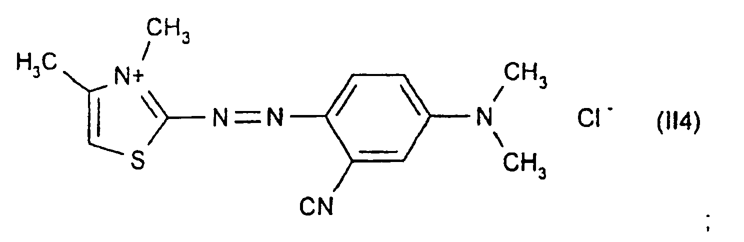 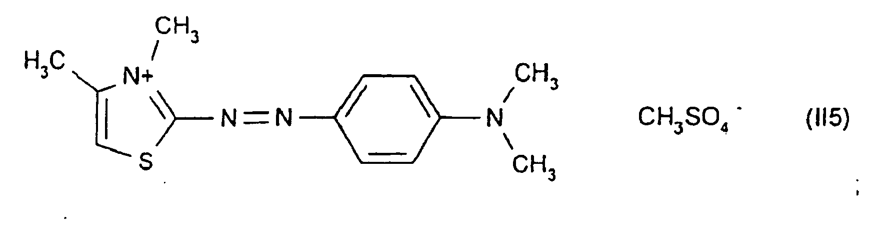 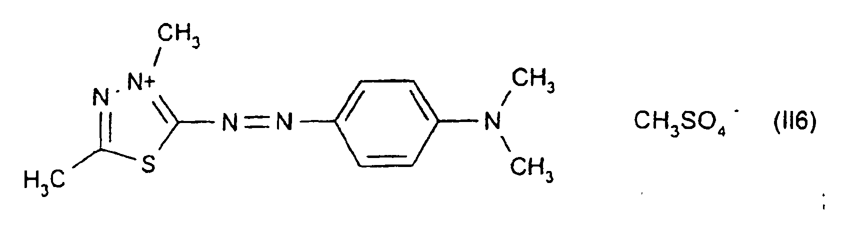 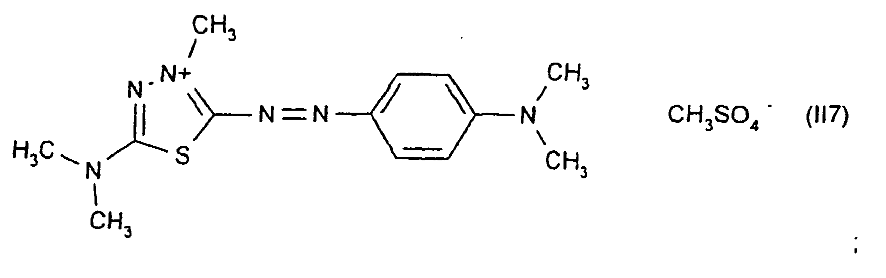 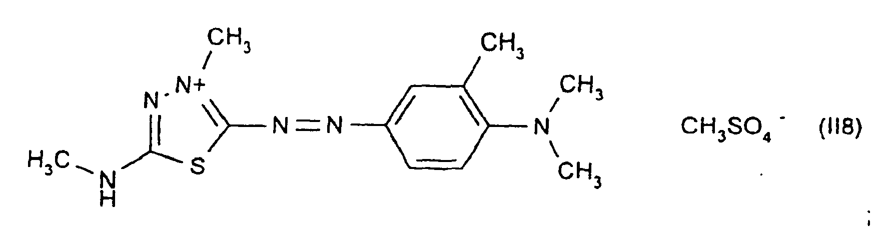 et

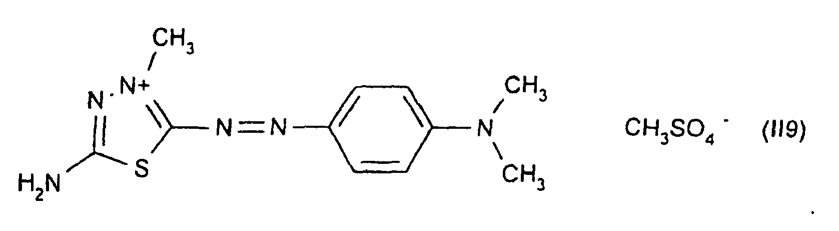

Parmi les colorants directs cationiques de formule (III), utilisables dans les compositions tinctoriales conformes à l'invention, on peut plus particulièrement citer les composés répondant aux structures (III1) à (III18) suivantes : 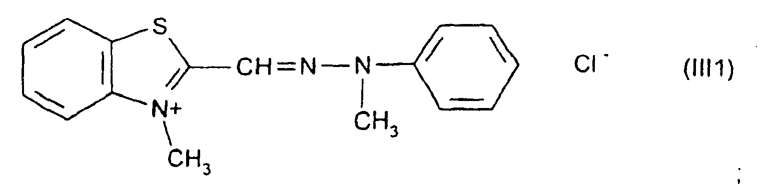 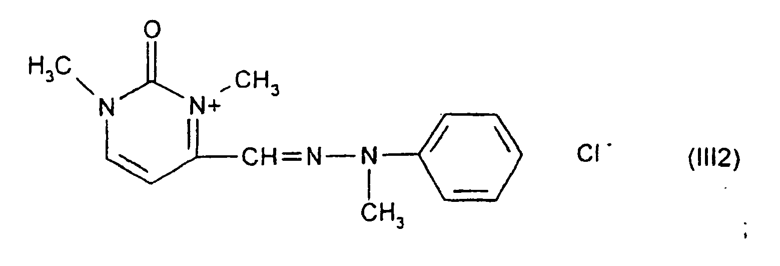 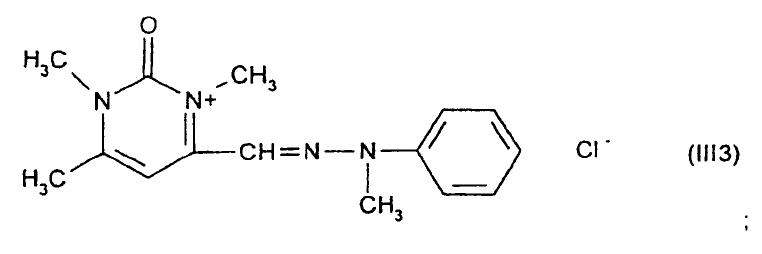 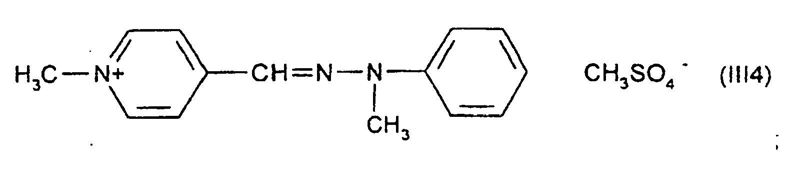 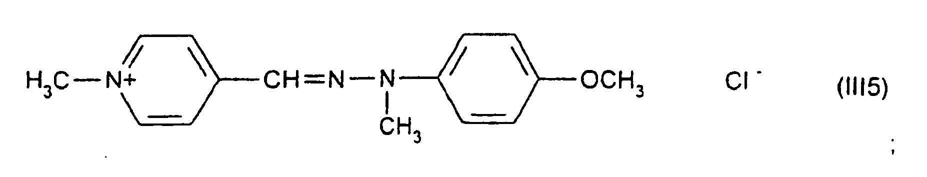 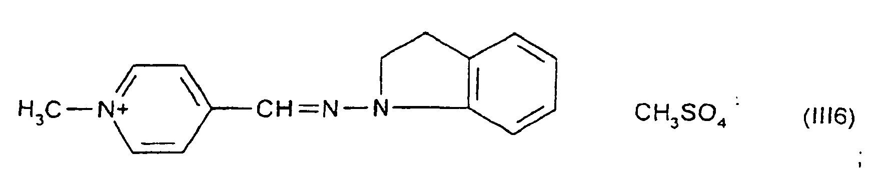 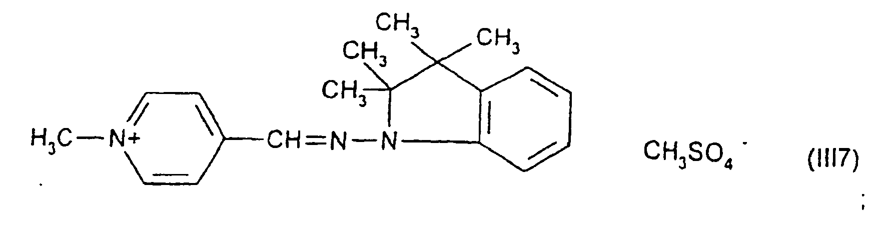 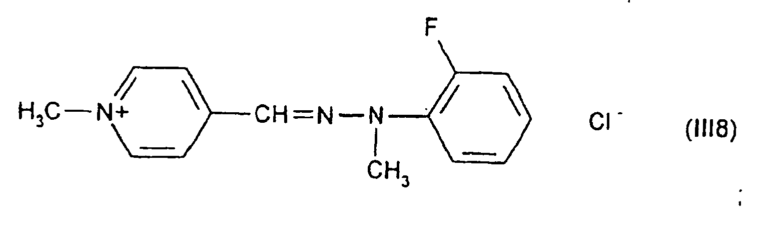 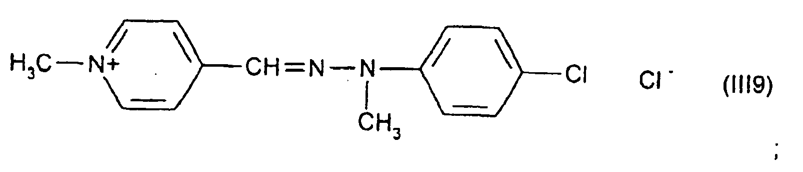 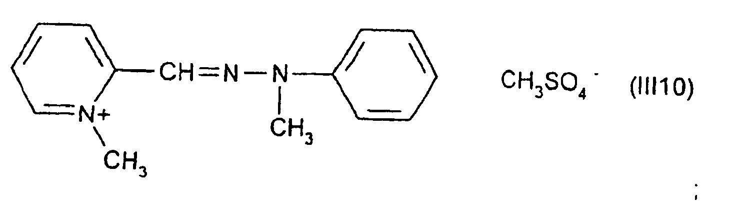 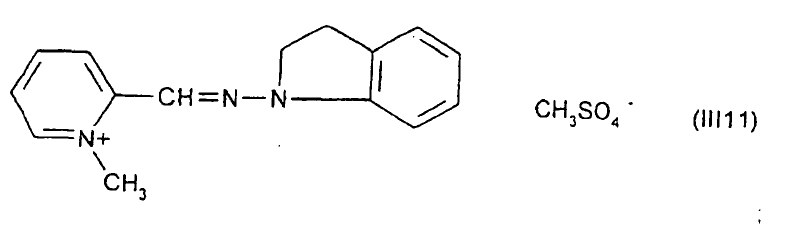 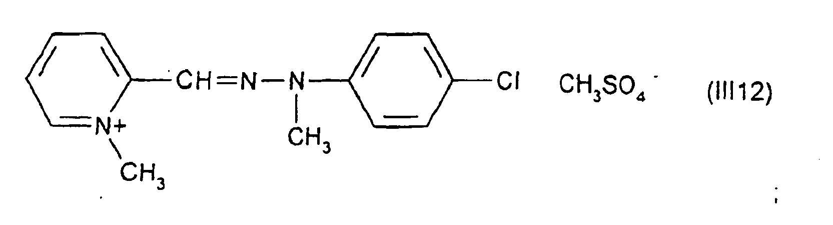 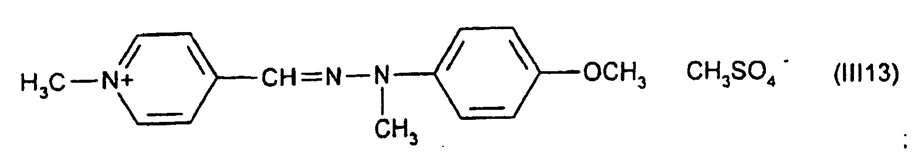 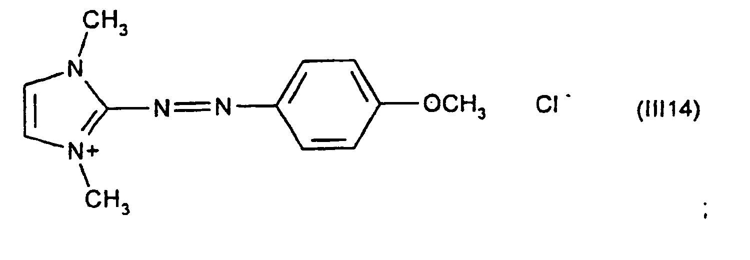 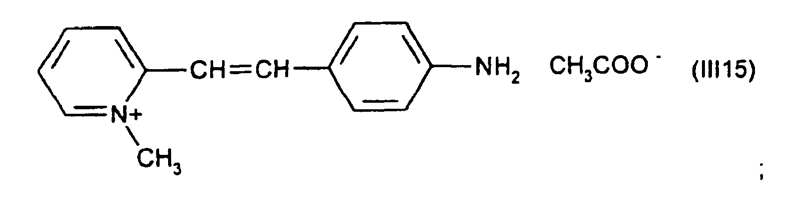 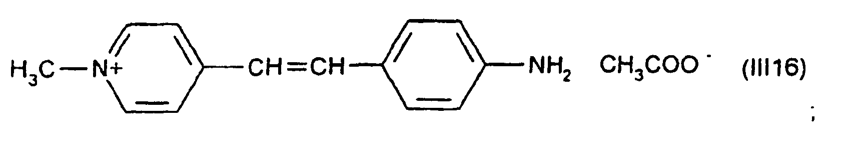 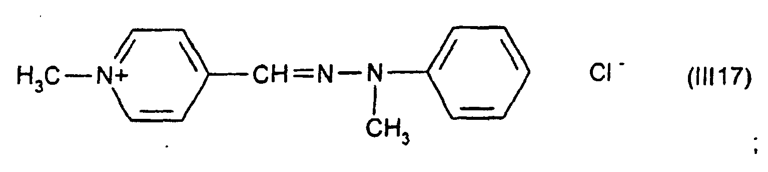 et

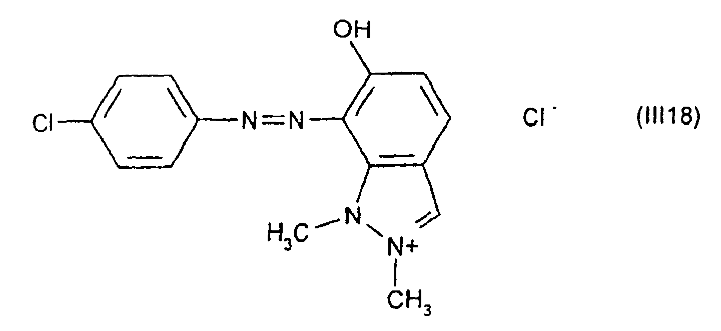

Parmi les composés particuliers de structures (III1) à (III18) décrits ci-dessus, on préfère tout particulièrement les composés répondant aux structures (III4), (III5) et (III13).

Parmi les colorants directs cationiques de formule (III'), utilisables dans les compositions tinctoriales conformes à l'invention, on peut plus particulièrement citer les composés répondant aux structures (III'1) à (III'3) suivantes : 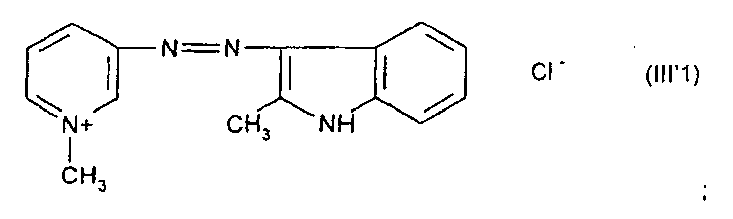 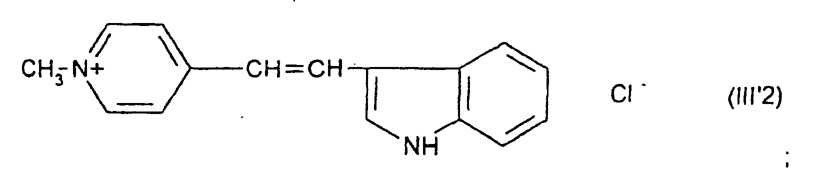 et

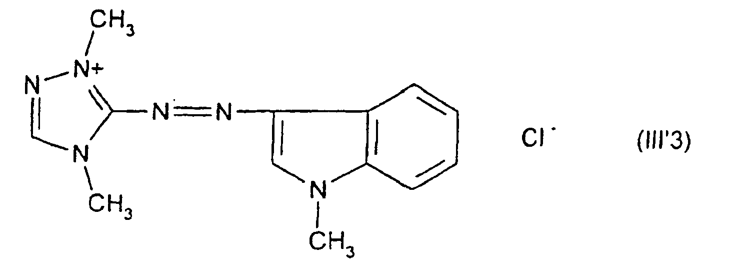

Parmi les colorants directs cationiques de formule (IV) utilisables dans les compositions tinctoriales conformes à l'invention, on peut citer plus particulièrement les composés de structures (IV)₁ à (IV)₇₇ suivantes :

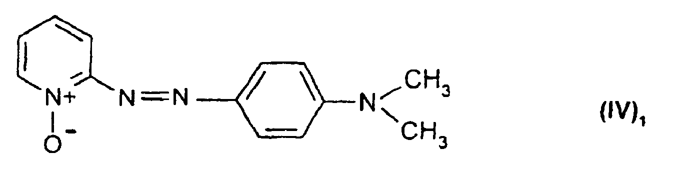

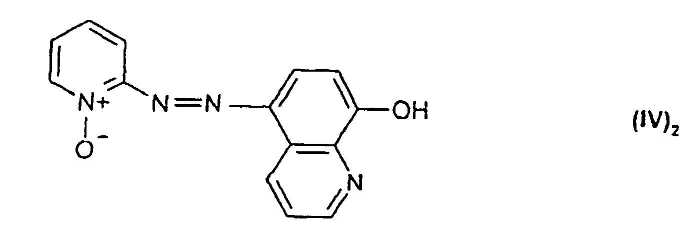

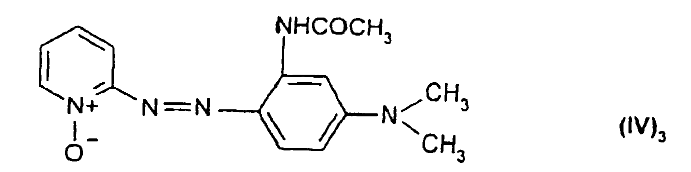

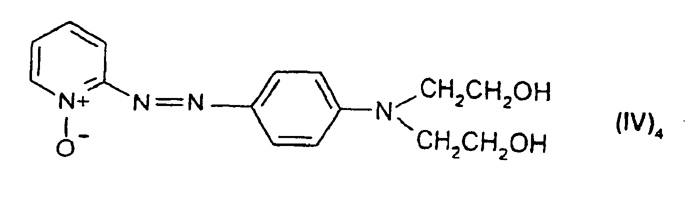

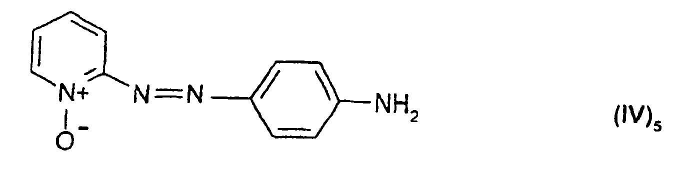

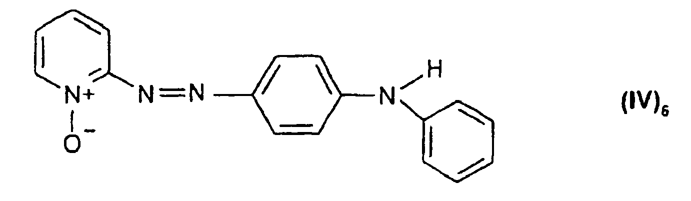

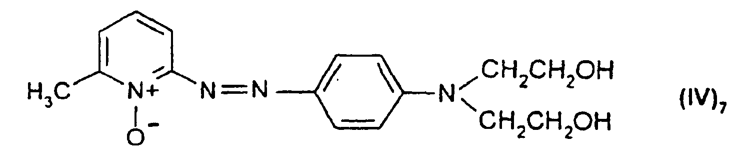

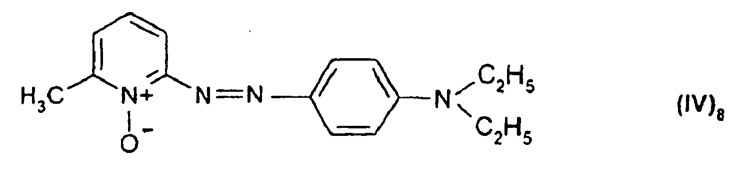

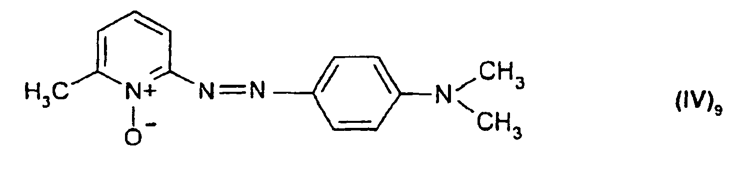

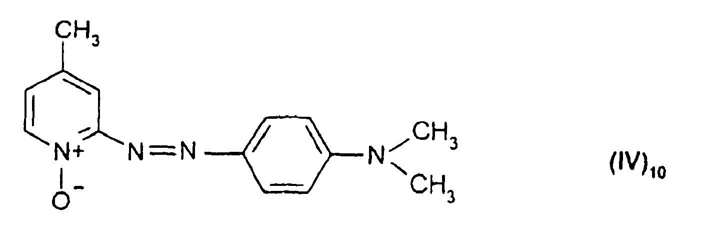

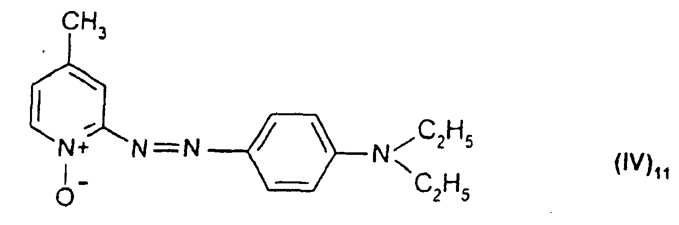

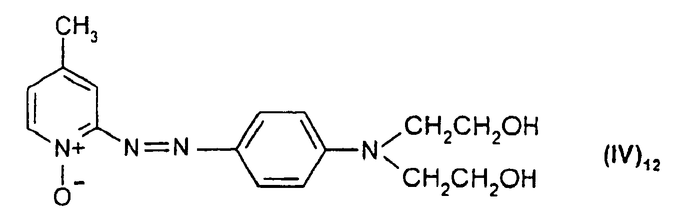

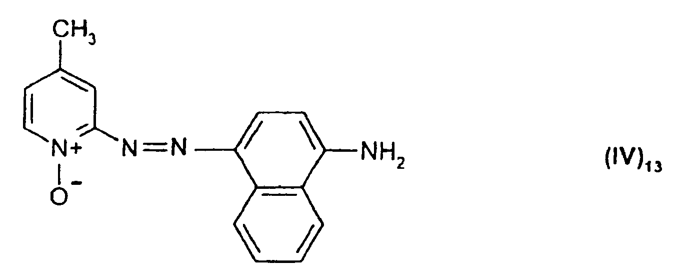

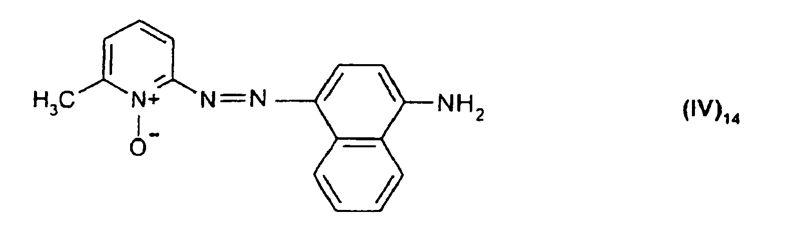

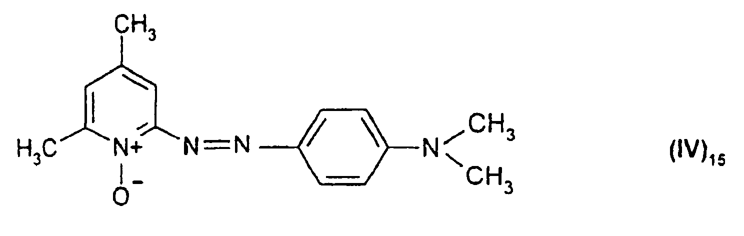

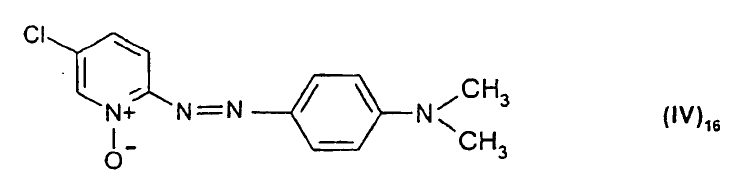

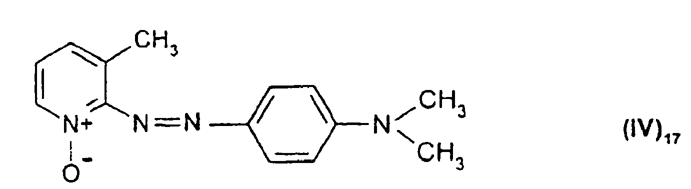

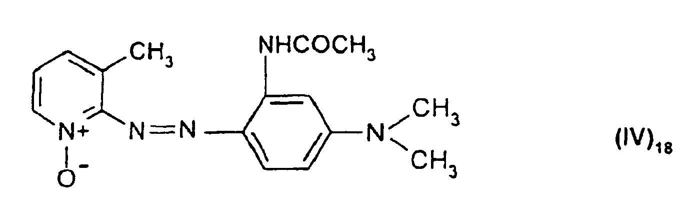

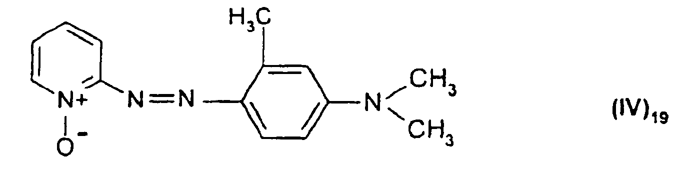

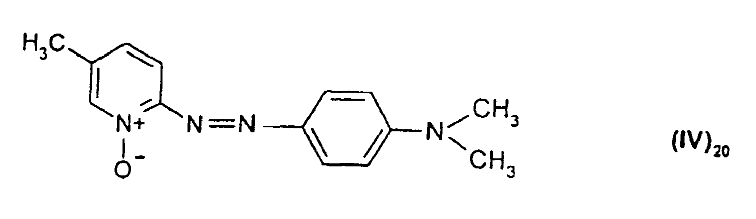

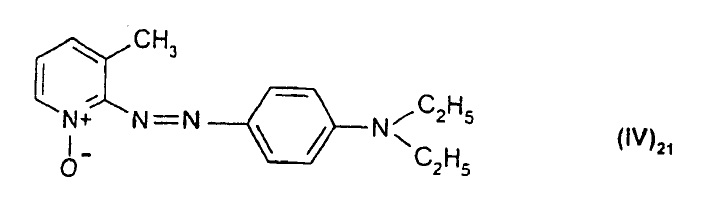

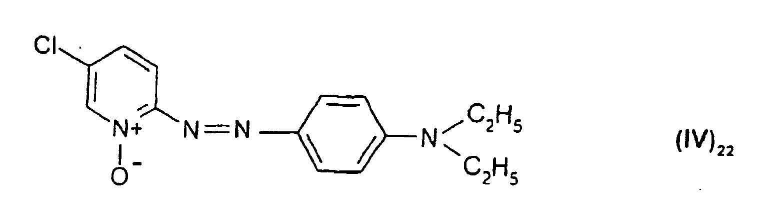

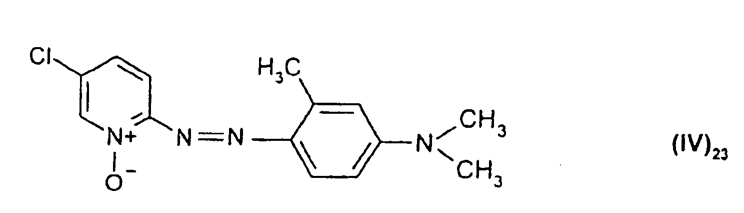

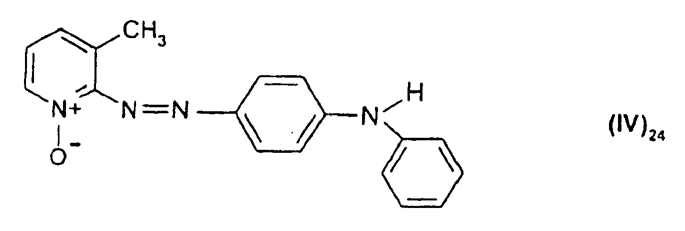

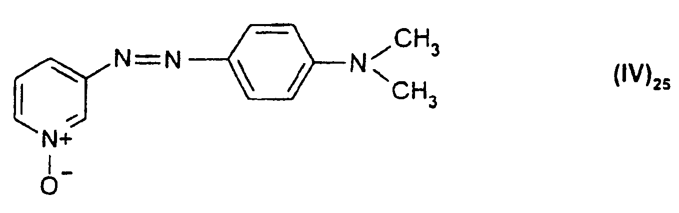

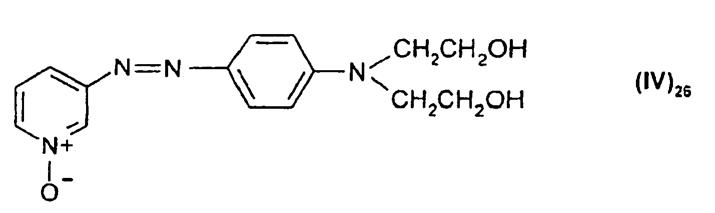

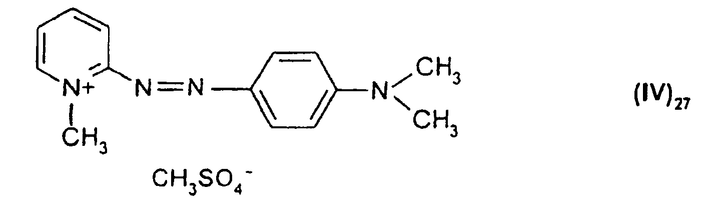

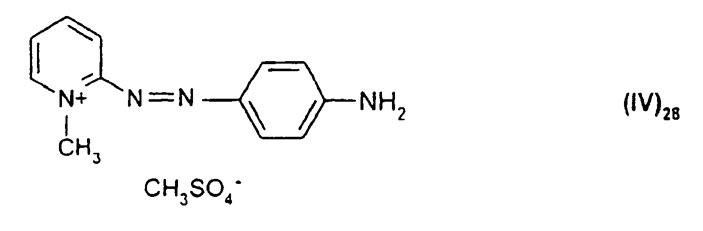

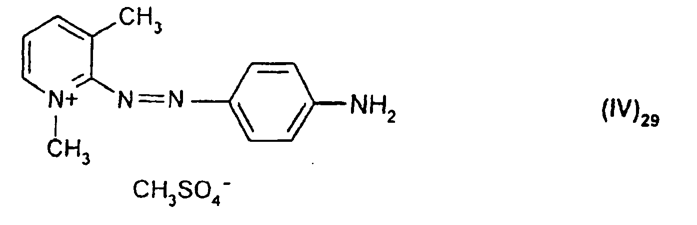

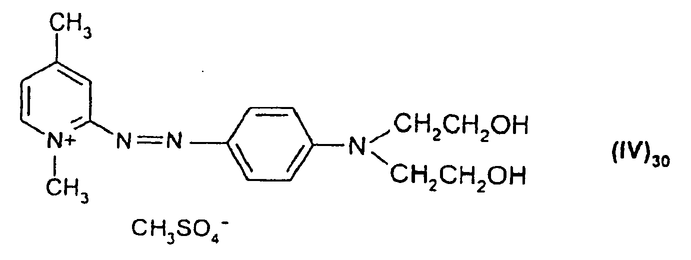

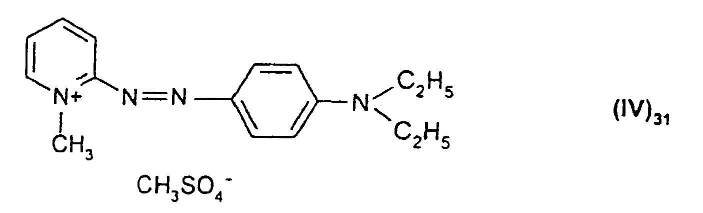

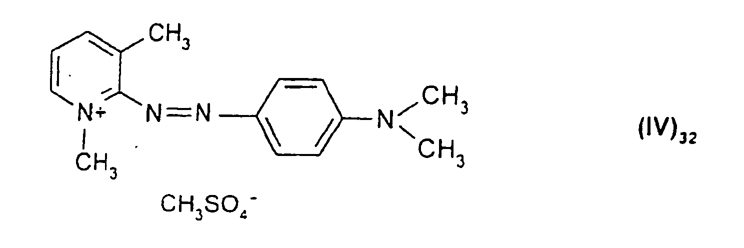

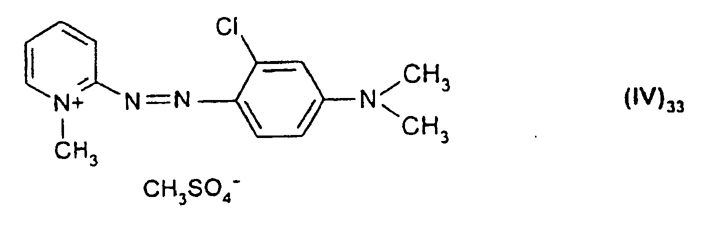

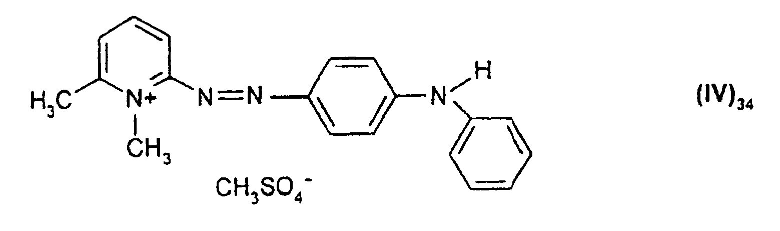

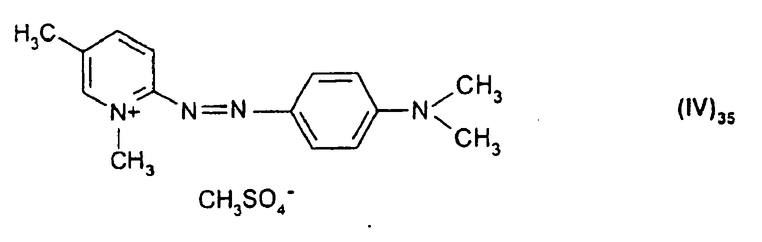

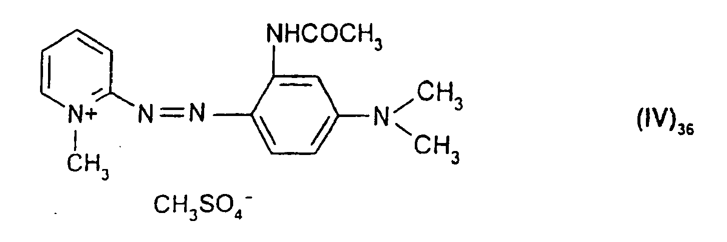

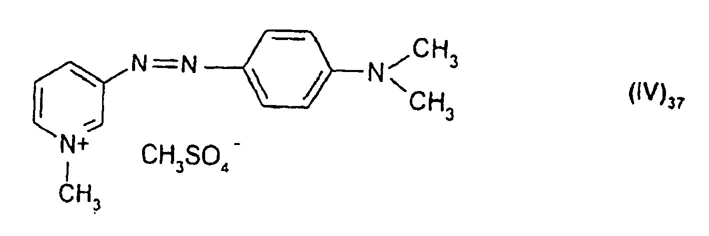

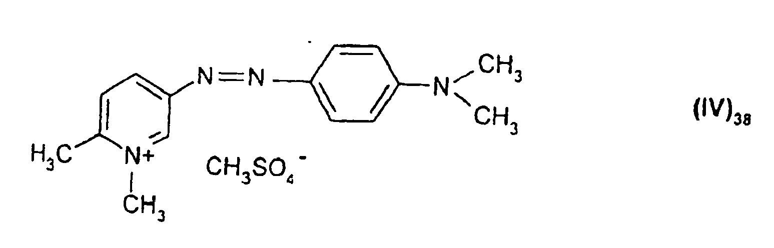

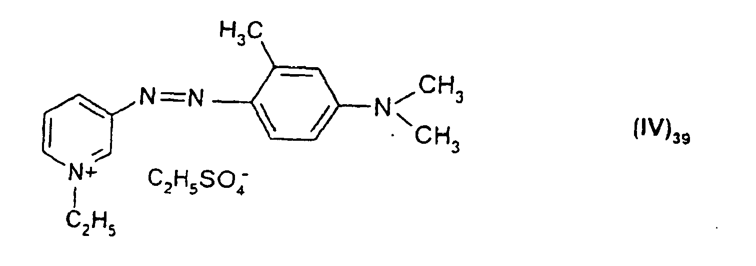

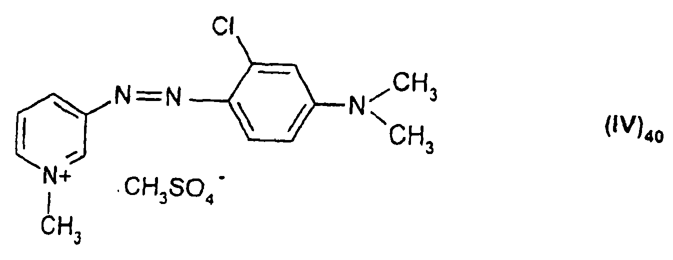

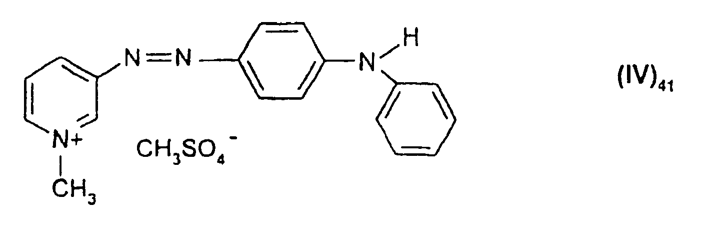

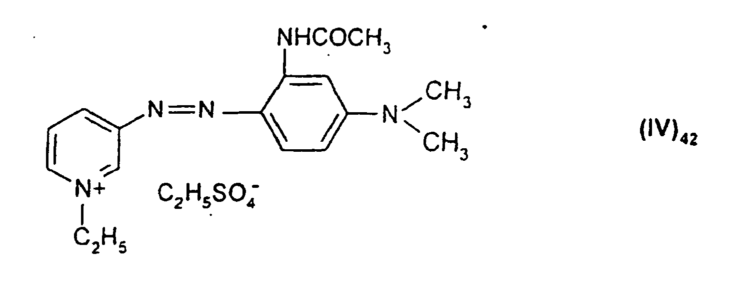

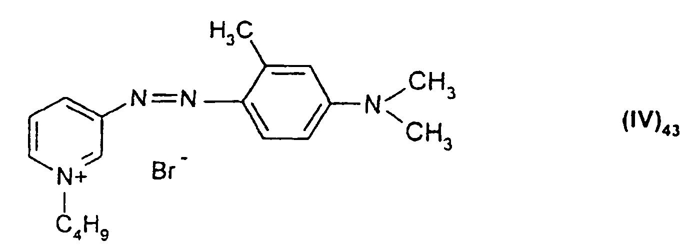

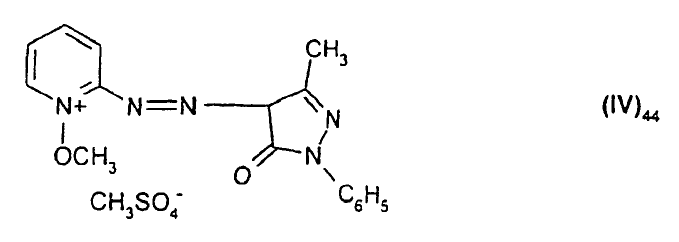

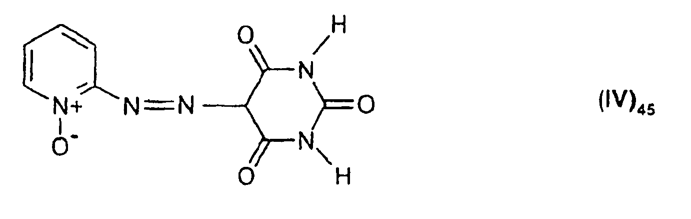

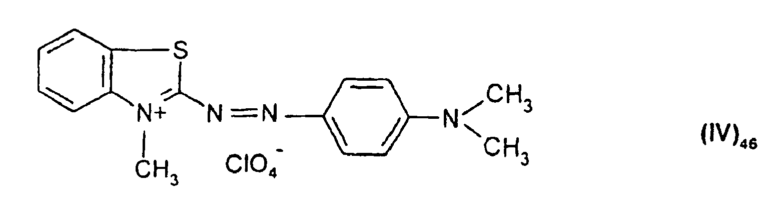

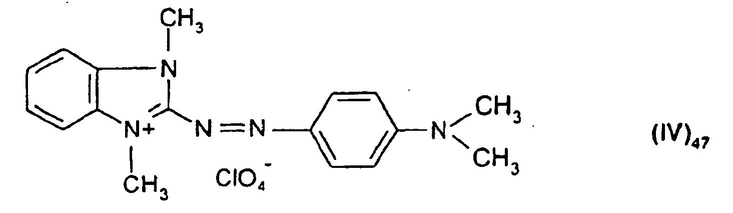

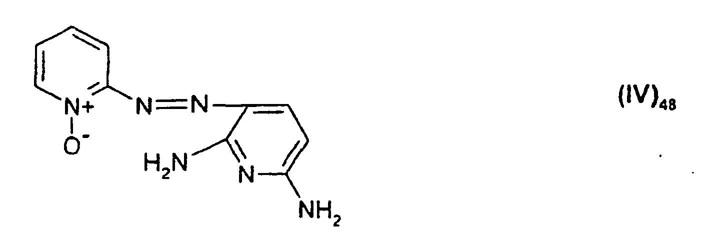

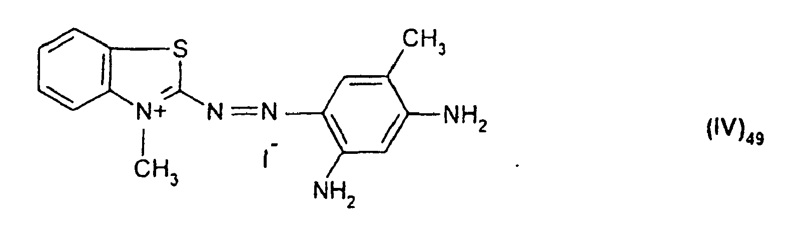

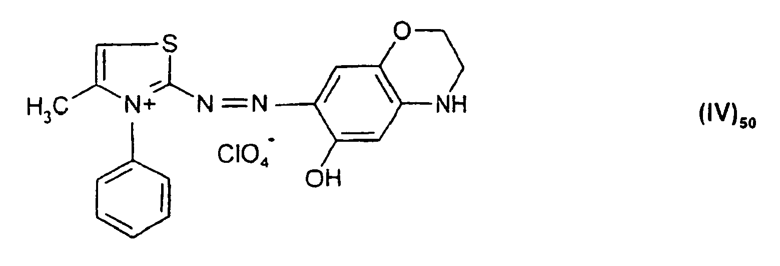

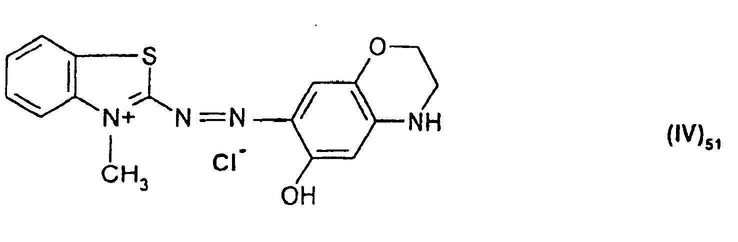

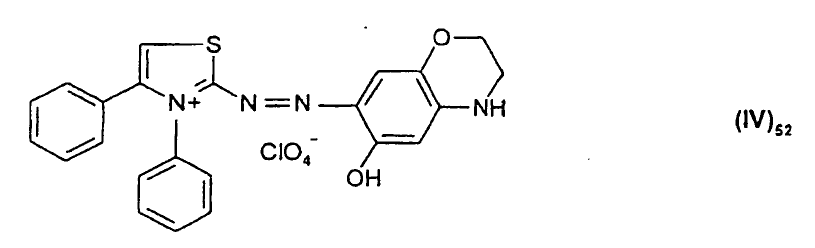

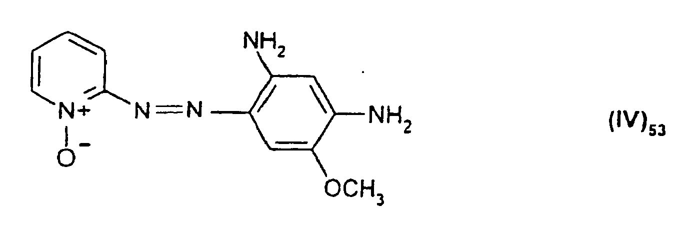

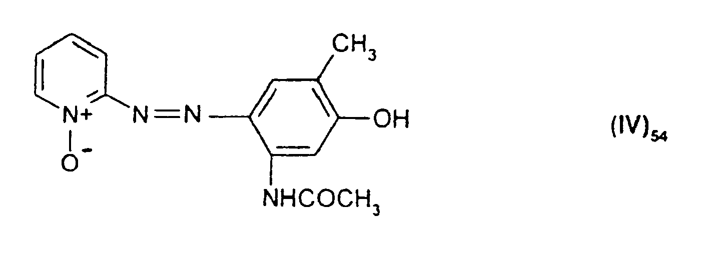

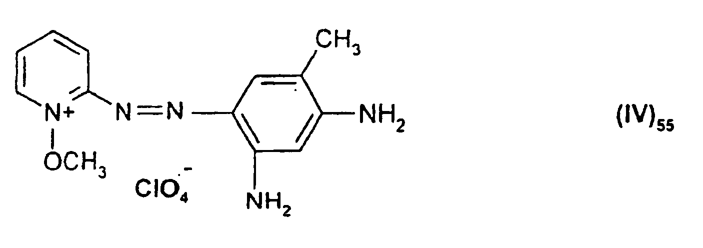

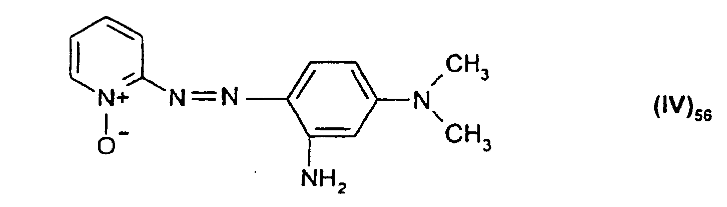

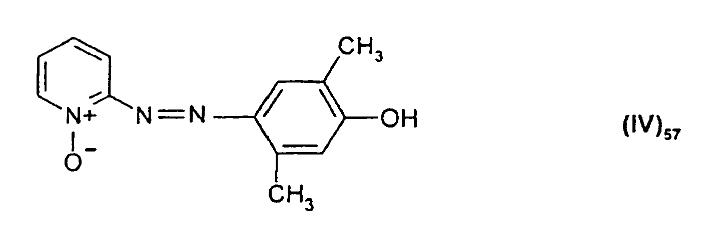

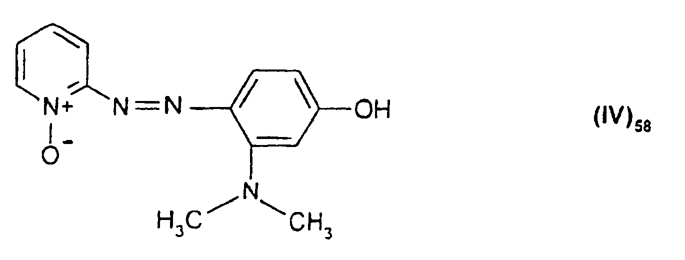

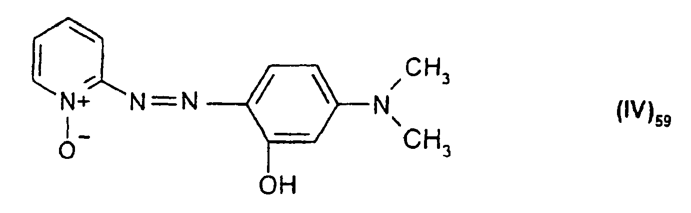

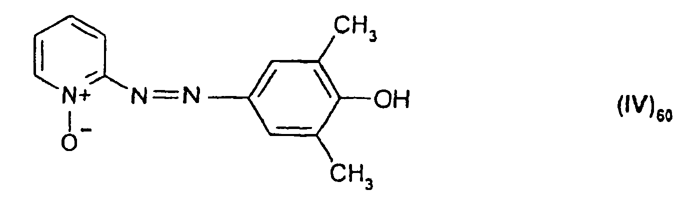

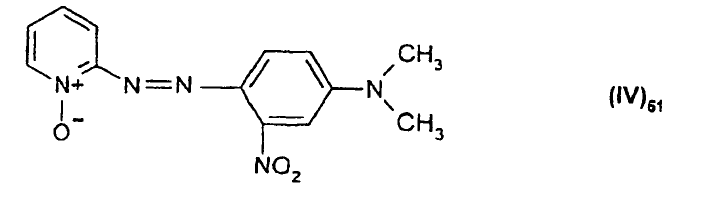

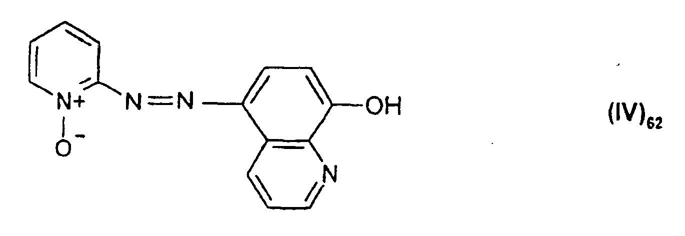

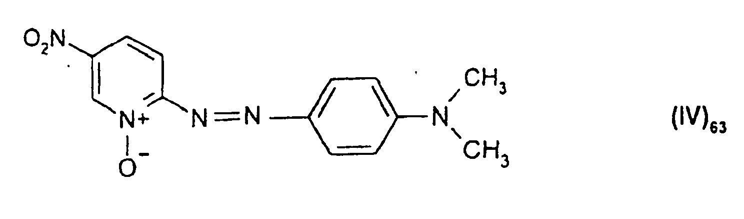

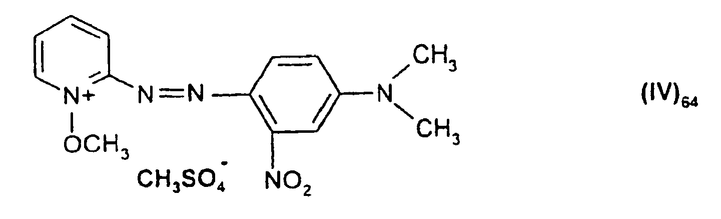

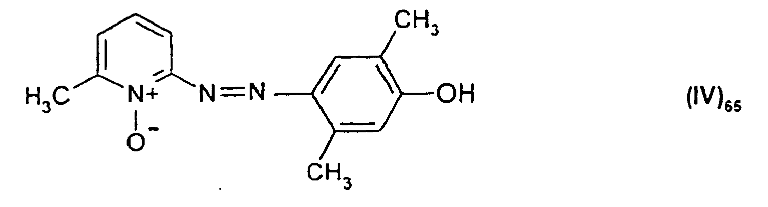

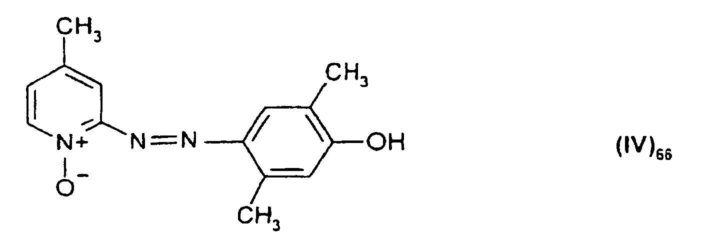

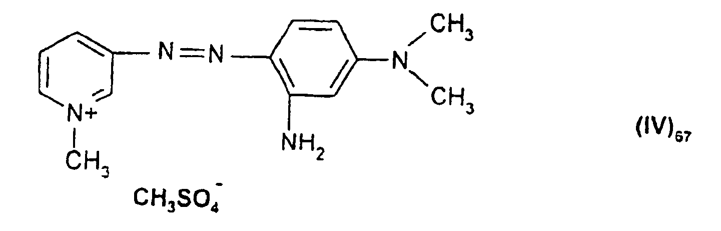

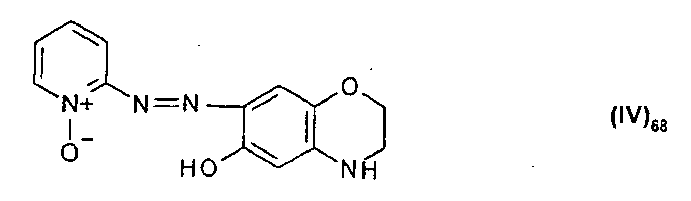

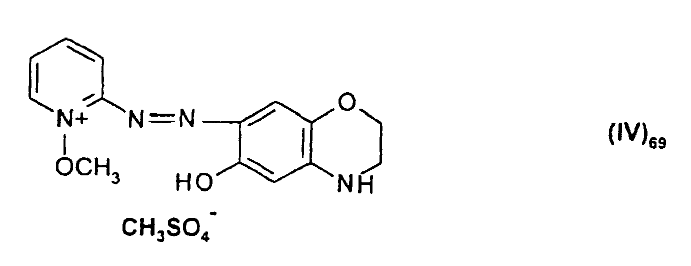

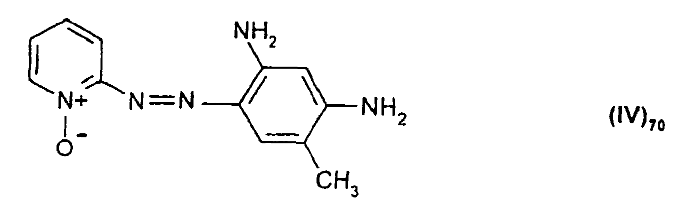

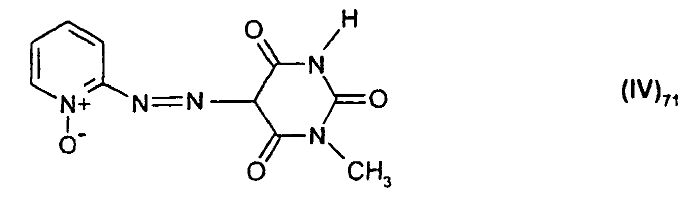

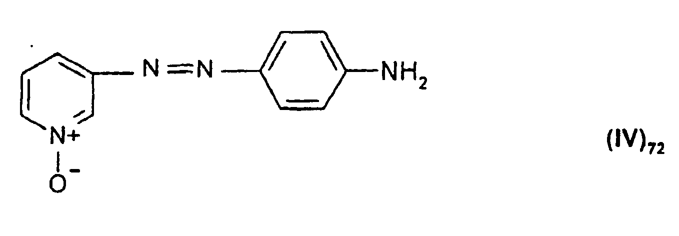

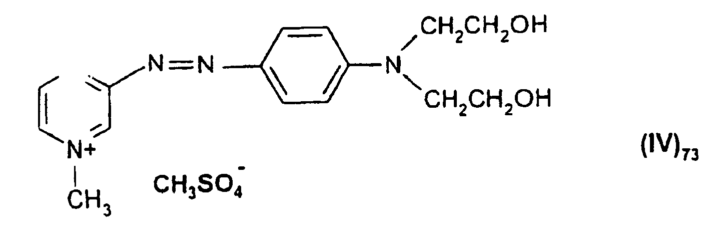

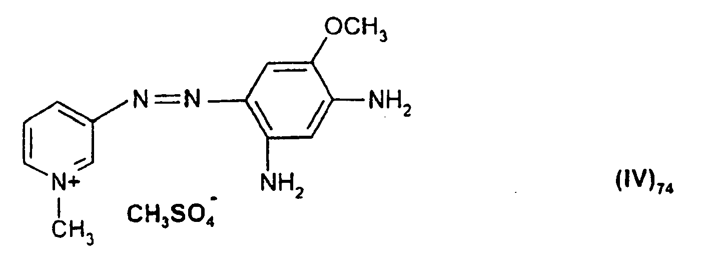

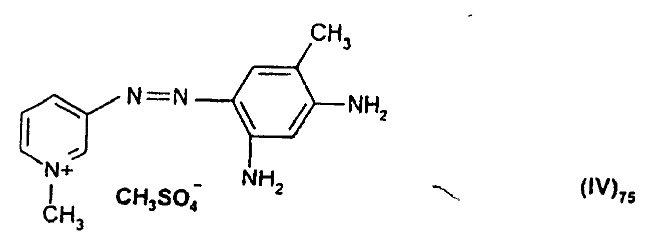

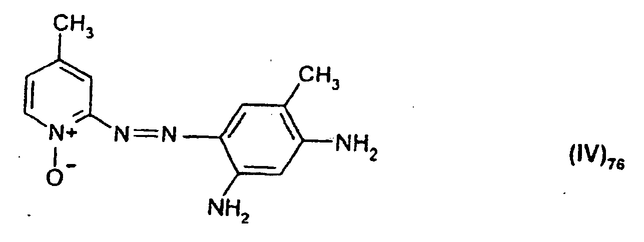

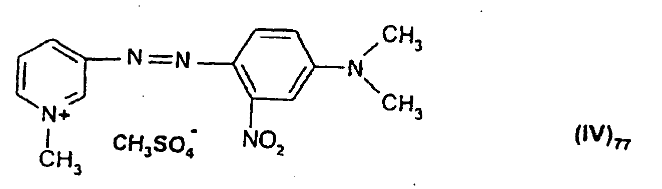

Le ou les colorants directs cationiques utilisés selon l'invention, représentent de préférence de 0,001 à 10 % en poids environ du poids total de la composition tinctoriale et encore plus préférentiellement de 0,005 à 5 % en poids environ de ce poids.
**(ii)** L'agent tensio-actif anionique utilisable selon la présente invention est choisi dans le groupe constitué par :
   **(ii)₁ -** les acyliséthionates ;
   **(ii)₂ -** les acyltaurates ;
   **(ii)₁₀-** leurs mélanges.

Les acyliséthionates **(ii)₁** et acyltaurates **(ii)₂** préférentiels conformes à l'invention correspondent à la structure générale suivante :

R¹-CH₂-CH₂-SO₃⁻ M⁺ (V)

où R¹ désigne un groupe R²COO ou un groupe R²CONR³ avec R² désignant un groupe aliphatique linéaire ou ramifié, saturé ou insaturé en C₈-C₃₀ et R³ désignant un atome d'hydrogène ou un radical alkyle en C₁-C₄ et,
où M désigne H, ammonium, Na ou K ou un reste d'amine organique notamment d'alcanolamine.

Le ou les agents tensio-actifs anioniques utilisés selon l'invention, représentent de préférence de 0,05 à 30 % en poids environ du poids total de la composition tinctoriale et encore plus préférentiellement de 0,1 à 15 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les alcools aromatiques comme l'alcool benzylique, ainsi que les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 2 et 11 environ, et de préférence entre 5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VIII) suivante : 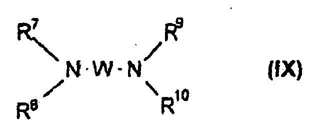 dans laquelle W est un reste propylène éventuellement substitué par un groupement hydroxyle ou un radical alkyle en C₁-C₆ ; R⁷, R⁸, R⁹ et R¹⁰, identiques ou différents, représentent un atome d'hydrogène, un radical alkyle en C₁-C₆ ou hydroxyalkyle en C₁-C₆.

La composition tinctoriale conforme à l'invention peut, en plus du ou des colorants directs cationiques (i) définis précédemment, contenir un ou plusieurs colorants directs additionnels qui peuvent par exemple être choisis parmi les colorants benzéniques nitrés, les colorants anthraquinoniques, les colorants naphtoquinoniques, les colorants triarylméthaniques, les colorants xanthéniques, les colorants azoïques non cationiques.

Lorsqu'elle est destinée à la teinture d'oxydation, la composition tinctoriale conforme à l'invention contient, en plus du ou des colorants directs cationiques (i) une ou plusieurs bases d'oxydation choisie parmi les bases d'oxydation classiquement utilisées pour la teinture d'oxydation et parmi lesquelles on peut notamment citer les paraphénylènediamines, les bis-phénylalkylènediamines, les para-aminophénols, les ortho-aminophénols et les bases hétérocycliques. Lorsqu'elles sont utilisées, la ou les bases d'oxydation représentent de préférence de 0,0005 à 12 % en poids environ du poids total de la composition tinctoriale, et encore plus préférentiellement de 0,005 à 6 % en poids environ de ce poids.

Lorsqu'elle est destinée à la teinture d'oxydation, la composition tinctoriale conforme à l'invention peut également renfermer, en plus du colorant direct cationique (i) et de l'agent tensio-actif anionique (ii) ainsi que des bases d'oxydation, un ou plusieurs coupleurs de façon à modifier ou à enrichir en reflets les nuances obtenues en mettant en oeuvre le ou les colorants direct(s) cationique(s) (i) et la ou les bases d'oxydation.

Les coupleurs utilisables dans la composition tinctoriale conforme à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques.
Lorsqu'ils sont présents, le ou les coupleurs représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut également renfermer divers adjuvants utilisés classiquement dans les compositions pour la teinture des cheveux, tels que des agents antioxydants, des agents de pénétration, des agents séquestrants, des parfums, des tampons, des agents dispersants, des agents filmogènes, des céramides, des agents conservateurs, des agents filtrants, des agents opacifiants.

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées.

La composition tinctoriale selon l'invention peut se présenter sous des formes diverses, telles que sous forme de liquides, de shampooings, de crèmes, de gels, ou sous toute autre forme appropriée pour réaliser une teinture des fibres kératiniques, et notamment des cheveux humains. Elle peut être obtenue par mélange extemporané d'une composition, éventuellement pulvérulente, contenant le ou les colorants directs cationiques avec une composition contenant l'agent tensio-actif anionique.

Lorsque l'association du colorant direct cationique (i) et de l'agent tensio-actif anionique (ii) selon l'invention est utilisée dans une composition destinée à la teinture d'oxydation (une ou plusieurs bases d'oxydation sont alors utilisées, éventuellement en présence d'un ou plusieurs coupleurs) ou lorsqu'elle est utilisée dans une composition destinée à la teinture directe éclaircissante, alors la composition tinctoriale conforme à l'invention renferme en outre au moins un agent oxydant, choisi par exemple parmi le peroxyde d'hydrogène, le peroxyde d'urée, les bromates de métaux alcalins, les persels tels que les perborates et persulfates, et les enzymes telles que les peroxydases, les laccases et les oxydo-réductases à deux électrons. L'utilisation du peroxyde d'hydrogène ou des enzymes est particulièrement préférée.

Un autre objet de l'invention est un procédé de teinture des fibres kératiniques et en particulier des fibres kératiniques humaines telles que les cheveux mettant en oeuvre la composition tinctoriale telle que définie précédemment.

Selon une première variante de ce procédé de teinture conforme à l'invention, on applique sur les fibres au moins une composition tinctoriale telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une deuxième variante de ce procédé de teinture conforme à l'invention, on applique sur les fibres au moins une composition tinctoriale telle que définie précédemment, pendant un temps suffisant pour développer la coloration désirée, sans rinçage final.

Selon une forme de réalisation particulière de ce procédé de teinture, et lorsque la composition tinctoriale conforme à l'invention renferme au moins une base d'oxydation et au moins un agent oxydant, le procédé de teinture comporte une étape préliminaire consistant à stocker sous forme séparée, d'une part, une composition (A1) comprenant, dans un milieu approprié pour la teinture, au moins un colorant direct cationique (i) tel que défini précédemment et au moins une base d'oxydation et, d'autre part, une composition (B1) renfermant, dans un milieu approprié pour la teinture, au moins un agent oxydant, puis à procéder à leur mélange au moment de l'emploi avant d'appliquer ce mélange sur les fibres kératiniques, la composition (A1) ou la composition (B1) contenant l'agent tensio-actif anionique (ii) tel que défini précédemment.

Selon une autre forme de réalisation particulière de ce procédé de teinture, et lorsque la composition tinctoriale conforme à l'invention renferme au moins un agent oxydant, le procédé de teinture comporte une étape préliminaire consistant à stocker sous forme séparée, d'une part, une composition (A2) comprenant, dans un milieu approprié pour la teinture, au moins un colorant direct cationique (i) tel que défini précédemment et, d'autre part, une composition (B2) renfermant, dans un milieu approprié pour la teinture, au moins un agent oxydant, puis à procéder à leur mélange au moment de l'emploi avant d'appliquer ce mélange sur les fibres kératiniques, la composition (A2) ou la composition (B2) contenant l'agent tensio-actif anionique tel que défini précédemment.

Un autre objet de l'invention est un dispositif à plusieurs compartiments ou "kit" de teinture ou tout autre système de conditionnement à plusieurs compartiments dont un premier compartiment renferme la composition (A1) ou (A2) telle que définie ci-dessus et un second compartiment renferme la composition (B1) ou (B2) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLE

On a préparé la composition de teinture directe réunie dans le tableau suivant :
(*toutes teneurs exprimées en grammes*)

| | **Exemple** |
|---|---|
| Colorant direct cationique de formule (I1) | |
| Colorant direct cationique de formule (I14) | |
| Colorant direct cationique de formule (I31) | 0,15 |
| Colorant direct cationique de formule (IV)₁₀ | |
| Colorant direct cationique de formule (IV)₂₇ | |
| Cocoyliséthionate de sodium vendu sous la dénomination JORDAPON CI POWDER par la société PPG | 5,0 MA* |
| CESALPINA | |
| Ethanol | 10 |
| 2-amino-2-méthyl-1-propanol ....qs. | pH 9 |
| Eau déminéralisée .....qsp. | 100 |
| MA* désigne Matière Active | |

La composition ci-dessus a été appliquée pendant 30 minutes sur des mèches de cheveux gris naturels à 90 % de blancs. Les mèches de cheveux ont ensuite été rincées, lavées avec un shampooing standard puis séchées.

Les mèches ont été teintes dans la nuance suivante :

| **Exemple** | **Nuance obtenue** |
|---|---|
| 3 | Violet puissant |

## Revendications

1. Composition pour la teinture des fibres kératiniques et en particulier des fibres kératiniques humaines telles que les cheveux, renfermant dans un milieu approprié pour la teinture, (i)au moins composé choisi parmi ceux de formules suivantes : 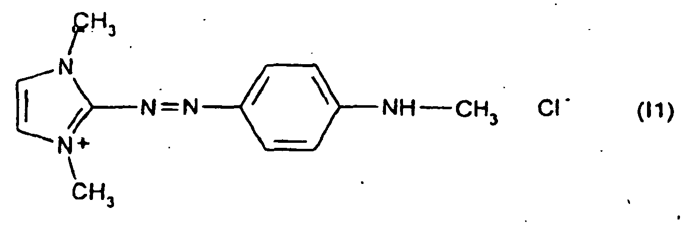 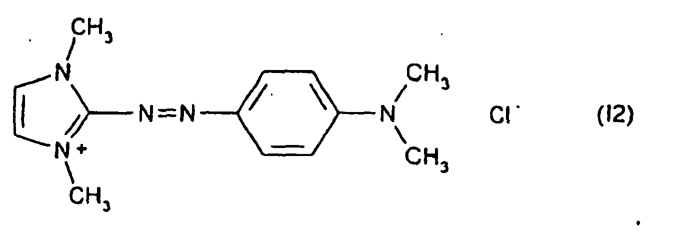 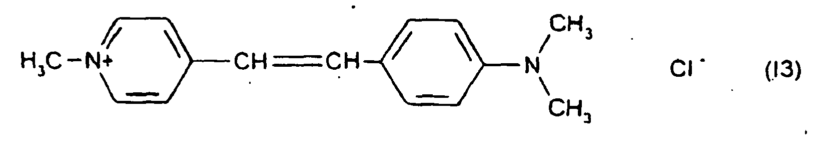 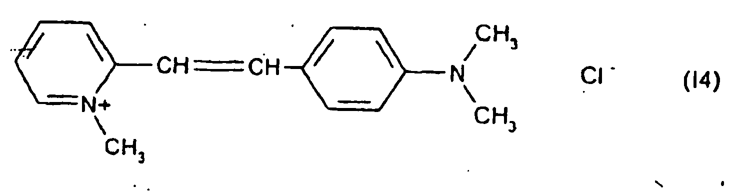 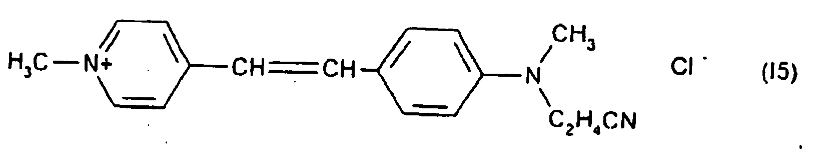 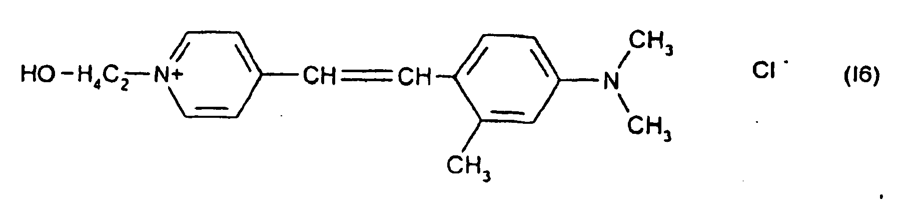 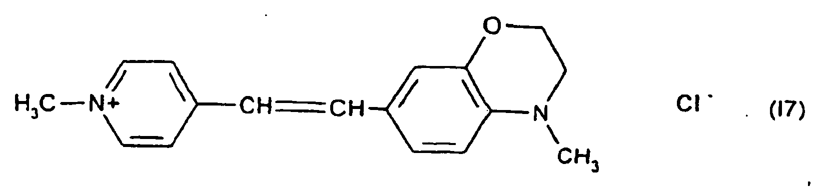 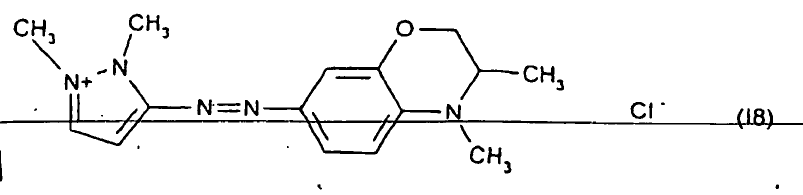 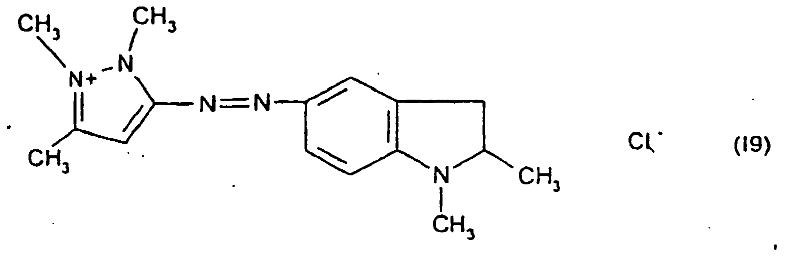 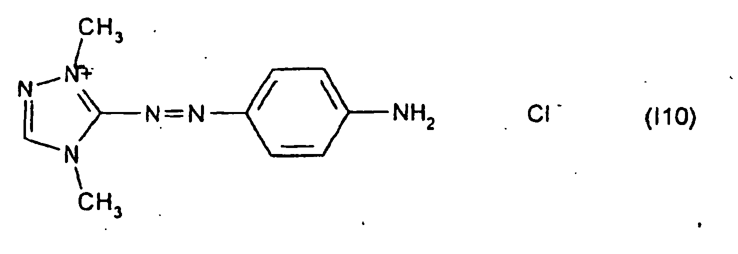 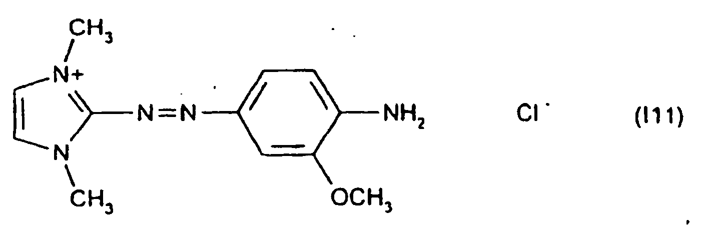 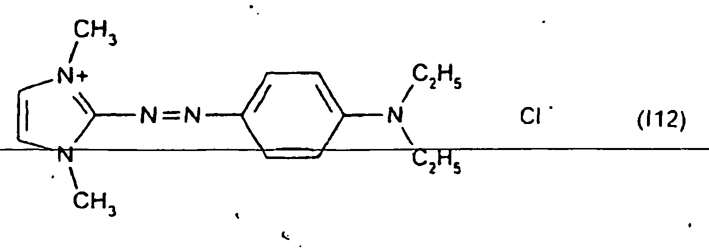 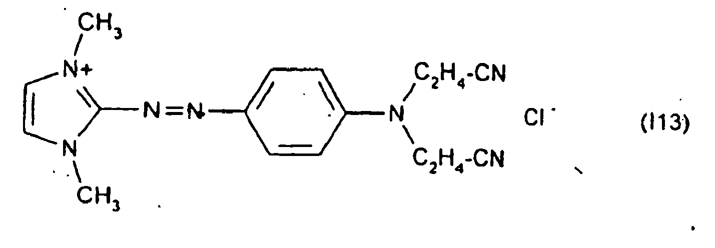 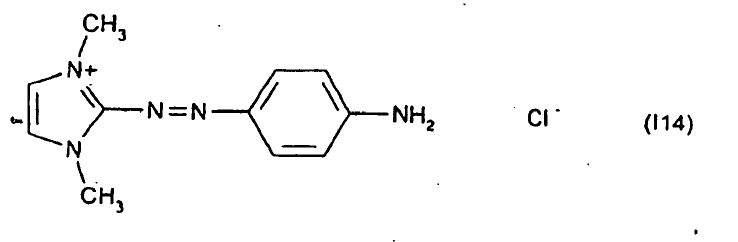 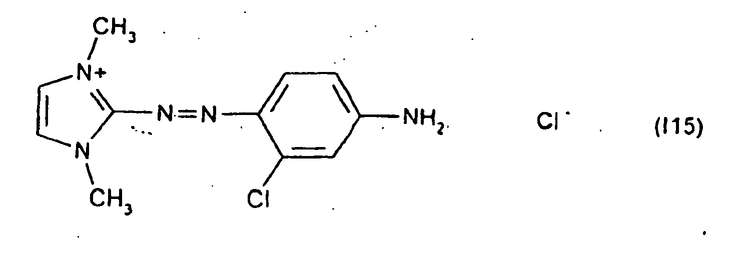 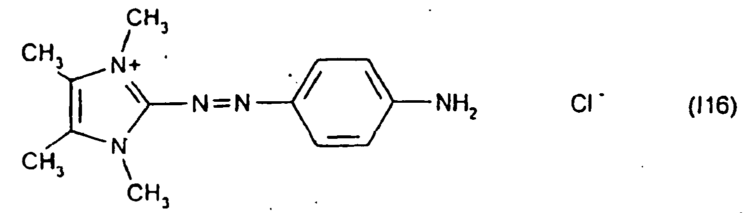 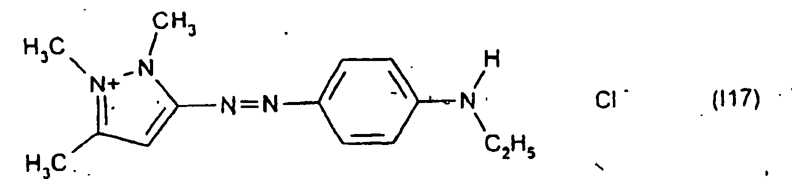 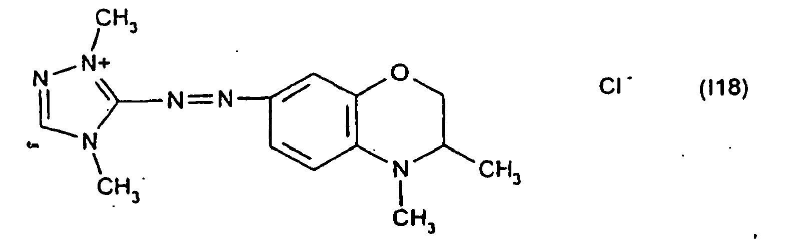 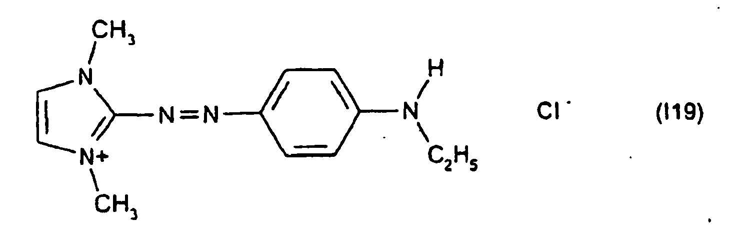 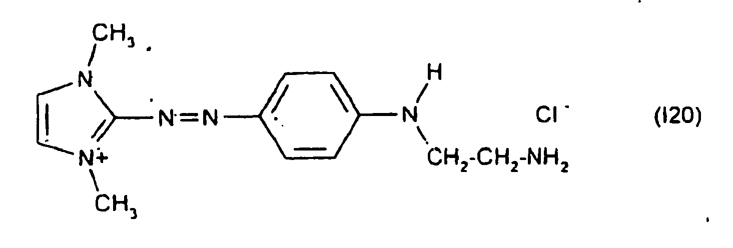 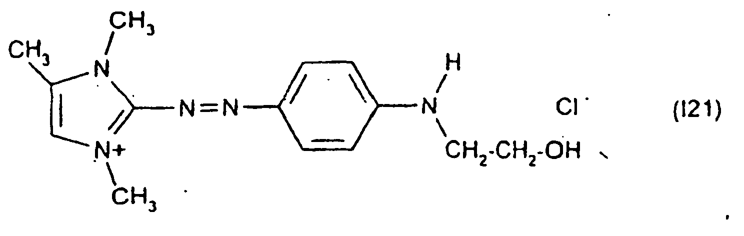 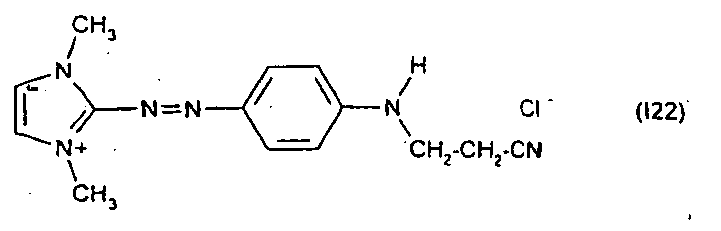 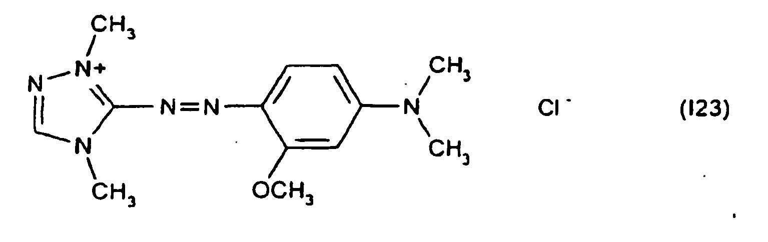 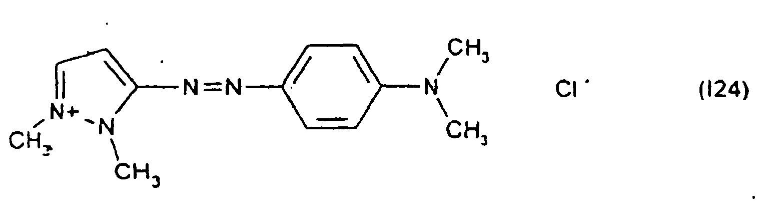 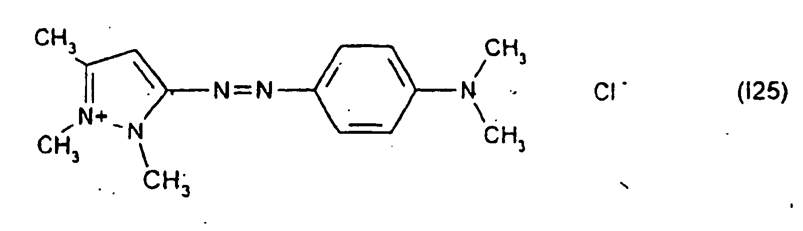 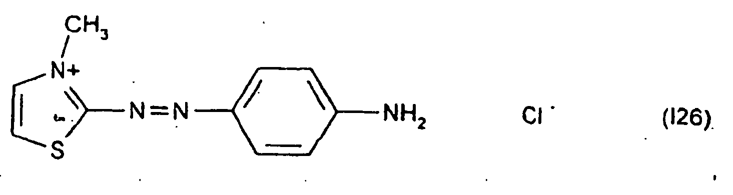 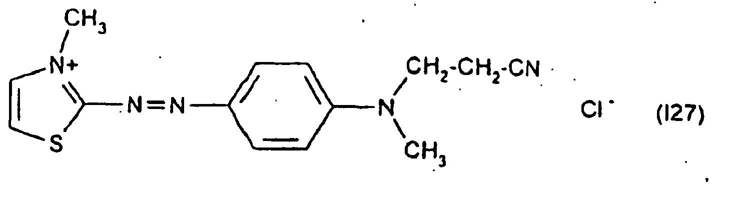 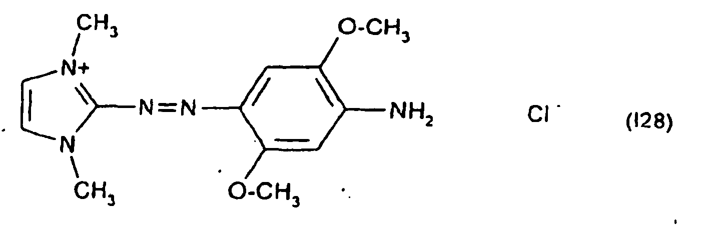 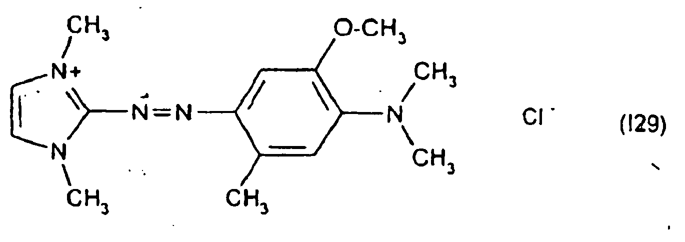 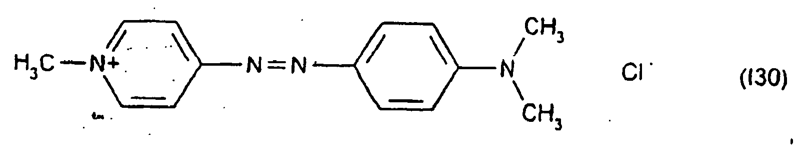 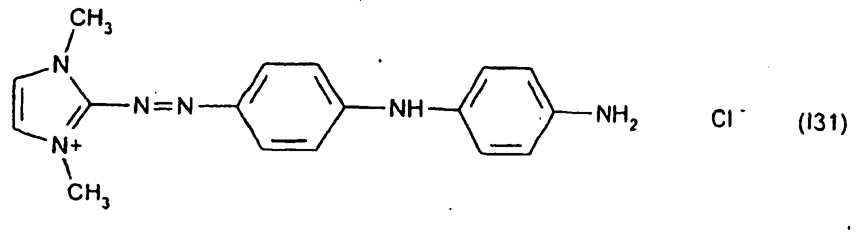 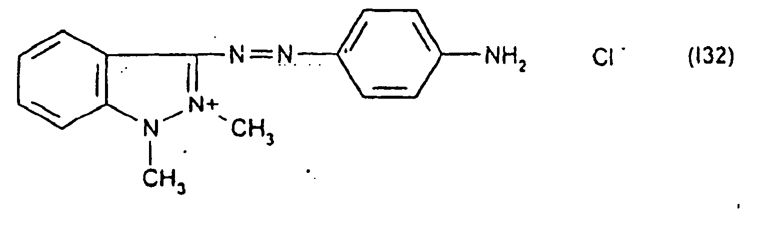 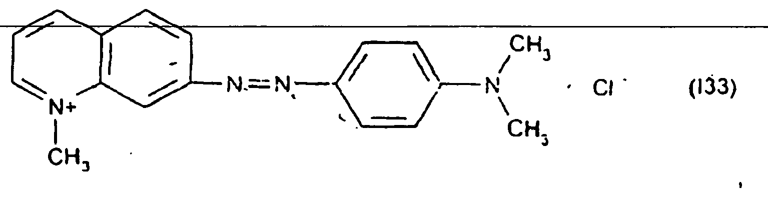 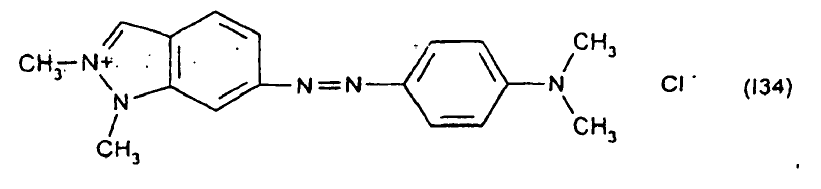 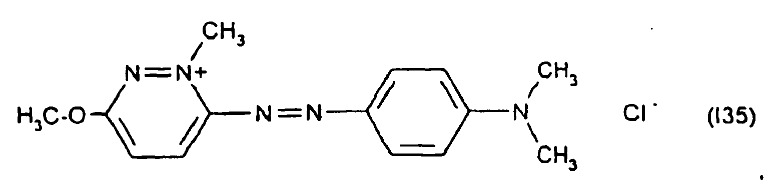 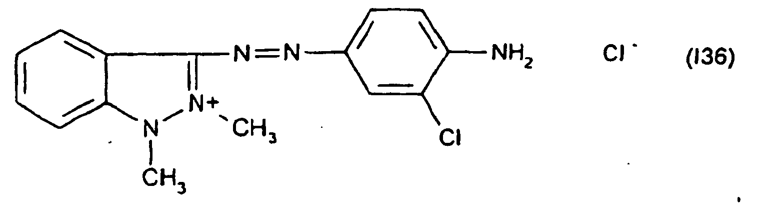 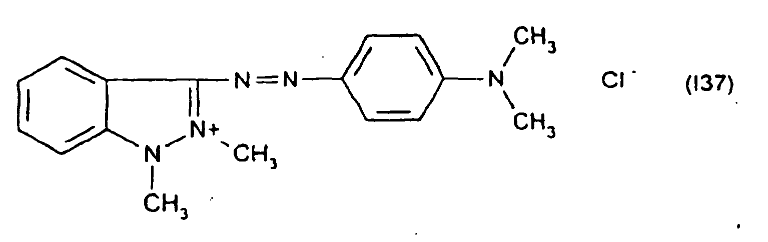 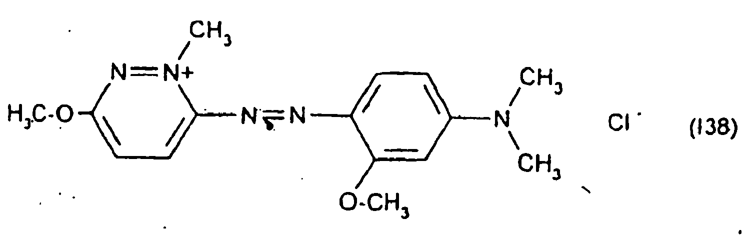 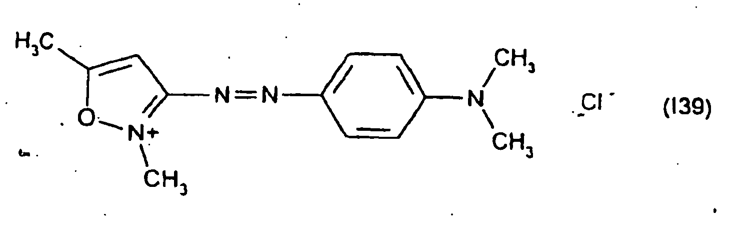 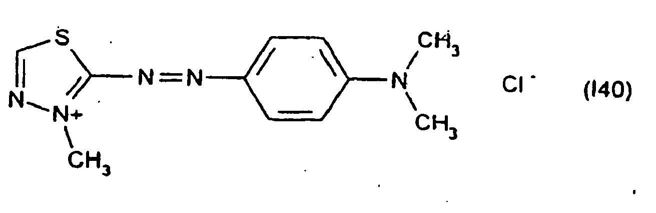 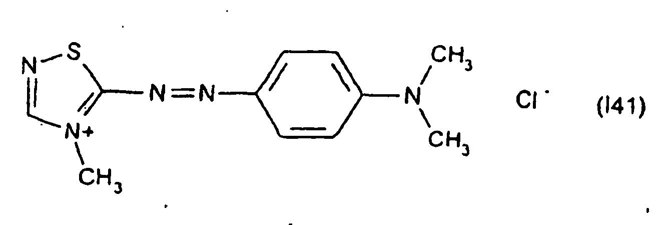 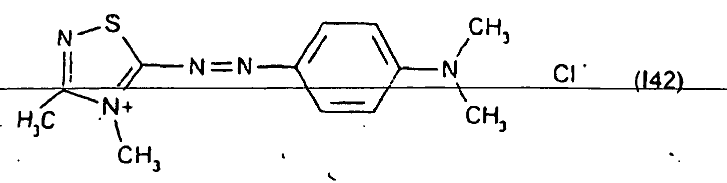 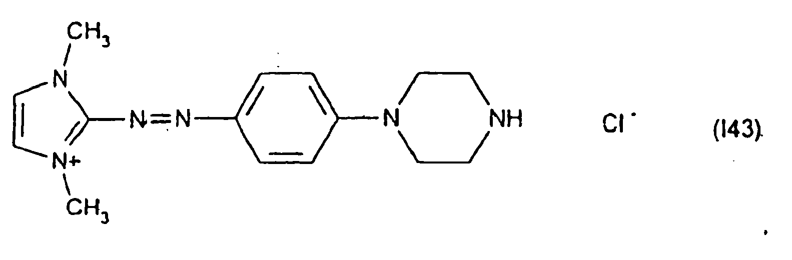 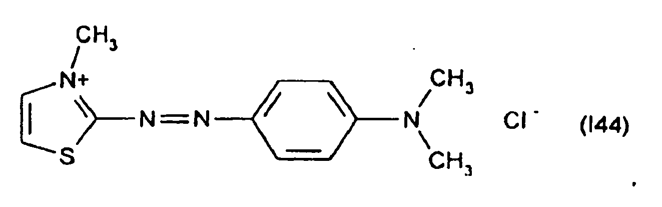 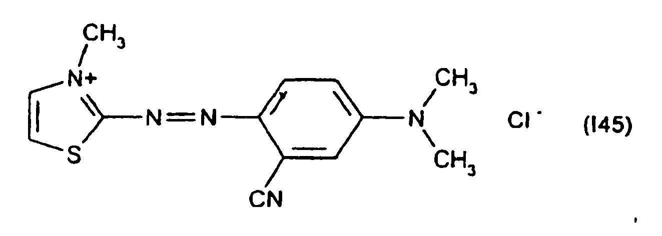 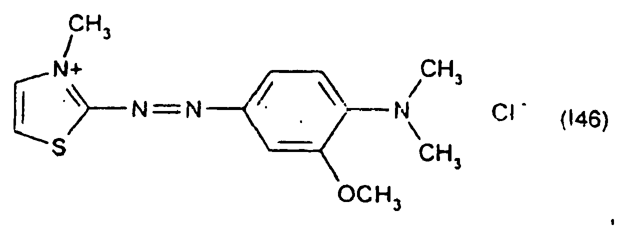 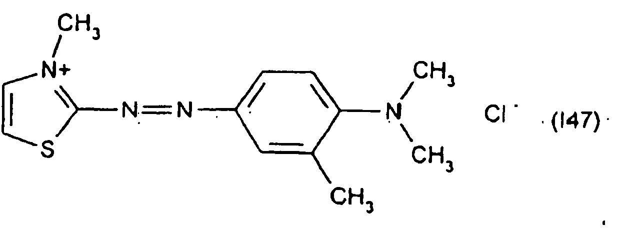 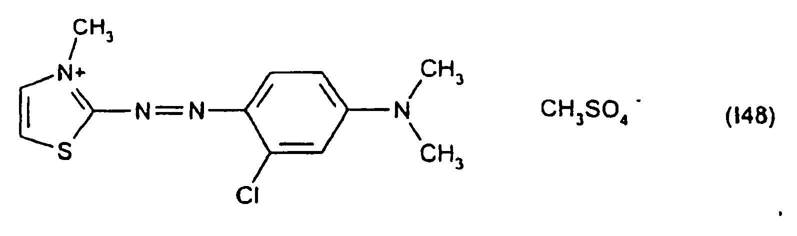 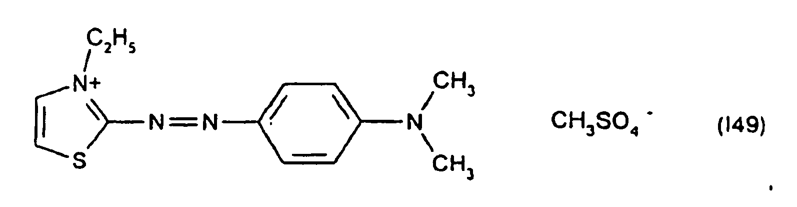 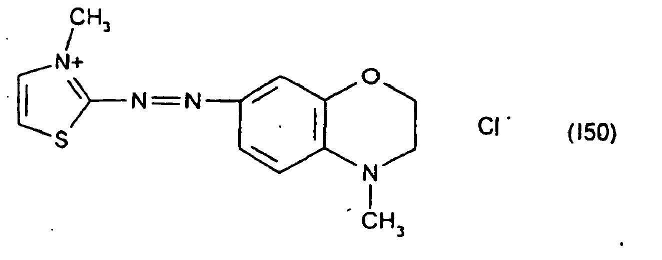 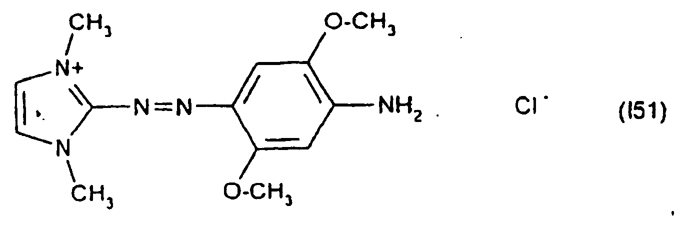 et 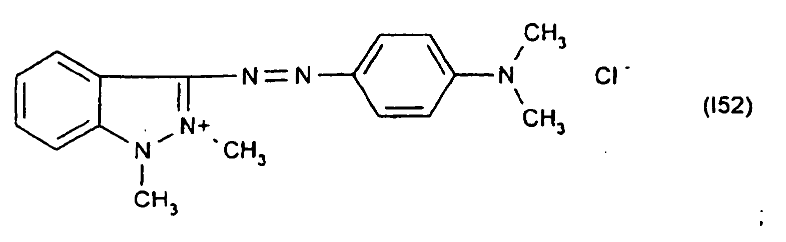 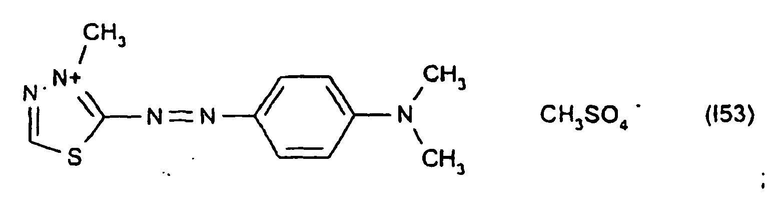 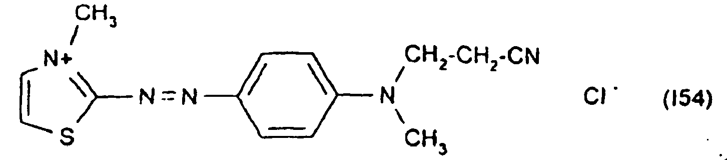 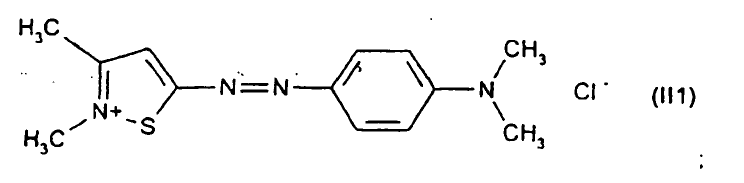 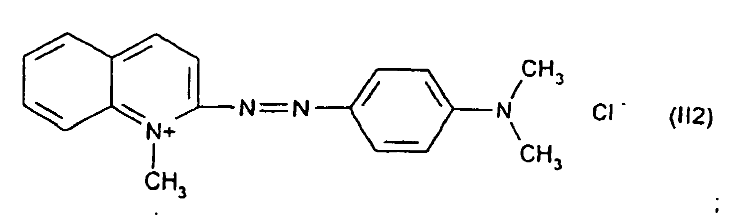 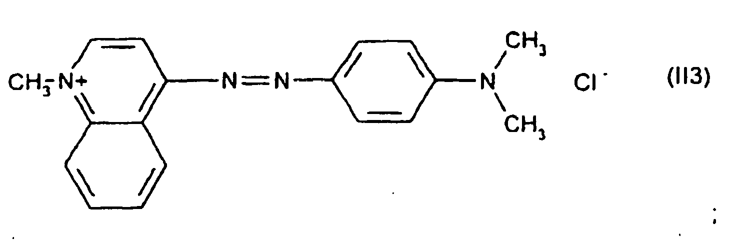 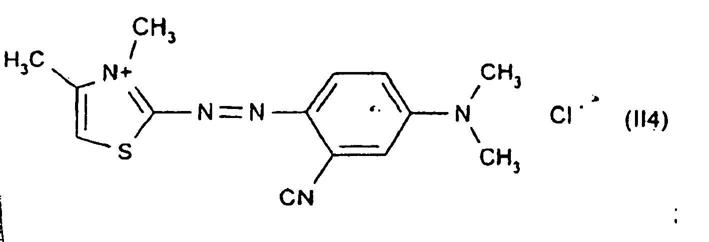 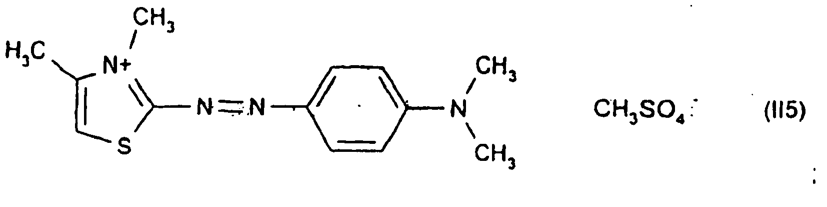 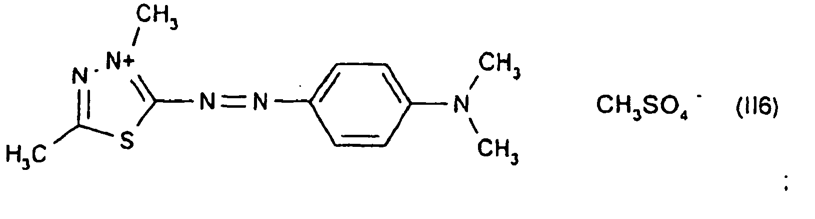 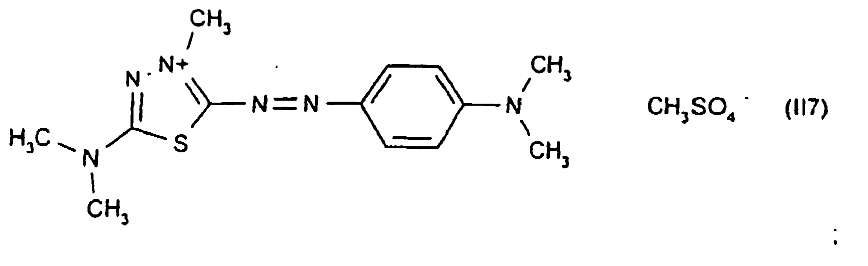 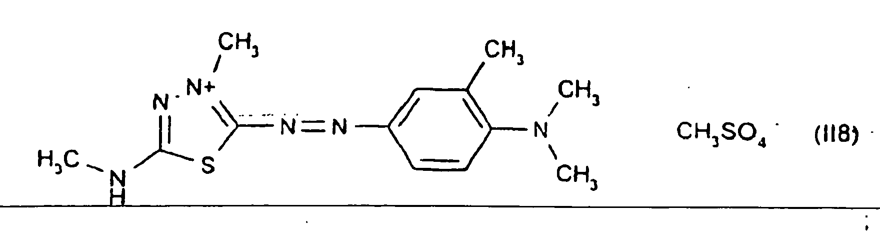 et 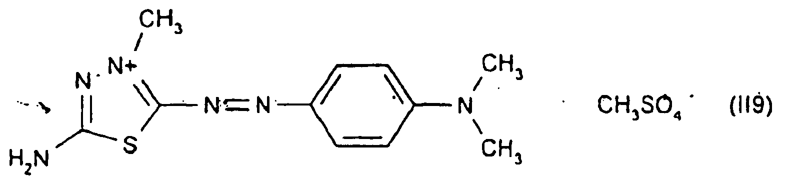 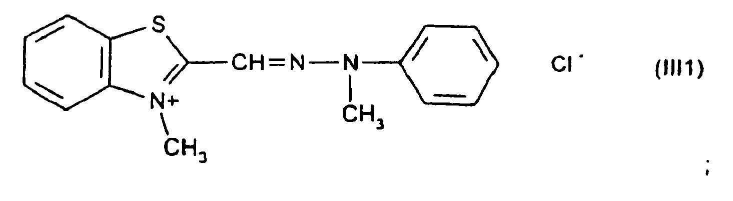 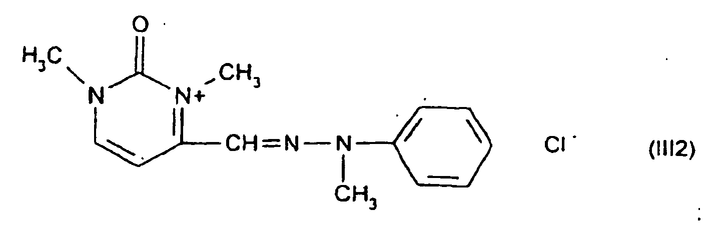 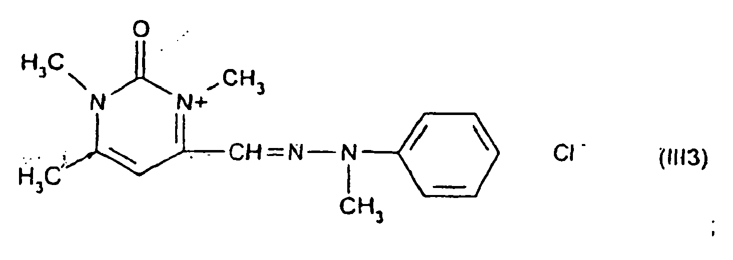 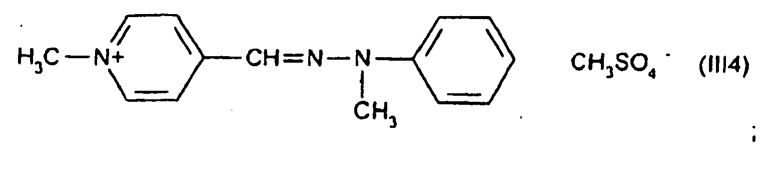 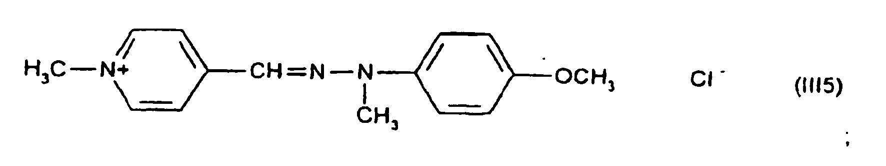 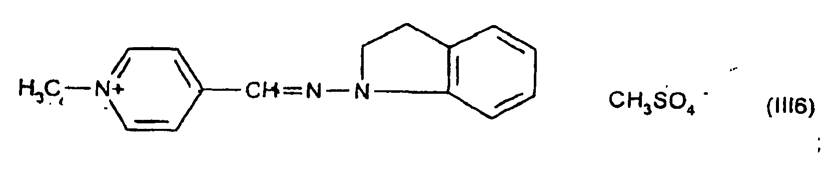 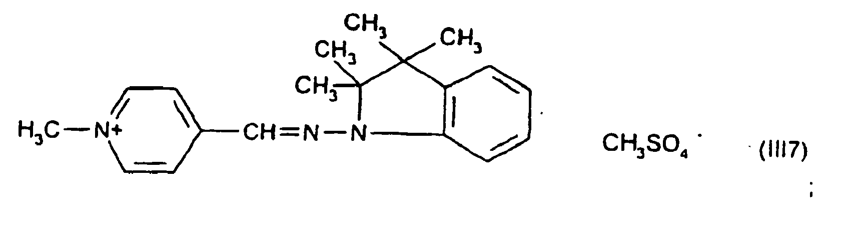 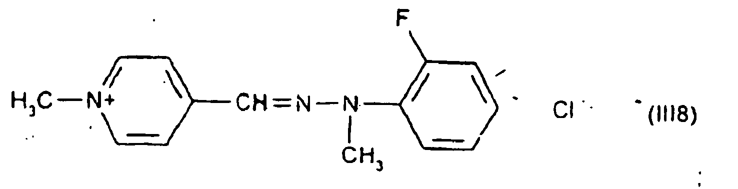 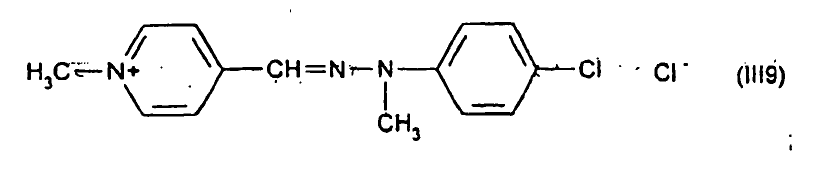 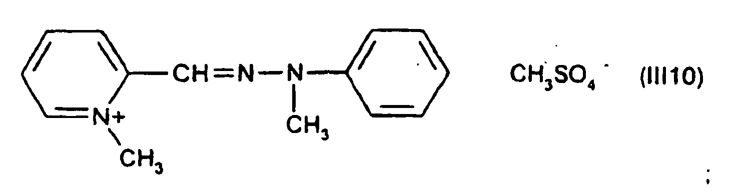 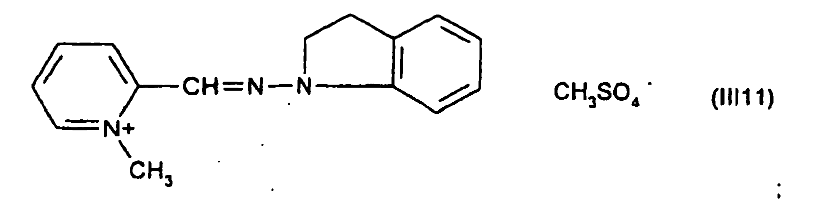 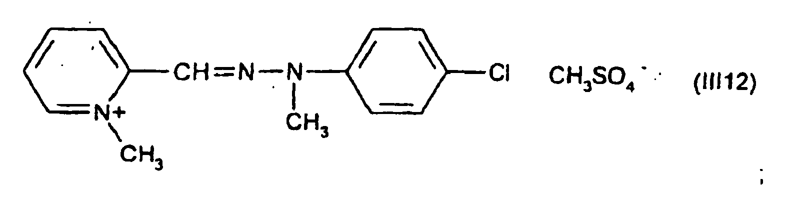 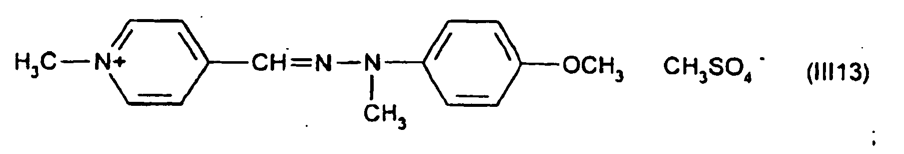 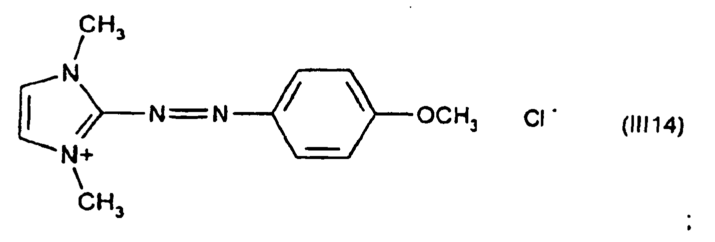 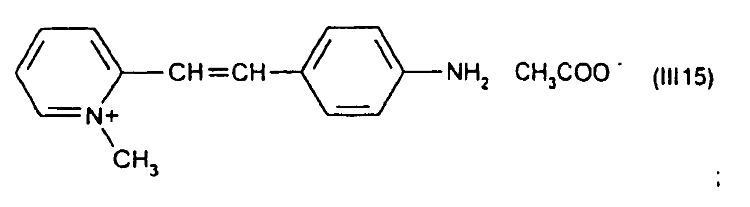 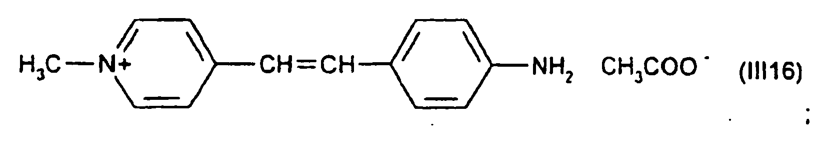 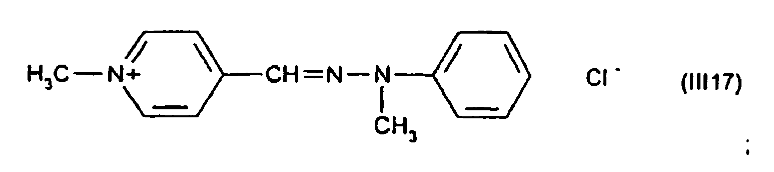 et 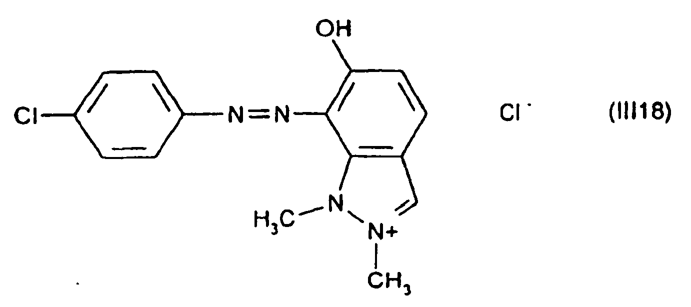 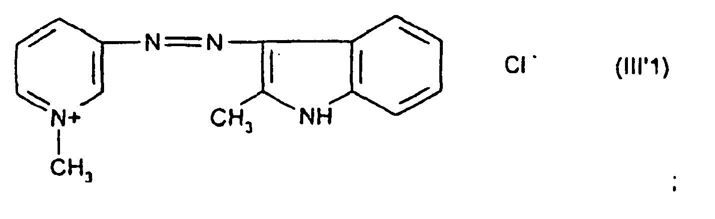 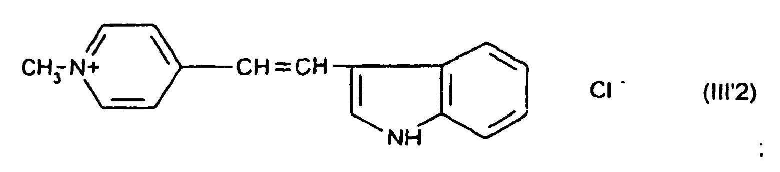 et 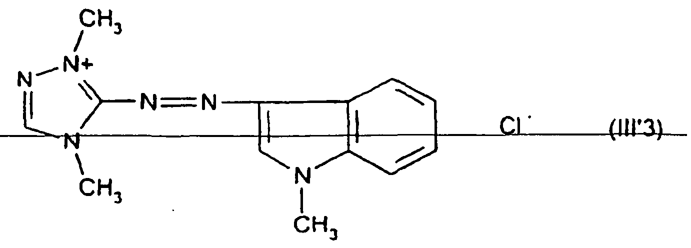 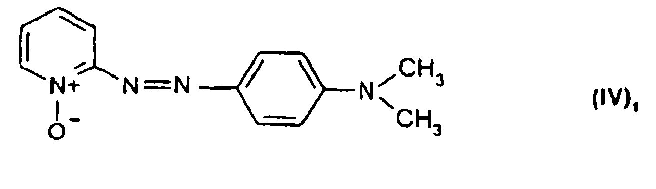 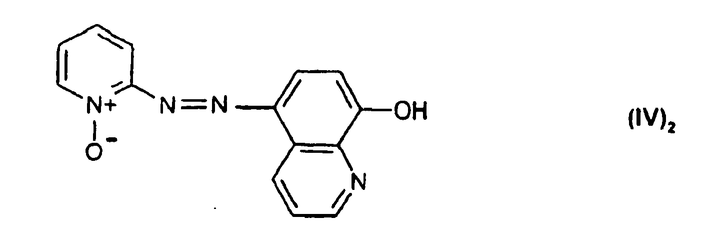 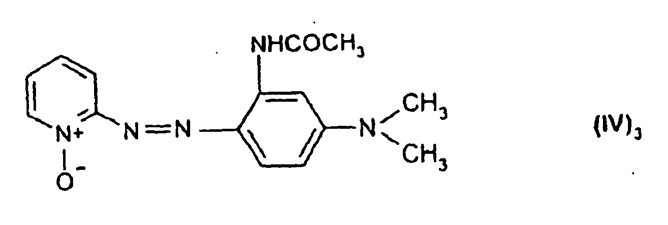 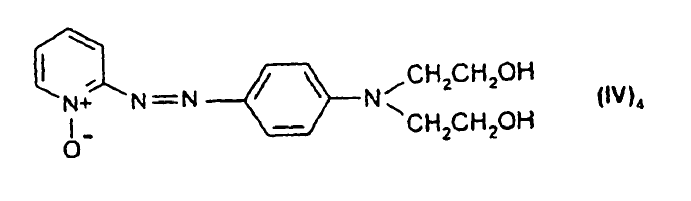 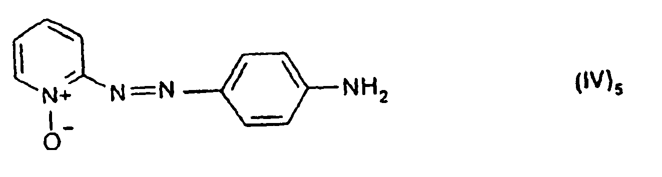 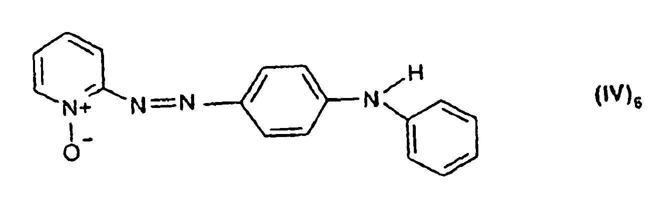 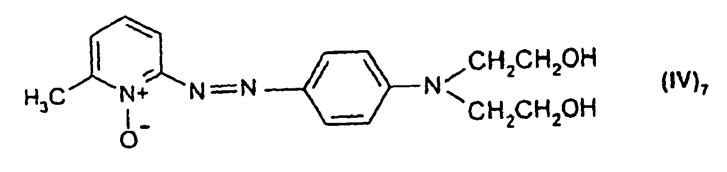 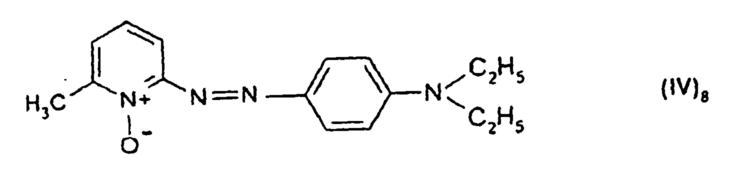 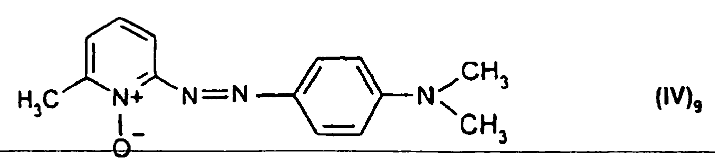 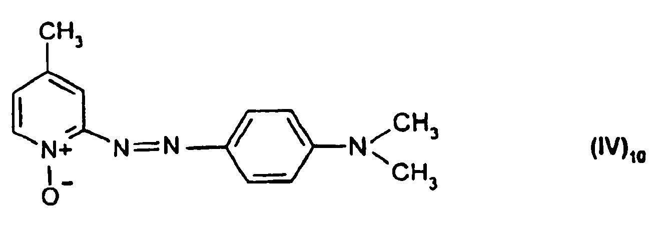 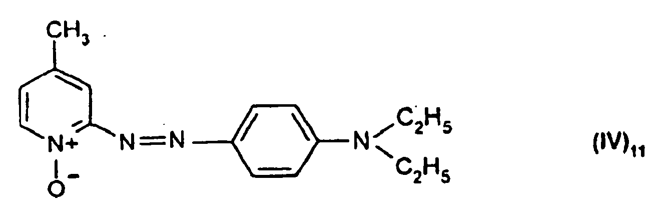 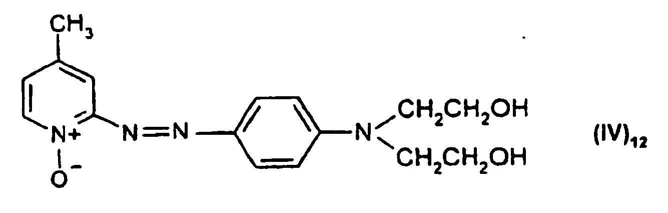 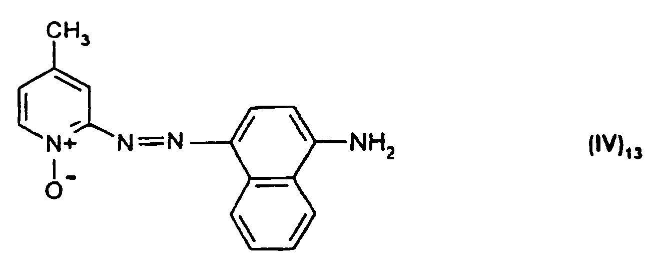 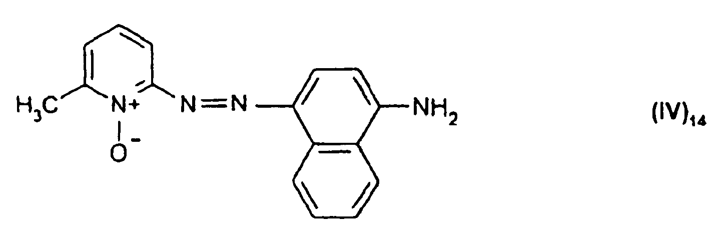 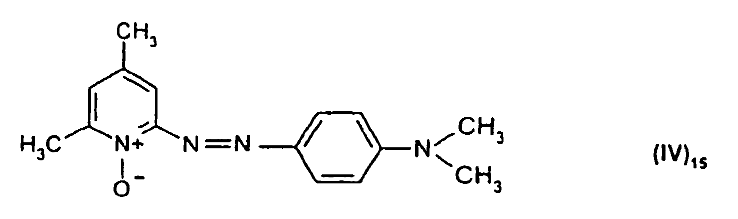 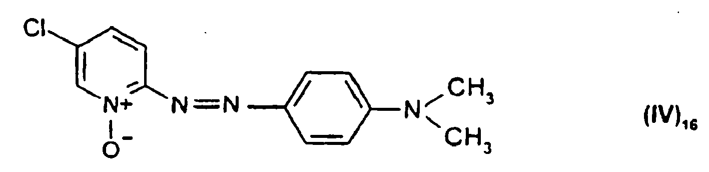 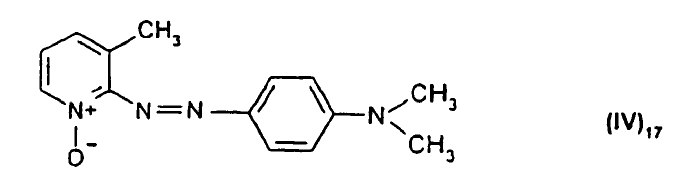 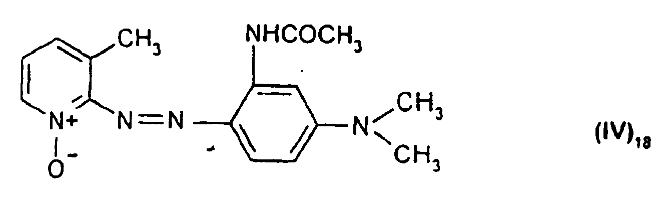 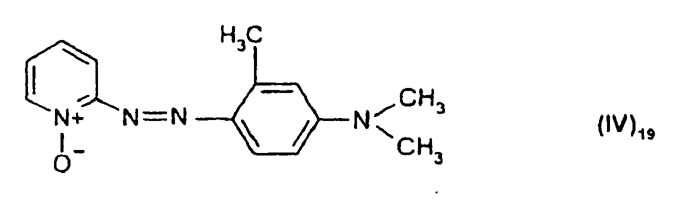 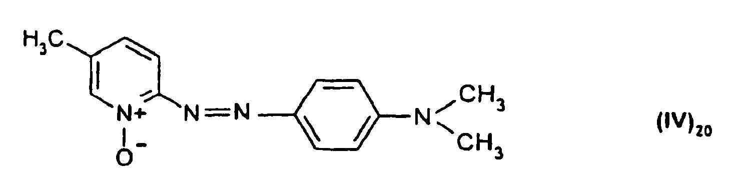 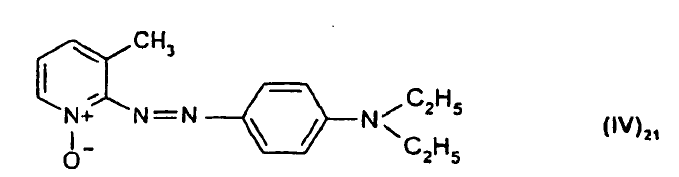 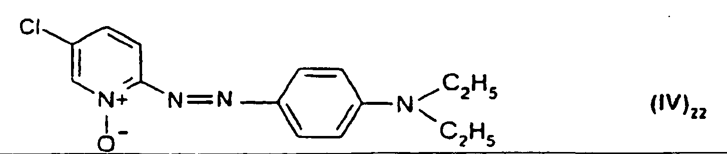 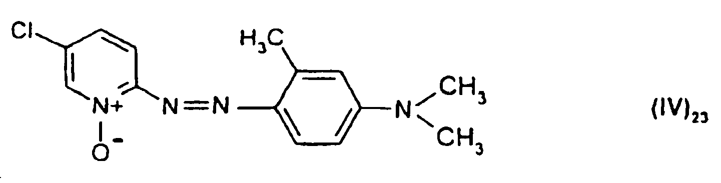 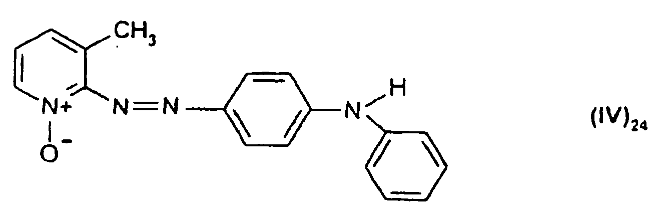 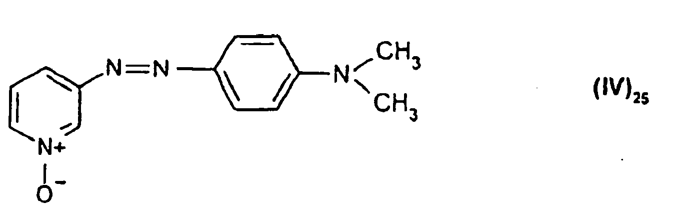 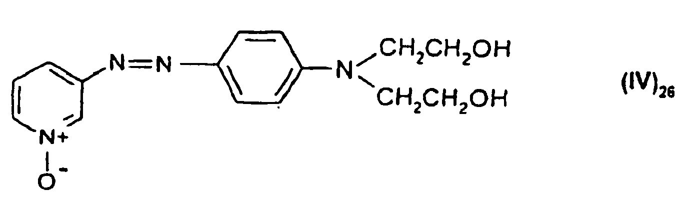 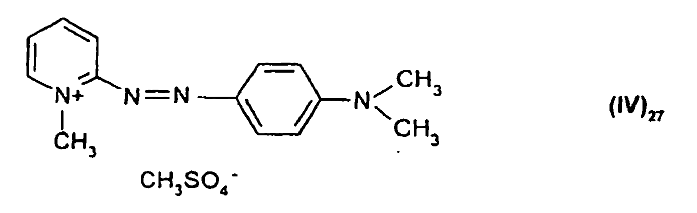 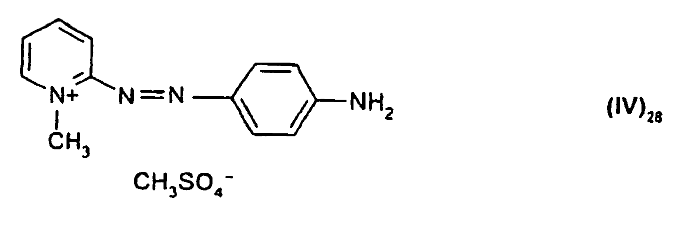 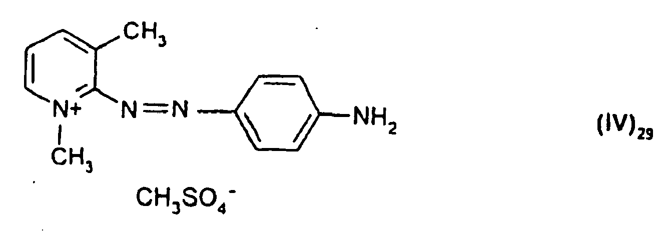 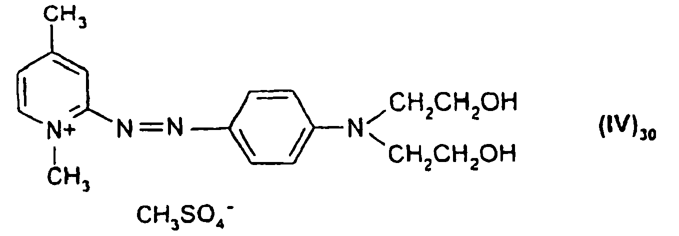 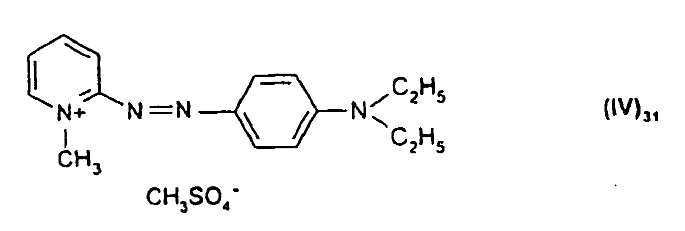 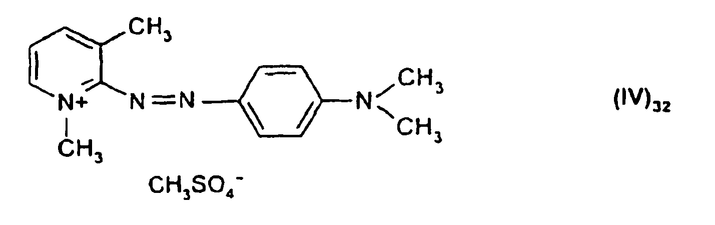 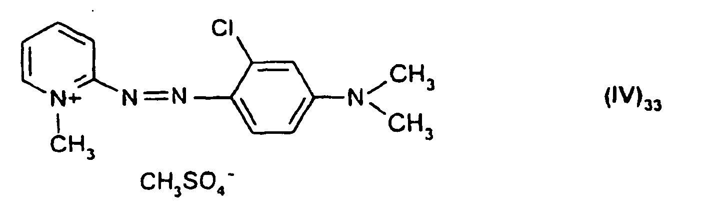 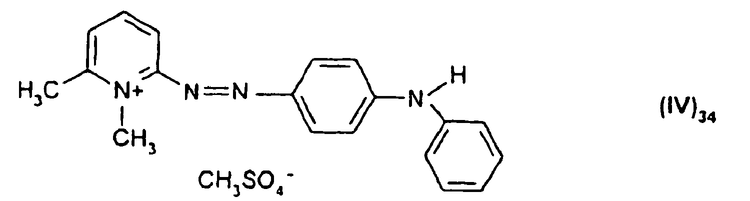 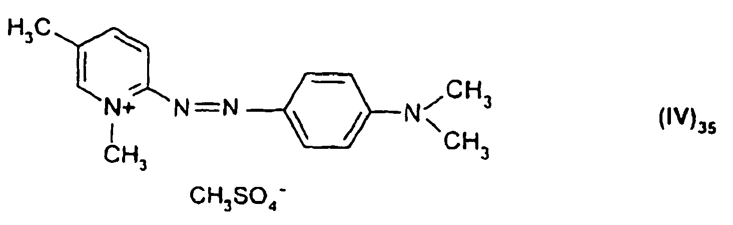 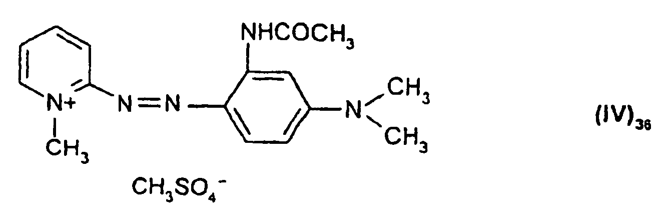 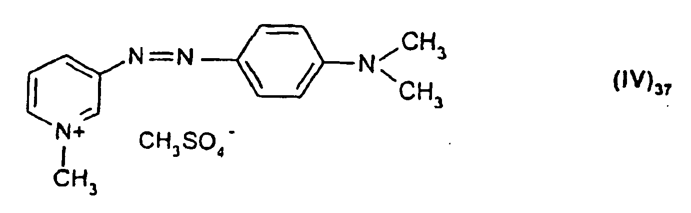 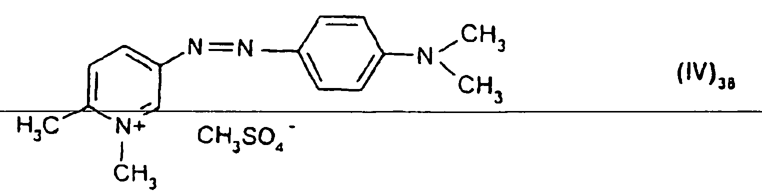 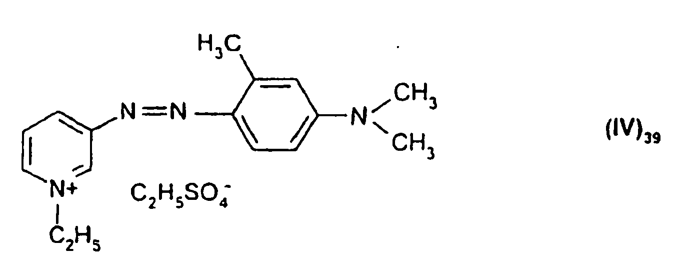 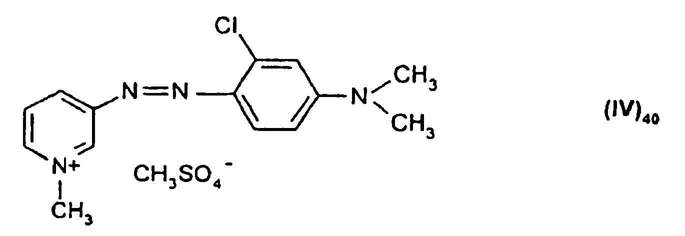 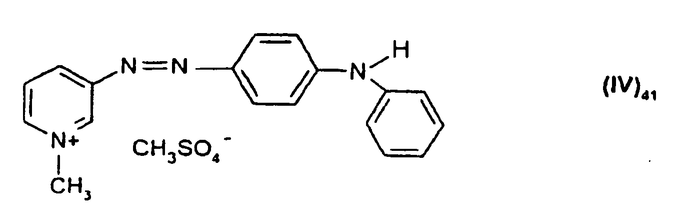 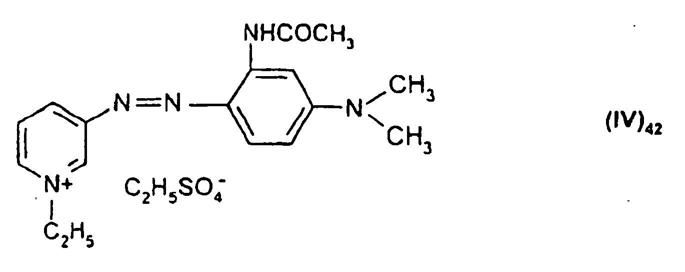 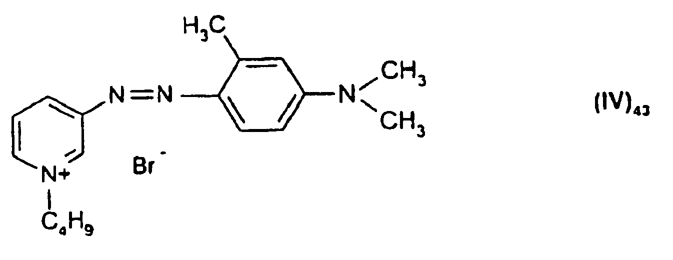 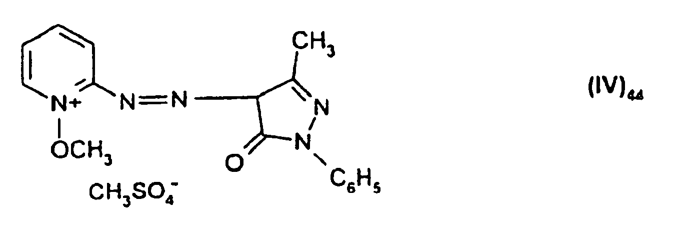 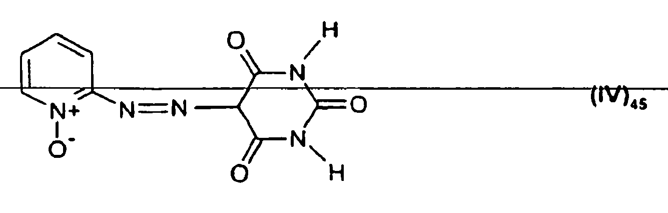 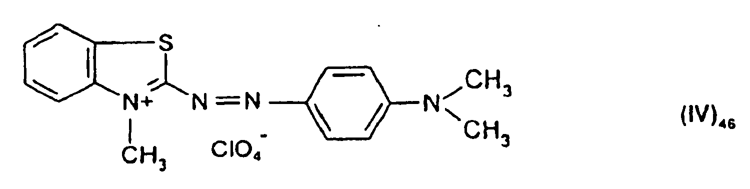 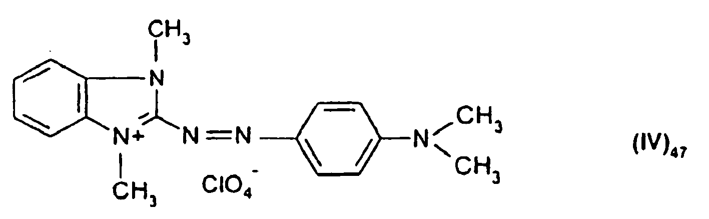 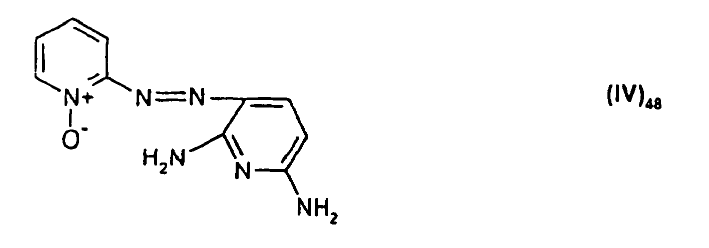 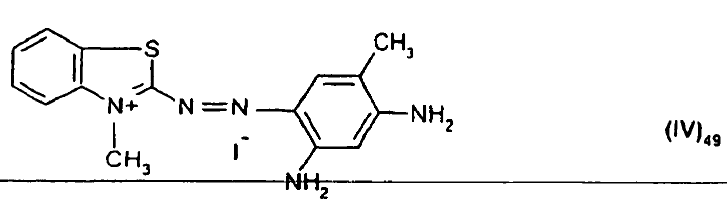 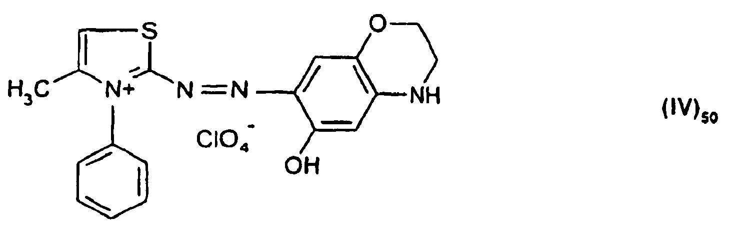 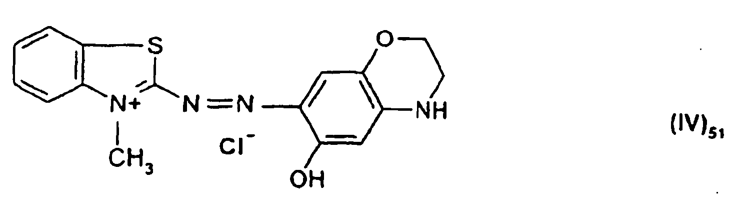 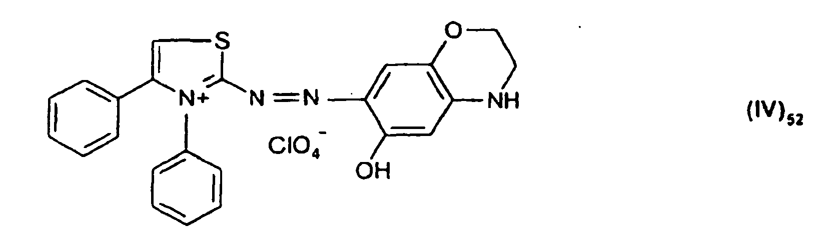 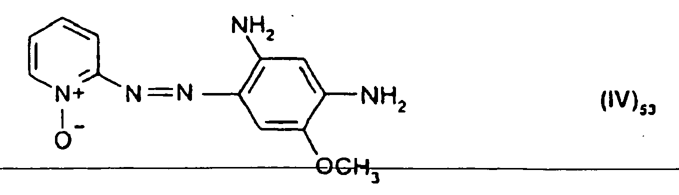 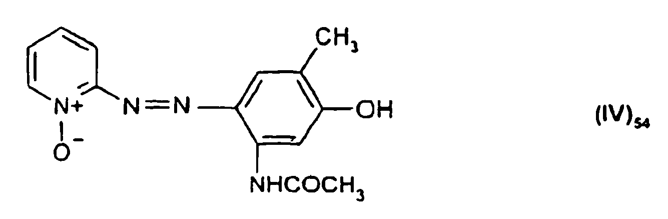 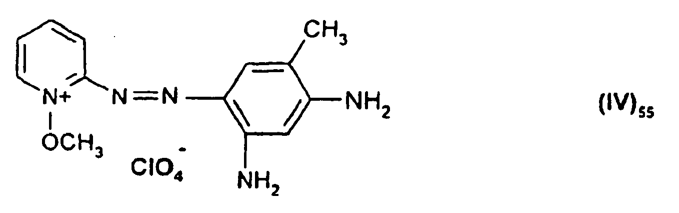 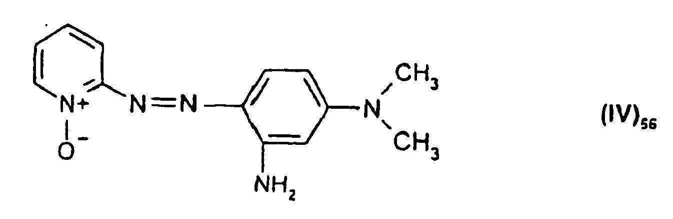 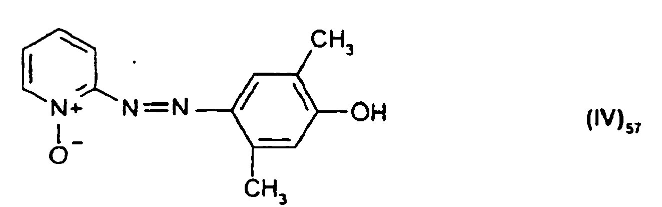 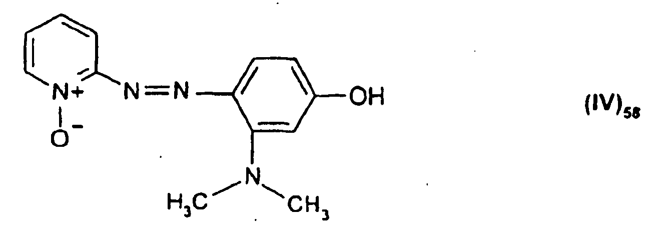 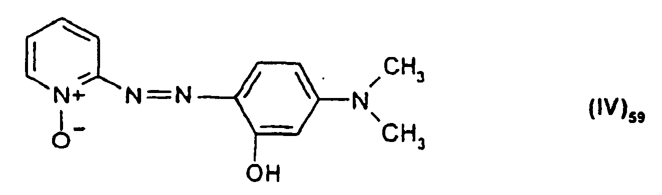 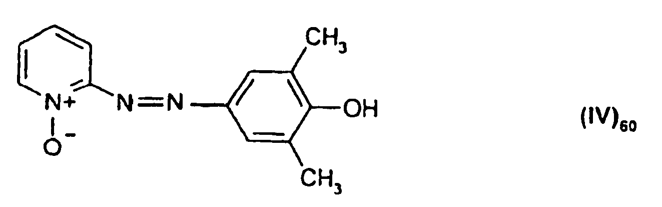 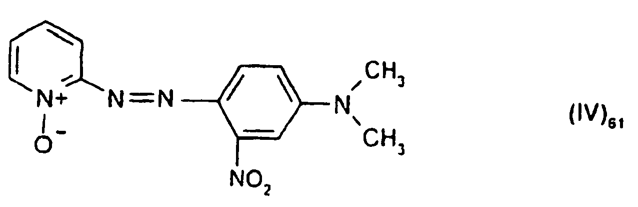 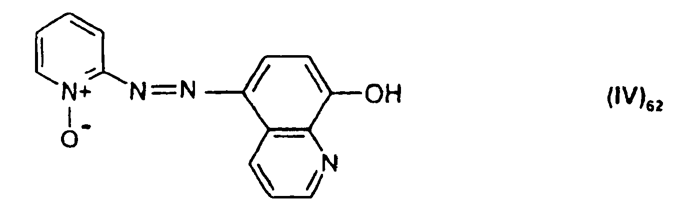 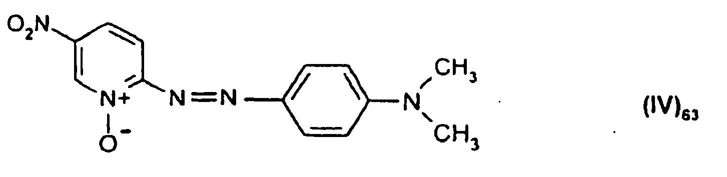 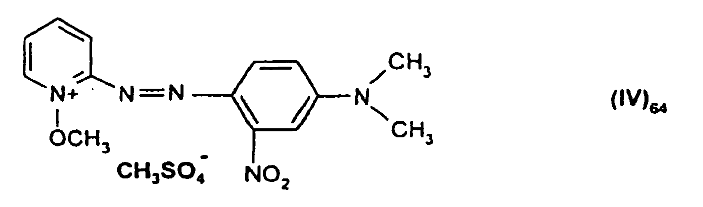 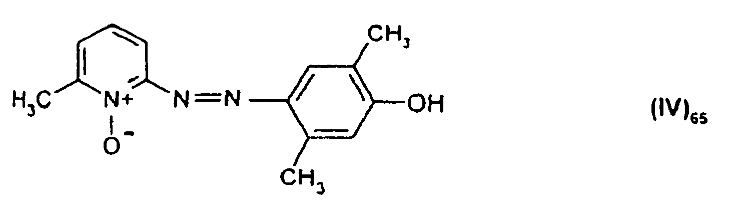 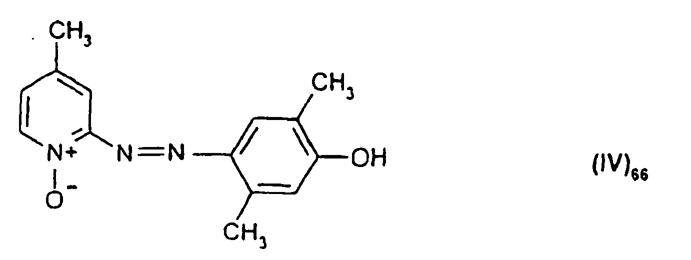 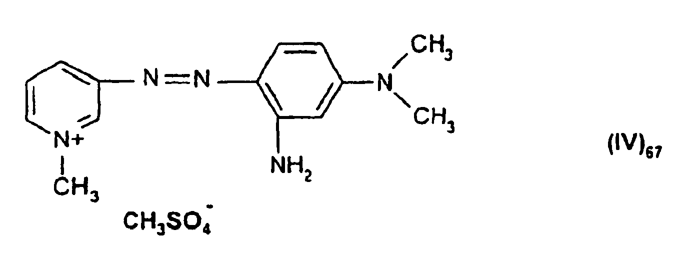 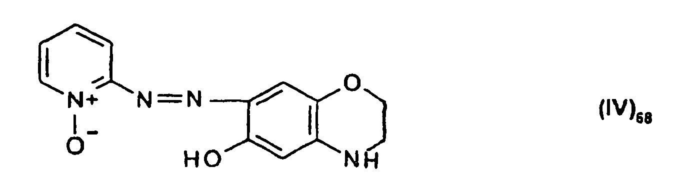 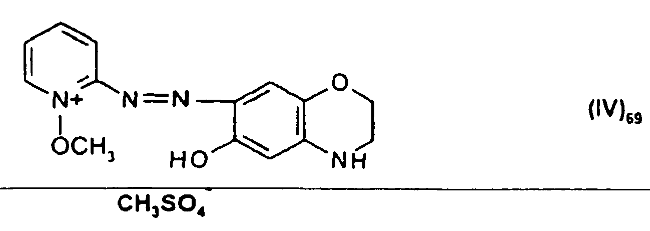 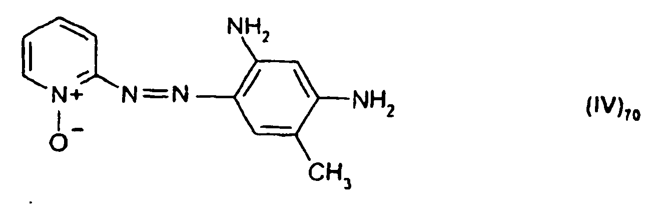 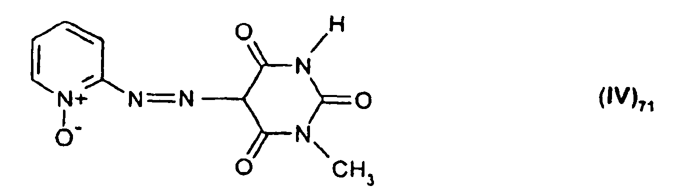 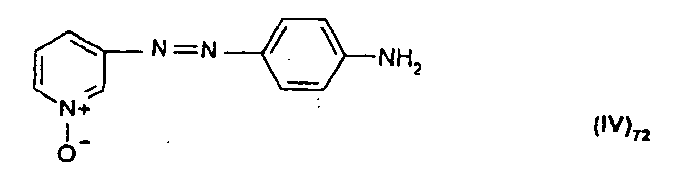 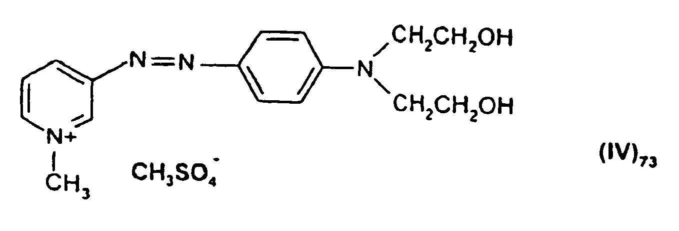 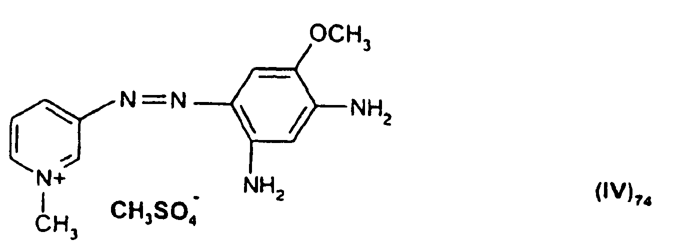 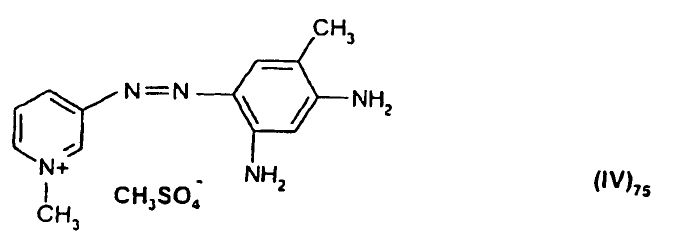 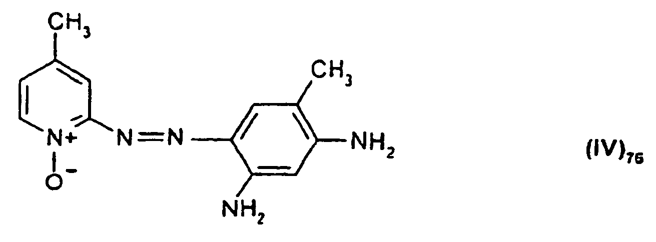 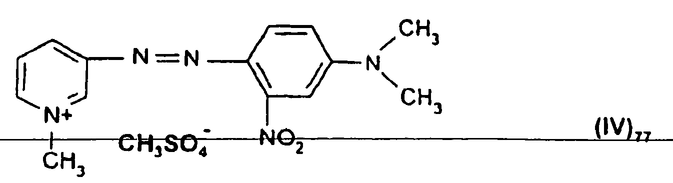 ladite composition étant **caractérisée par le fait qu'**elle contient en outre
(ii)au moins un agent tensio-actif anionique choisi dans le groupe comprenant :
**(ii)₁** - les acyliséthionates ;
**(ii)₂** - les acyltaurates ;
**(ii)₁₀**- leurs mélanges.

2. Composition selon la revendication 1, **caractérisée par le fait que** les colorants directs cationiques répondent aux structures (I1), (I2), (I14), et (I31).

3. Composition selon la revendication 1, **caractérisée par le fait que** les colorants directs cationiques de formule (III) sont choisis parmi les composés répondant aux structures (III4), (III5) et (III13).

4. Composition selon l'une quelconque des revendications précédentes, **caractérisée par le fait que** le ou les colorants directs cationiques de formules selon la revendication 1 représentent de 0,001 à 10 % en poids du poids total de la composition.

5. Composition selon la revendication 4, **caractérisée par le fait que** le ou les colorants directs cationiques de formules selon la revendication 1 représentent de 0,005 à 5 % en poids du poids total de la composition.

6. Composition selon l'une quelconque des revendications précédentes, **caractérisée par le fait que** l'agent tensio-actif anionique (ii) de type acyliséthionate **(ii)₁** et acyltaurate **(ii)₂** correspond à la formule générale :
R¹-CH₂-CH₂-SO₃⁻ M ⁺ (V)
dans laquelle,
R¹ désigne un groupe R²COO ou un groupe R²CONR³, avec R² désignant un groupe aliphatique linéaire ou ramifié, saturé ou insaturé en C₈-C₃₀ et R³ désignant un atome d'hydrogène ou un radical alkyle en C₁-C₄ et,
où M désigne H, ammonium, Na ou K ou un reste d'amine organique.

7. Composition selon l'une quelconque des revendications précédentes, **caractérisée par le fait que** le ou les agents tensio-actifs anioniques représentent de 0,05 à 30 % en poids du poids total de la composition.

8. Composition selon la revendication 7, **caractérisée par le fait que** le ou les agents tensio-actifs anioniques représentent de 0,1 à 15 % en poids du poids total de la composition.

9. Composition selon l'une quelconque des revendications précédentes, **caractérisée par le fait que** le milieu approprié pour la teinture (ou support) est constitué par de l'eau ou par un mélange d'eau et d'au moins un solvant organique.

10. Composition selon l'une quelconque des revendications précédentes, **caractérisée par le fait qu'**elle présente un pH compris entre 2 et 11, et de préférence entre 5 et 10.

11. Composition selon l'une quelconque des revendications précédentes, **caractérisée par le fait qu'**elle est destinée à la teinture d'oxydation et qu'elle contient une ou plusieurs bases d'oxydation choisie parmi les paraphénylènediamines, les bis-phënylallcylènediamines, les para-aminophénols, les ortho-aminophénols et les bases hétérocycliques.

12. Composition selon la revendication 11, **caractérisée par le fait que** la ou les bases d'oxydation représentent 0,0005 à 12 % en poids du poids total de la composition tinctoriale.

13. Composition selon la revendication 12, **caractérisée par le fait que** la ou les bases d'oxydation représentent 0,005 à 6 % en poids du poids total de la composition tinctoriale.

14. Composition selon l'une quelconque des revendications 11 à 13, **caractérisée par le fait qu'**elle renferme un ou plusieurs coupleurs choisis parmi les métaphénylènediamines, les méta-aminophénols, les métadiphénols et les coupleurs hétérocycliques.

15. Composition selon la revendication 14, **caractérisée par le fait que** le ou les coupleurs représentent de 0,0001 à 10 % en poids du poids total de la composition tinctoriale.

16. Composition selon la revendication 15, **caractérisée par le fait que** le ou les coupleurs représentent de 0,005 à 5 % en poids du poids total de la composition tinctoriale.

17. Composition selon l'une quelconque des revendications précédentes, **caractérisée par le fait qu'**elle est destinée à la teinture directe éclaircissante ou la teinture d'oxydation et qu'elle renferme alors au moins un agent oxydant.

18. Procédé de teinture des fibres kératiniques et en particulier des fibres kératiniques humaines telles que les cheveux, **caractérisé par le fait que** qu'on applique sur les fibres au moins une composition tinctoriale telle que définie à l'une quelconque des revendications 1 à 17, pendant un temps suffisant pour développer la coloration désirée, après quoi on rince, on lave éventuellement au shampooing, on rince à nouveau et on sèche.

19. Procédé de teinture des fibres kératiniques et en particulier des fibres kératiniques humaines telles que les cheveux, **caractérisé par le fait que** qu'on applique sur les fibres au moins une composition tinctoriale telle que définie à l'une quelconque des revendications 1 à 17, pendant un temps suffisant pour développer la coloration désirée, sans rinçage final.

20. Procédé de teinture des fibres kératiniques et en particulier des fibres kératiniques humaines telles que les cheveux, **caractérisé par le fait qu'**il comporte une étape préliminaire consistant à stocker sous forme séparée, d'une part, une composition (A1) comprenant, dans un milieu approprié pour la teinture, au moins un colorant direct cationique (i) tel que défini dans les revendications précédentes et au moins une base d'oxydation et, d'autre part, une composition (B1) renfermant, dans un milieu approprié pour la teinture, au moins un agent oxydant, puis à procéder à leur mélange au moment de l'emploi avant d'appliquer ce mélange sur les fibres kératiniques, la composition (A1) ou la composition (B1) contenant l'agent tensio-actif anionique (ii) tel que défini dans les revendications précédentes.

21. Procédé de teinture des fibres kératiniques et en particulier des fibres kératiniques humaines telles que les cheveux, **caractérisé par le fait qu'**il comporte une étape préliminaire consistant à stocker sous forme séparée, d'une part, une composition (A2) comprenant, dans un milieu approprié pour la teinture, au moins un colorant direct cationique (i) tel que défini dans les revendications précédentes et, d'autre part, une composition (B2) renfermant, dans un milieu approprié pour la teinture, au moins un agent oxydant, puis à procéder à leur mélange au moment de l'emploi avant d'appliquer ce mélange sur les fibres kératiniques, la composition (A2) ou la composition (B2) contenant l'agent tensio-actif anionique (ii) tel que défini dans les revendications précédentes.

22. Dispositif à plusieurs compartiments ou "kit" de teinture à plusieurs compartiments, **caractérisé par le fait qu'**un premier compartiment renferme la composition (A1) ou (A2) telle que définie à la revendication 20 ou 21 et un second compartiment renferme la composition (B1) ou (B2) telle que définie à la revendication 20 ou 21.

## Patentansprüche

1. Zusammensetzung zum Färben von Keratinfasern und insbesondere menschlichen Keratinfasern, wie dem Haar, die in einem zum Färben geeigneten Medium enthält: (i) mindestens eine Verbindung, die unter den Verbindungen der folgenden Formeln ausgewählt ist: 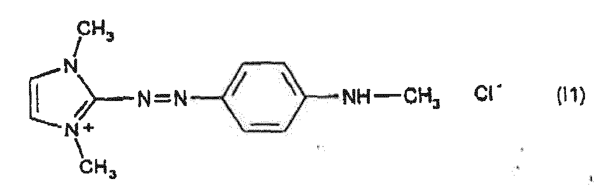 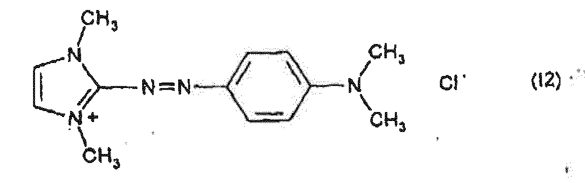 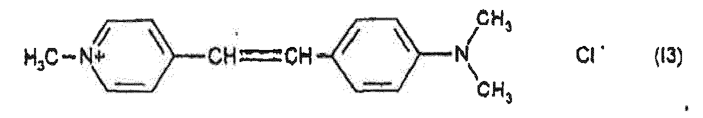 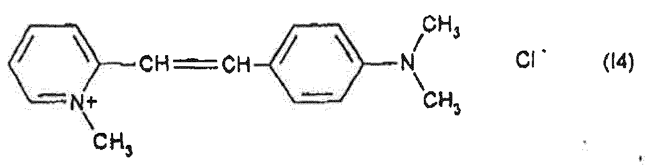 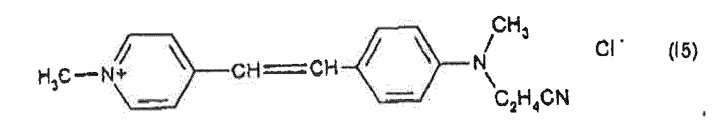 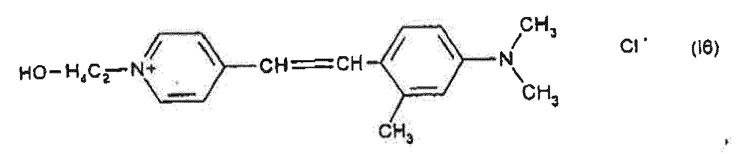 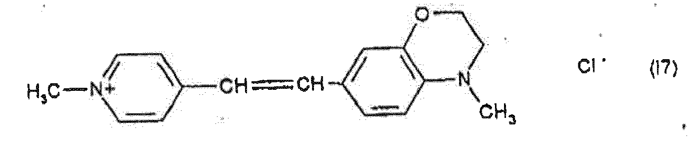 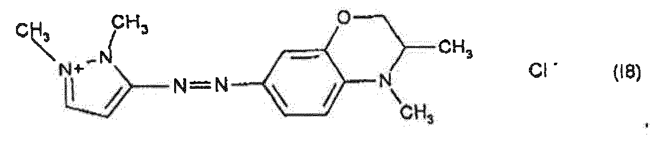 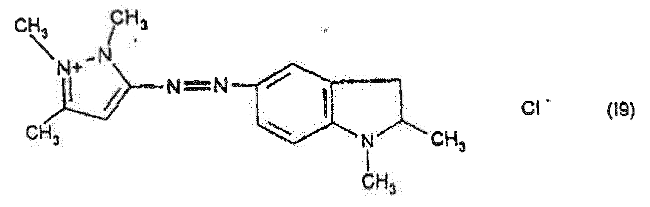 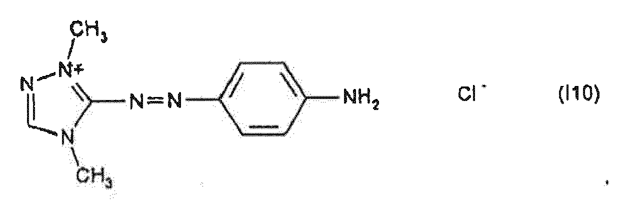 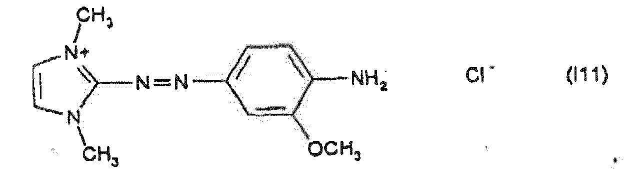 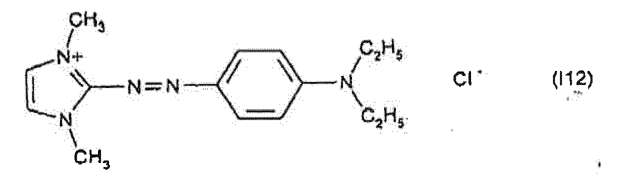 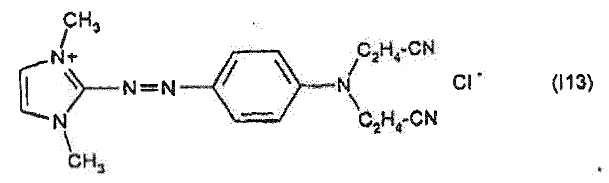 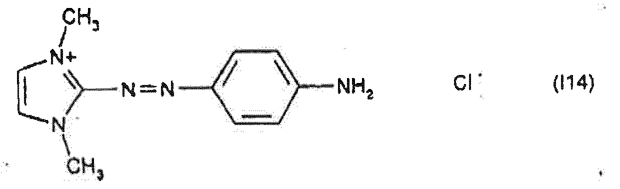 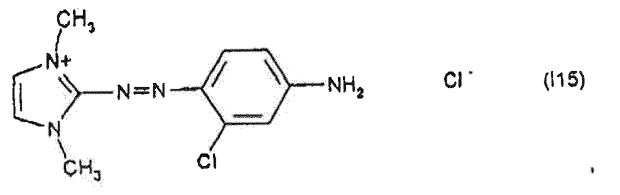 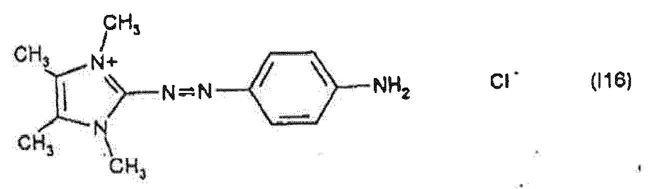 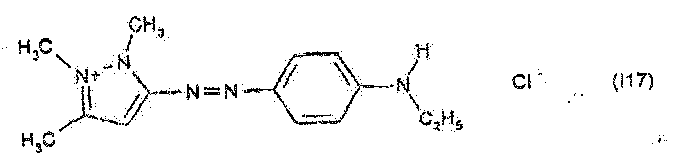 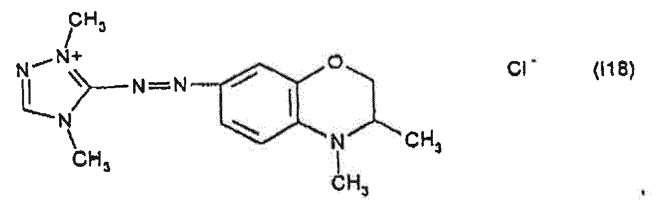 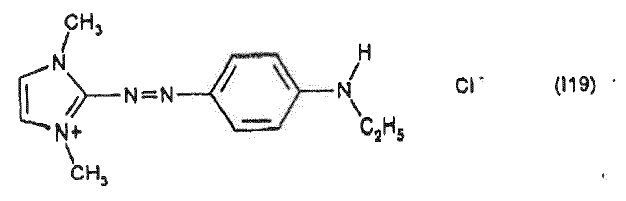 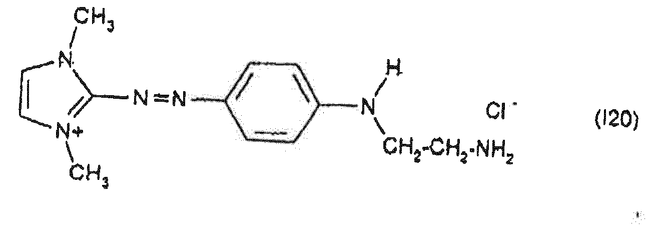 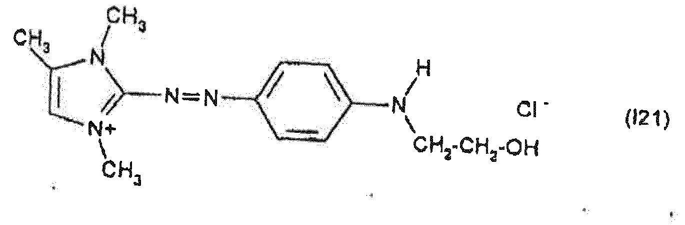 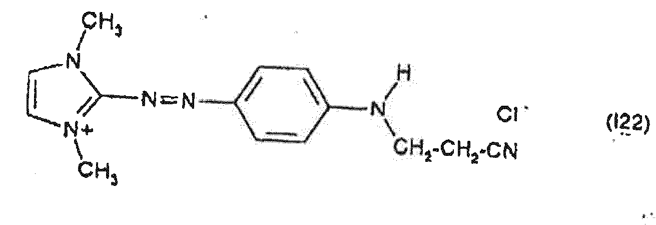 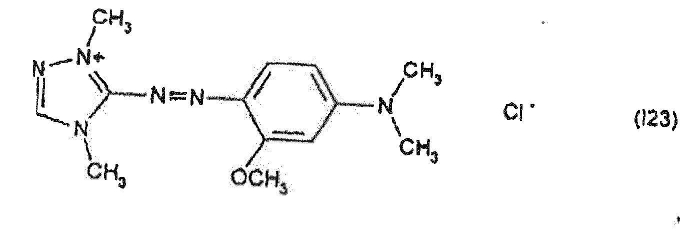 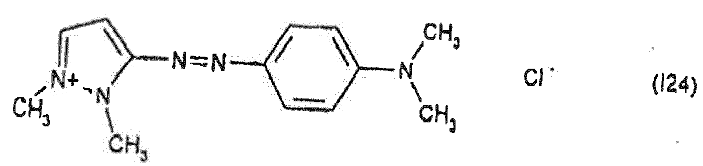 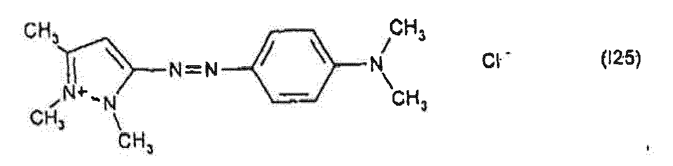 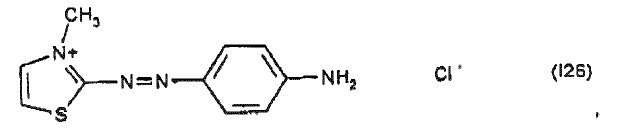 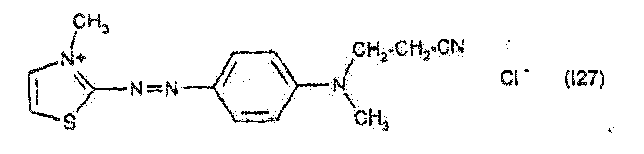 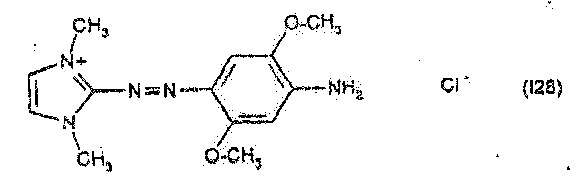 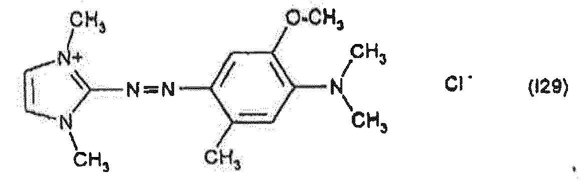 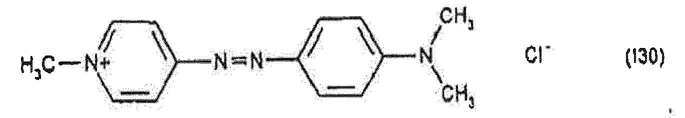 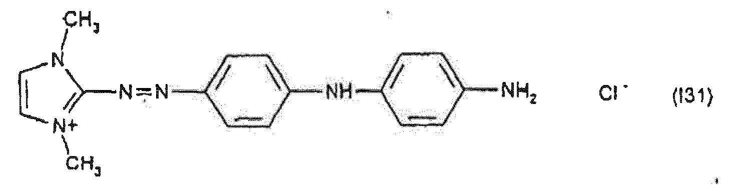 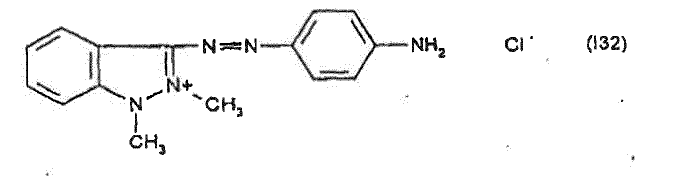 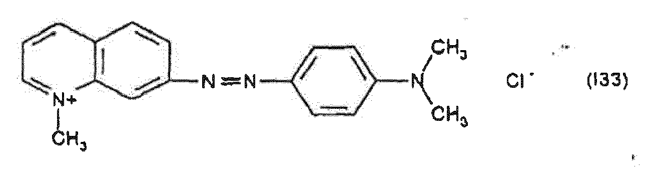 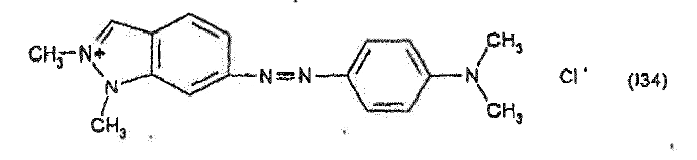 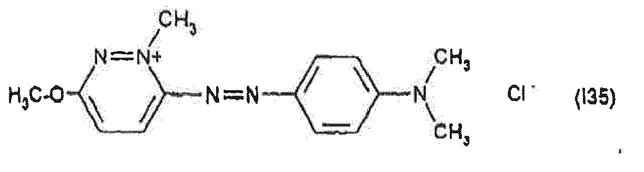 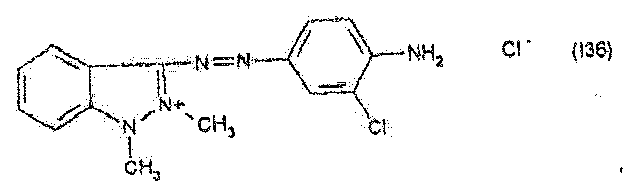 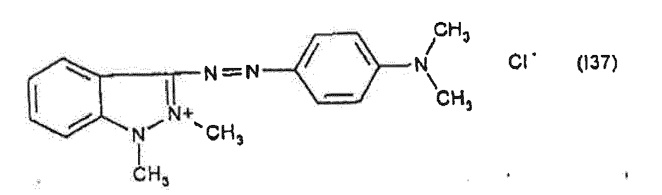 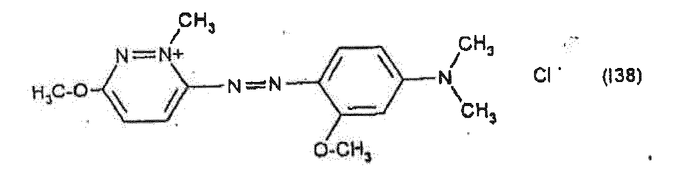 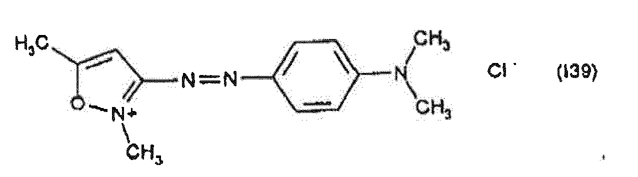 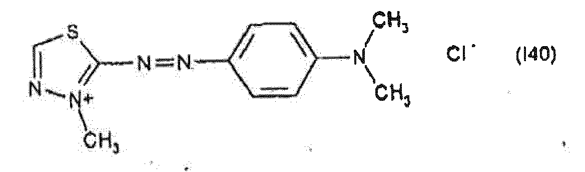 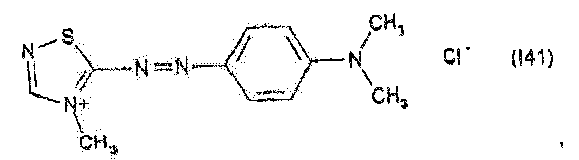 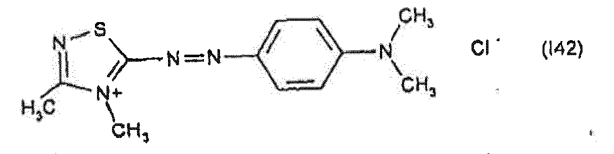 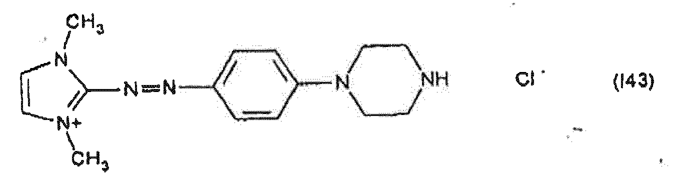 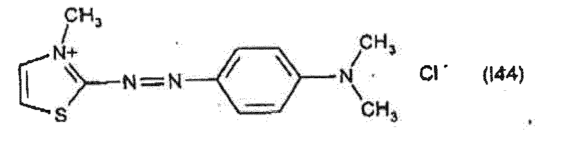 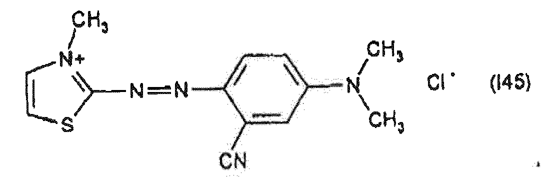 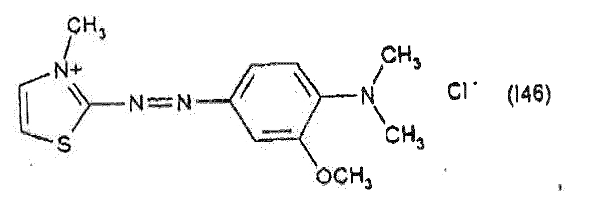 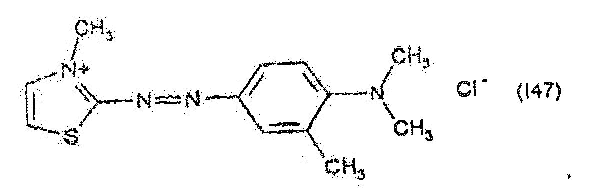 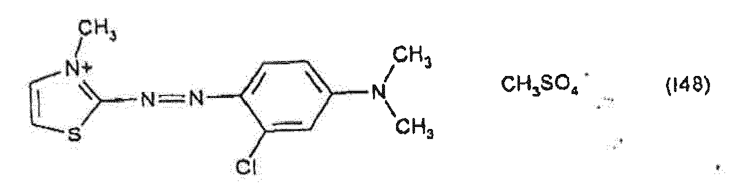 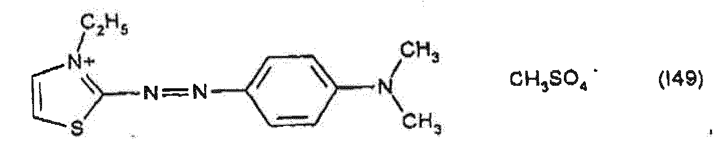 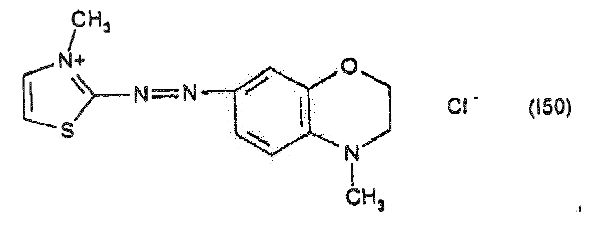 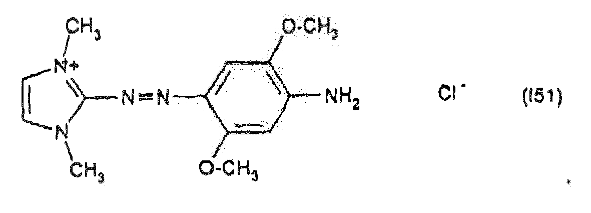 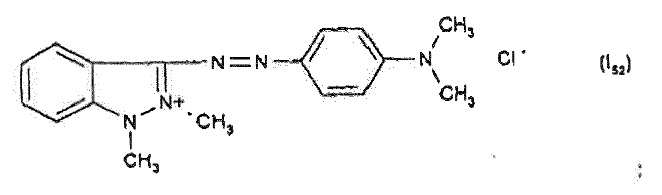 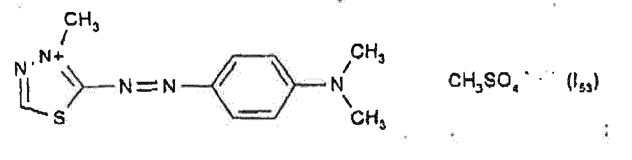 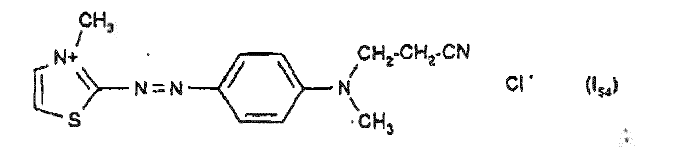 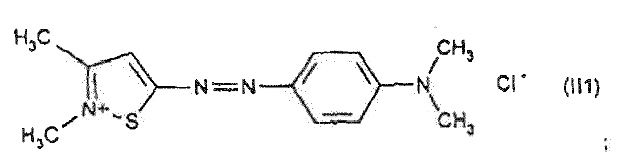 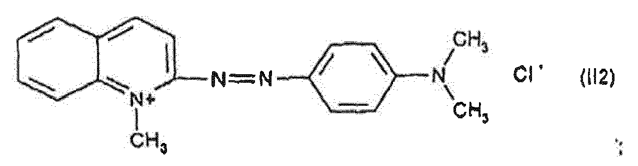 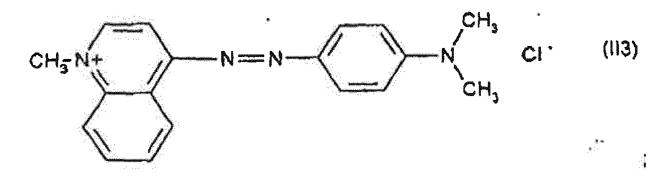 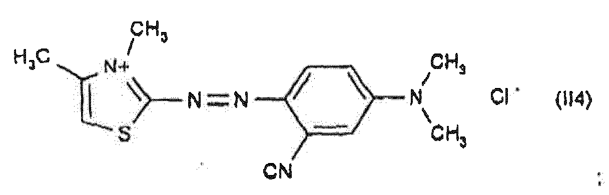 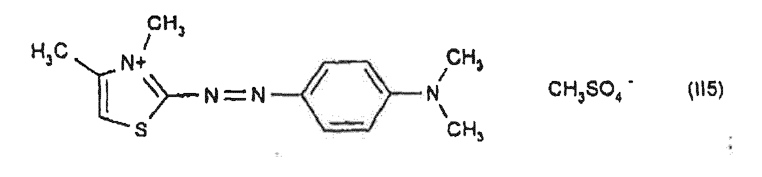 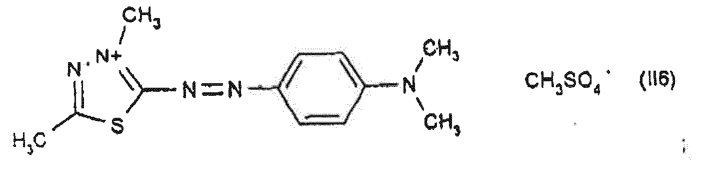 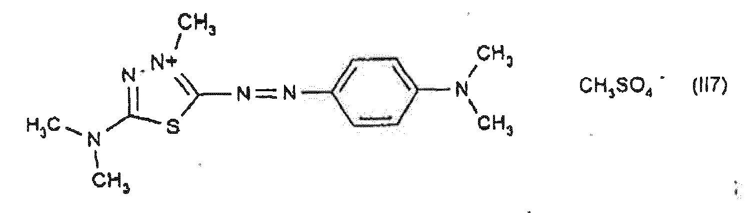 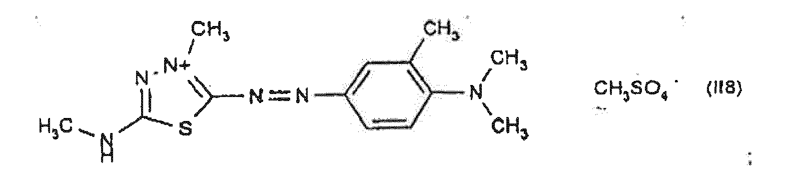 und 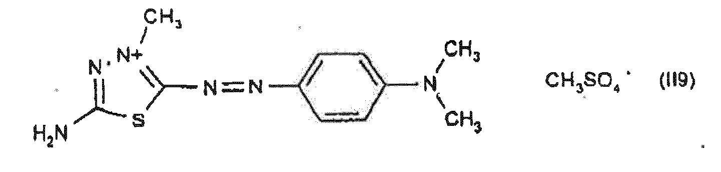 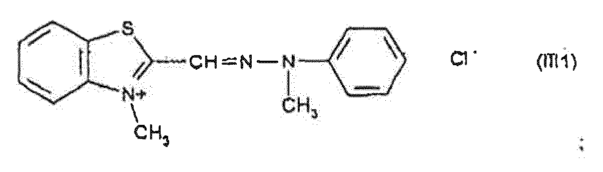 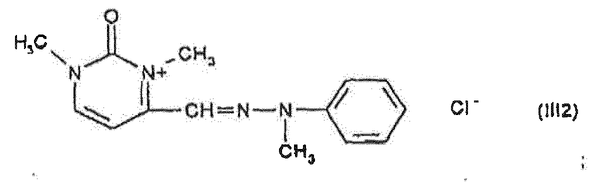 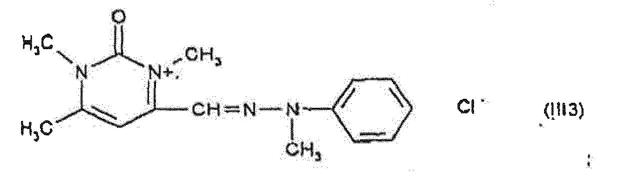 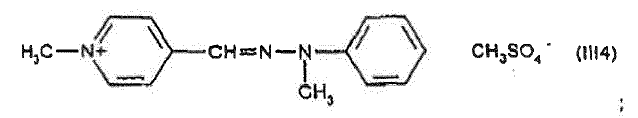 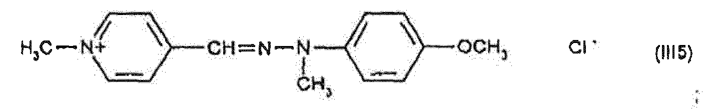 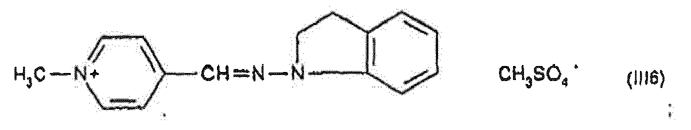 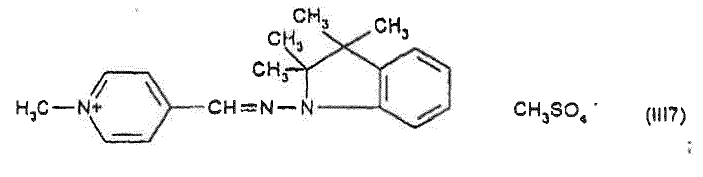 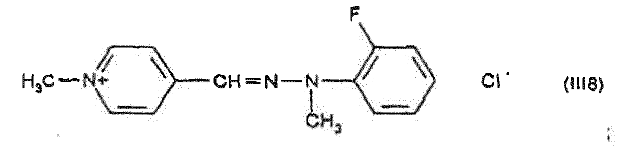 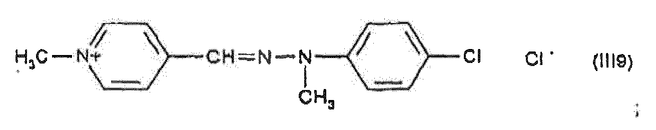 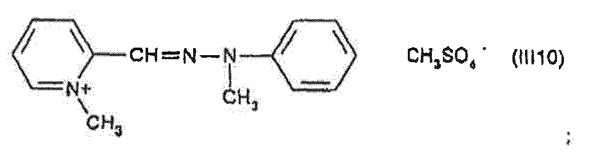 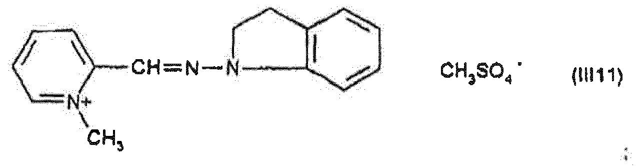 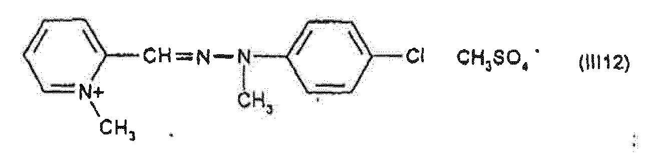 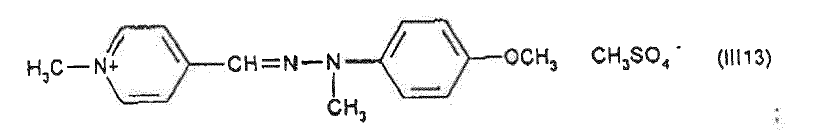 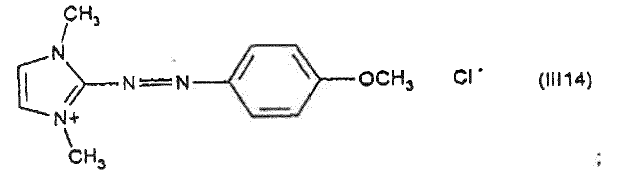 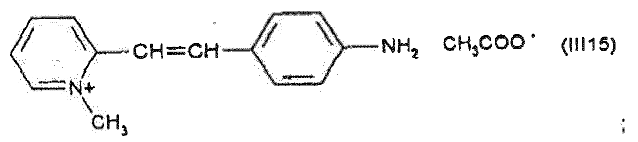 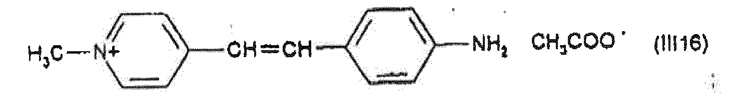 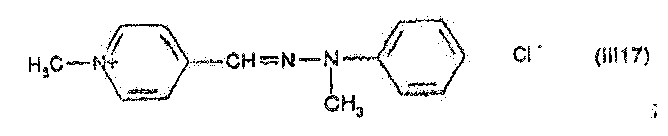 und 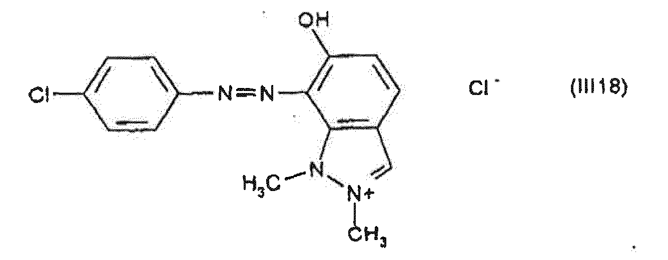 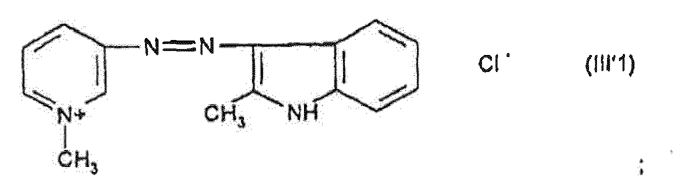 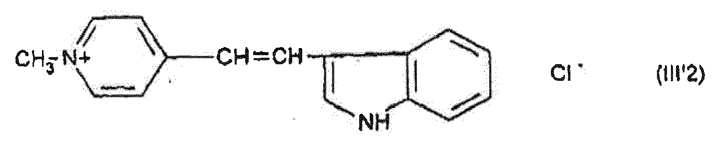 und 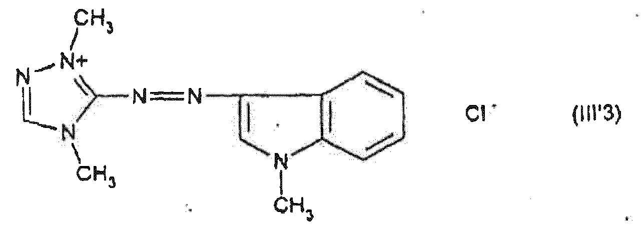 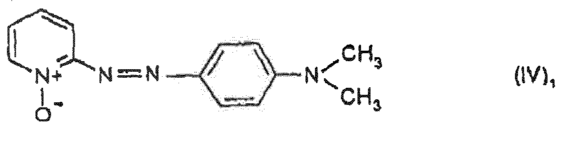 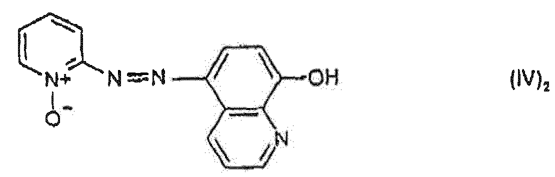 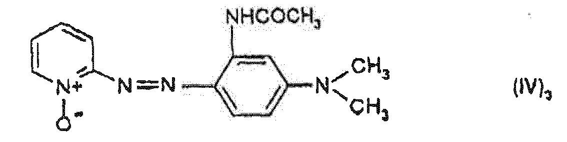 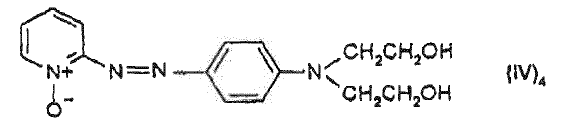 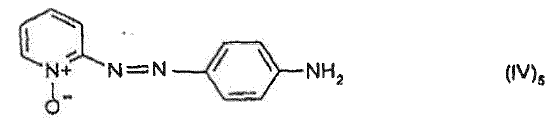 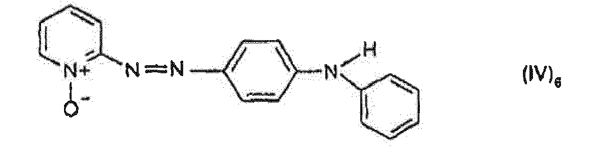 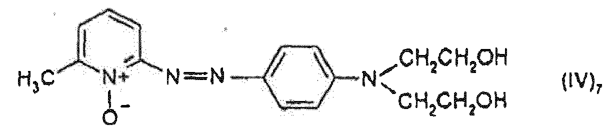 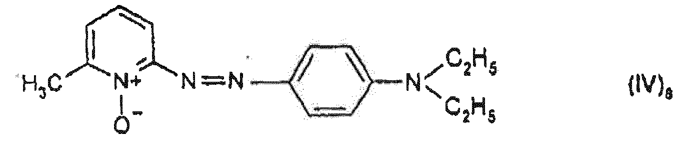 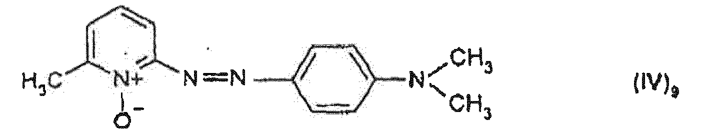 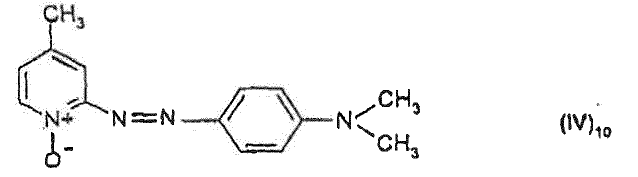 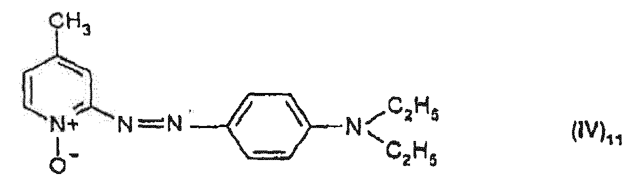 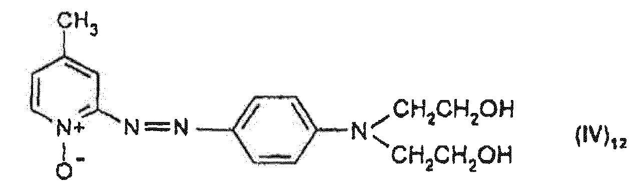 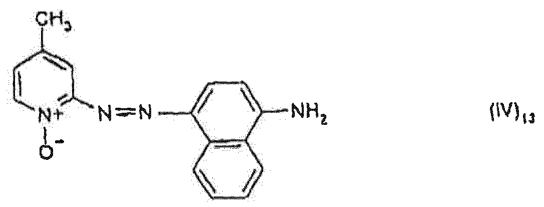 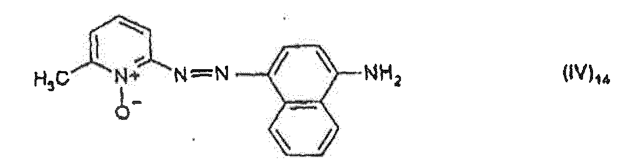 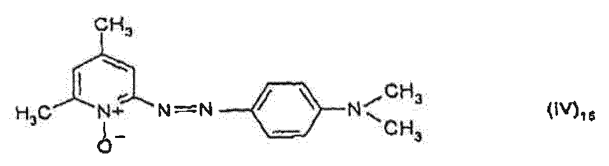 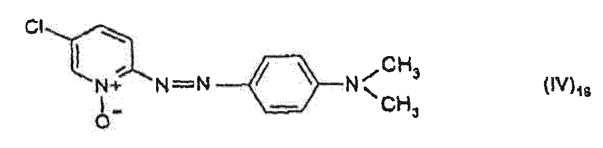 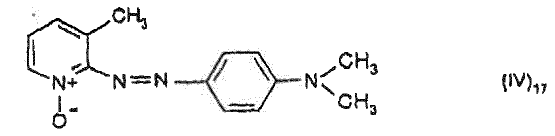 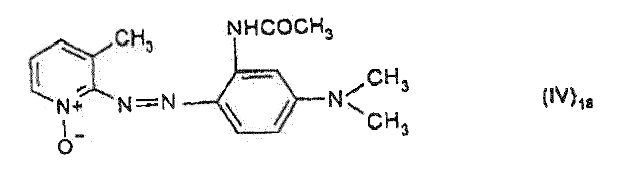 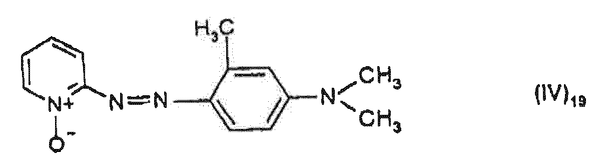 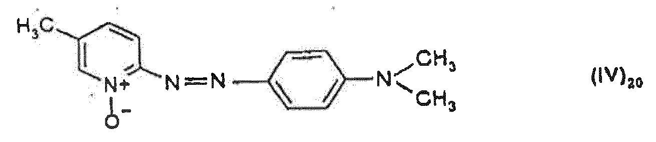 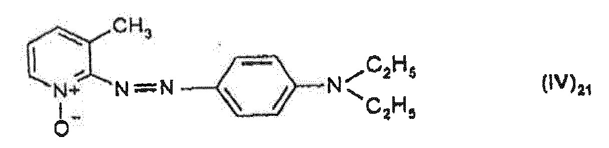 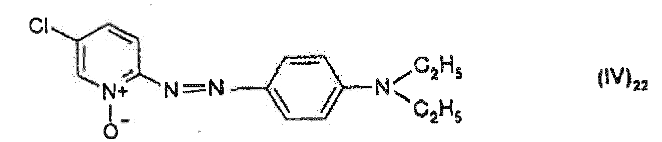 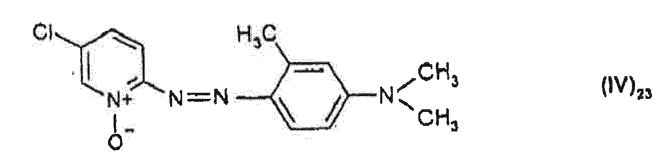 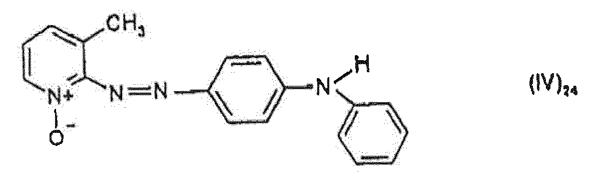 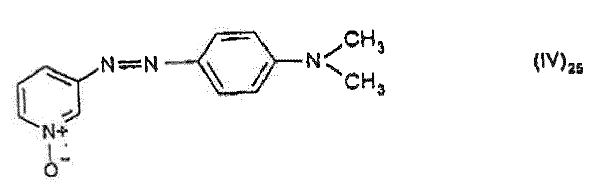 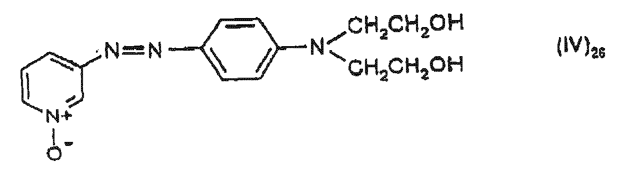 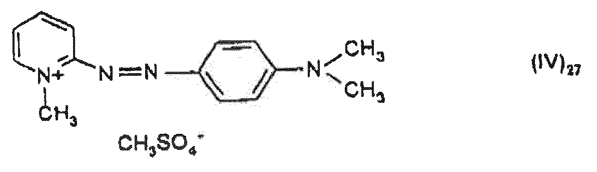 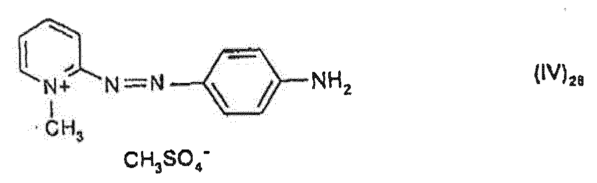 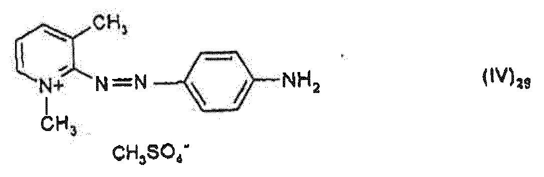 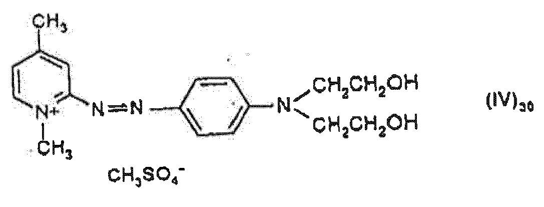 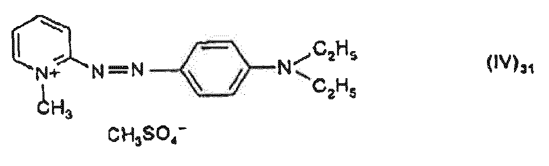 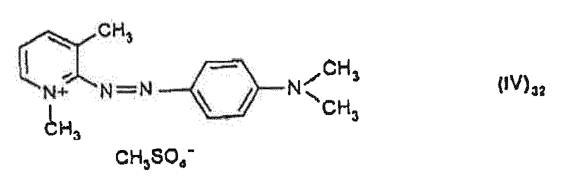 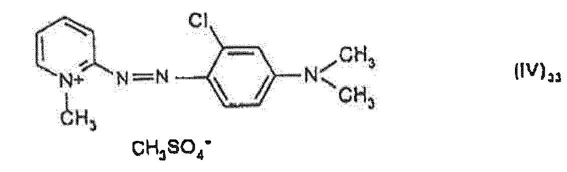 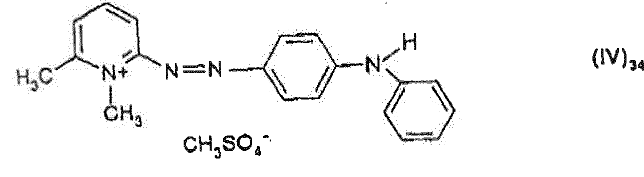 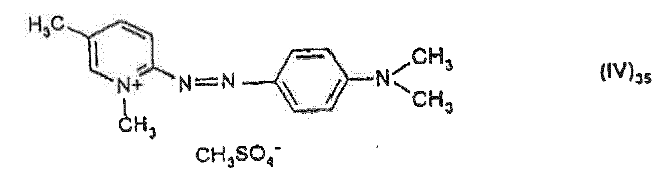 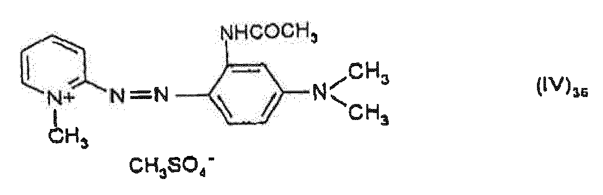 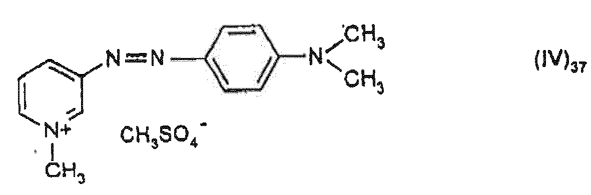 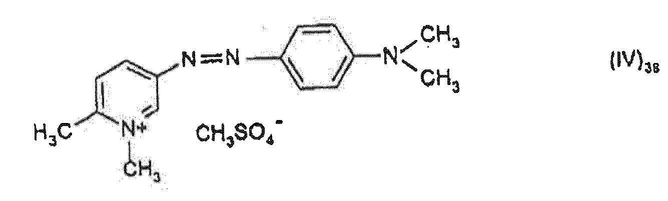 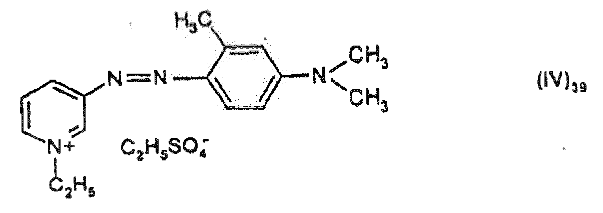 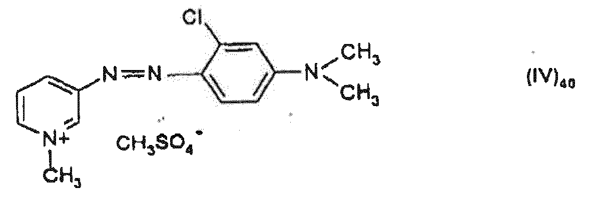 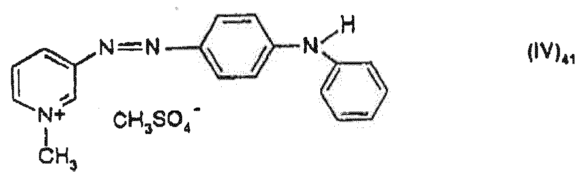 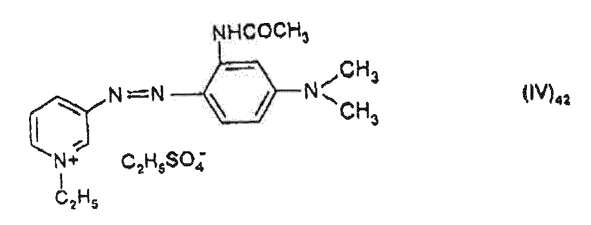 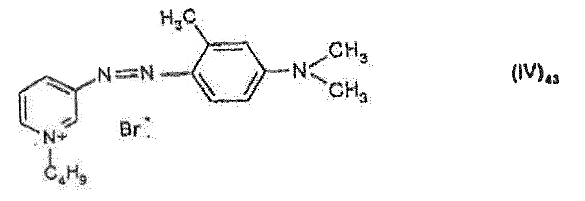 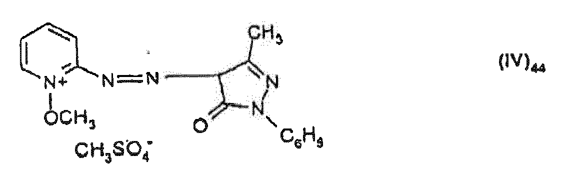 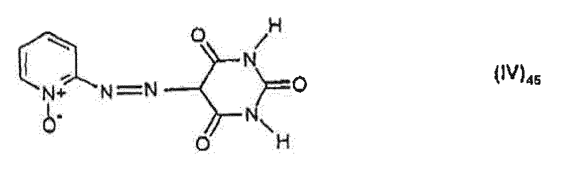 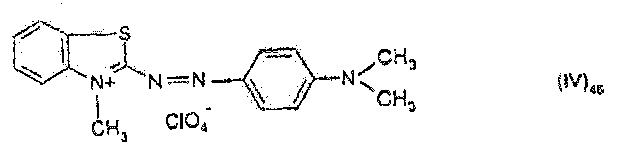 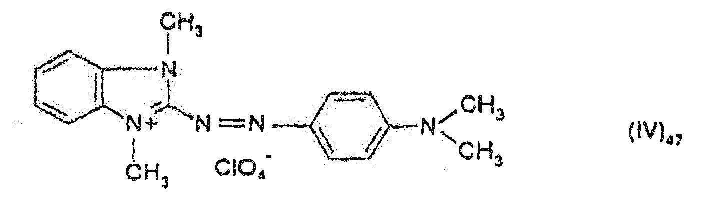 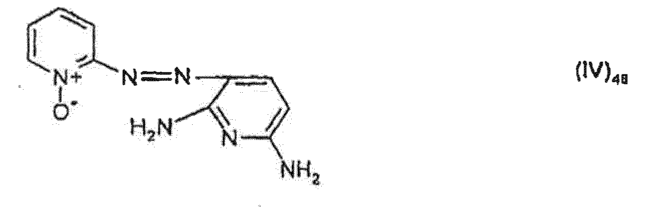 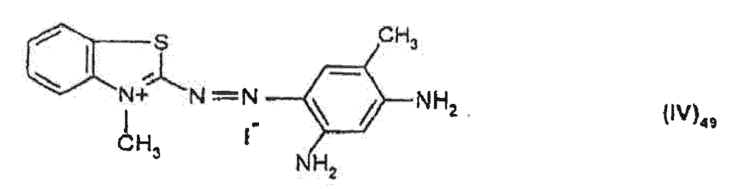 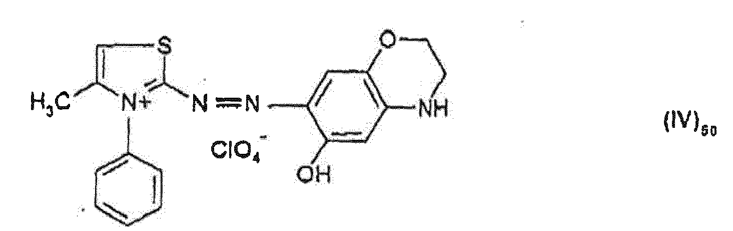 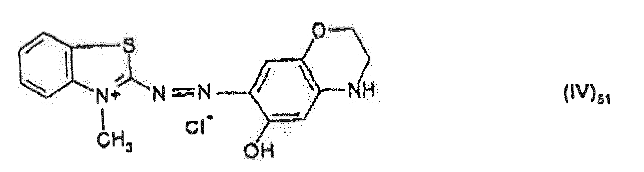 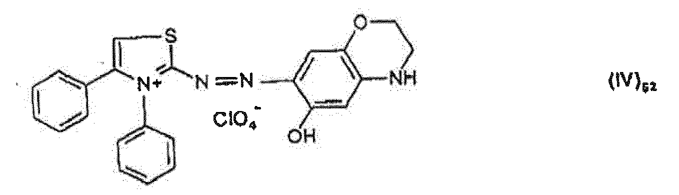 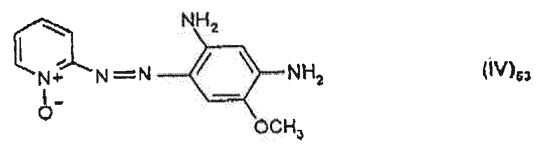 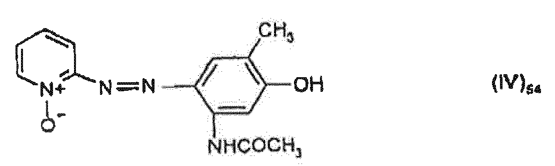 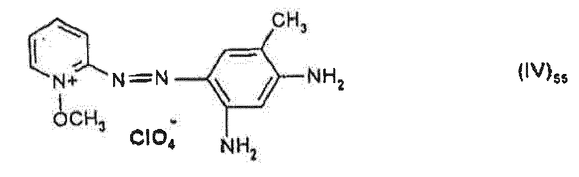 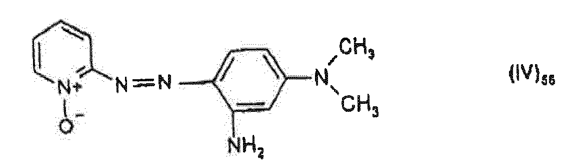 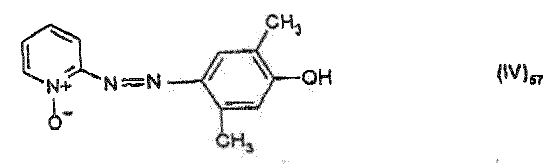 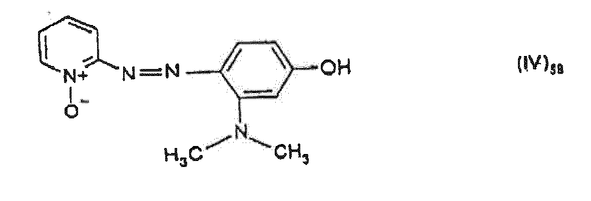 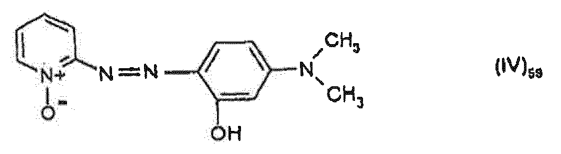 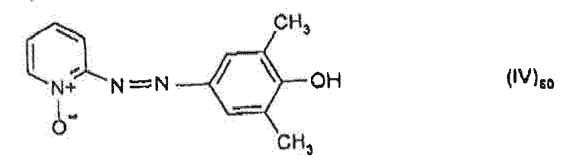 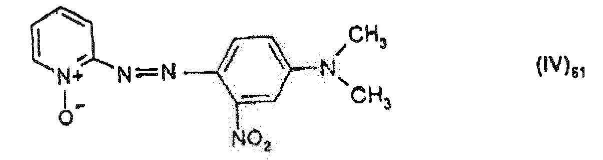 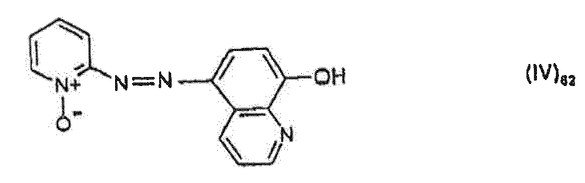 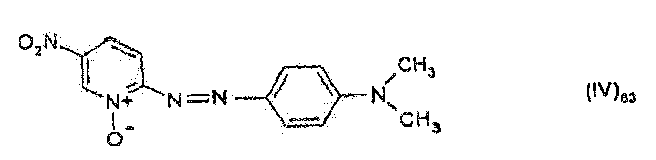 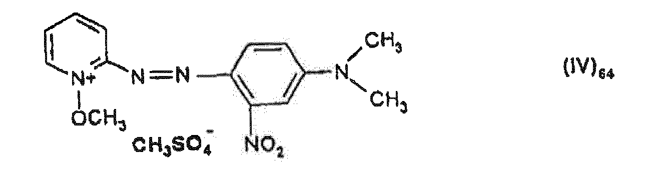 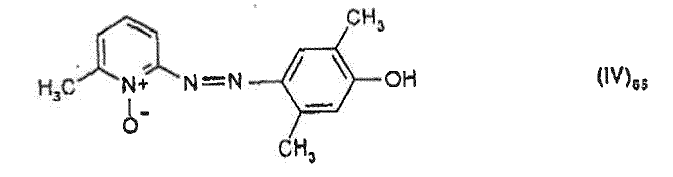 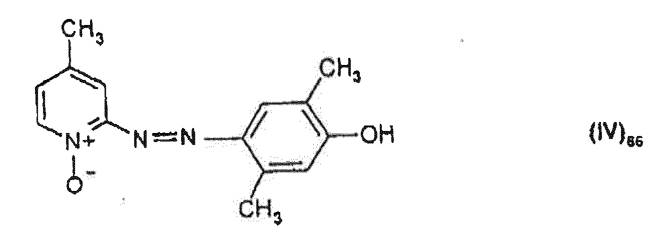 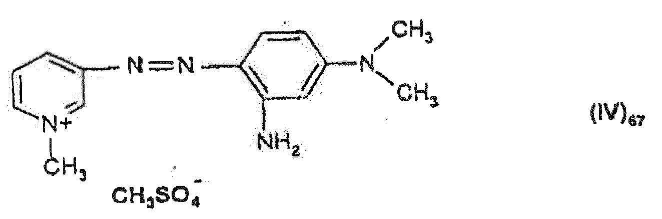 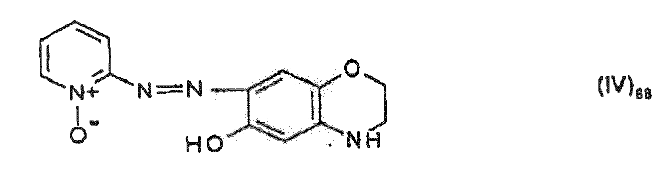 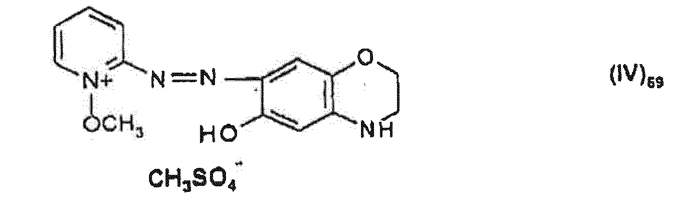 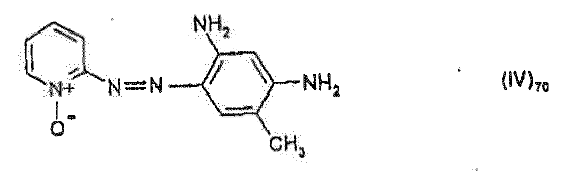 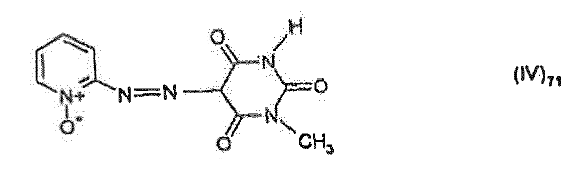 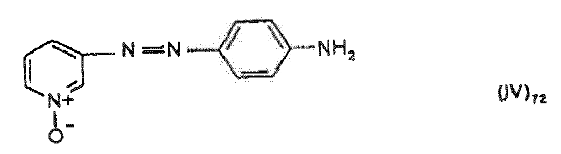 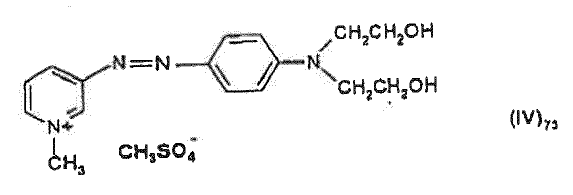 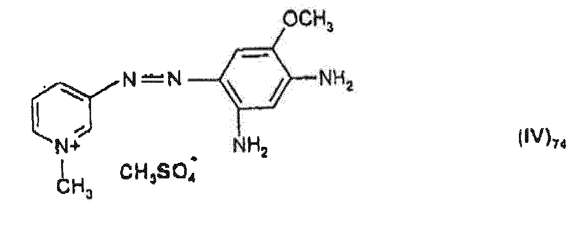 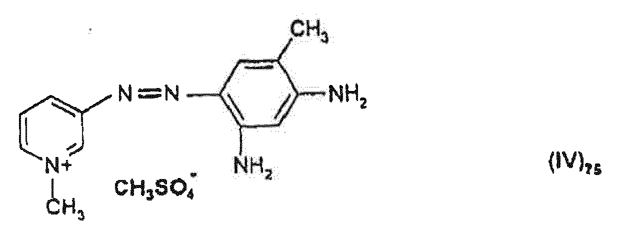 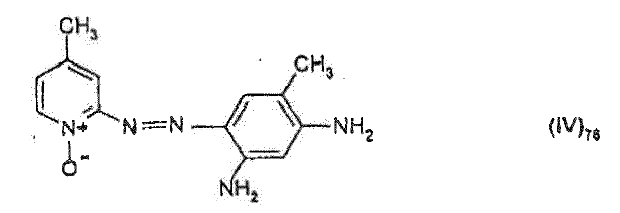 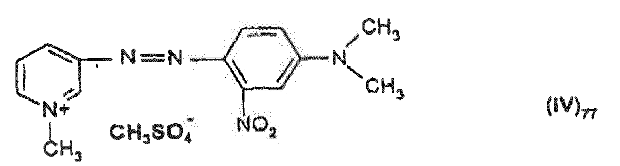 wobei die Zusammensetzung **dadurch gekennzeichnet ist, dass** sie ferner enthält:
(ii) mindestens einen anionischen grenzflächenaktiven Stoff, der unter den folgenden Verbindungen ausgewählt ist:
(ii)₁ - Acylisethionaten;
(ii)₂ - Acyltauraten;
(iii)₁₀ - deren Gemischen.

2. Zusammensetzung nach Anspruch 1, **dadurch gekennzeichnet, dass** die kationischen Direktfarbstoffe den Strukturen (I1), (I2), (I14) und (I31) entsprechen.

3. Zusammensetzung nach Anspruch 1, **dadurch gekennzeichnet, dass** die kationischen Direktfarbstoffe der Formel (III) den Strukturen (III4), (III5) und (III13) entsprechen.

4. Zusammensetzung nach einem der vorhergehenden Ansprüche, **dadurch gekennzeichnet, dass** der oder die kationische(n) Direktfarbstoff(e) der Formeln nach Anspruch 1 0,001 bis 10 Gew.-% des Gesamtgewichts der Zusammensetzung ausmachen.

5. Zusammensetzung nach Anspruch 4, **dadurch gekennzeichnet, dass** der oder die kationische(n) Direktfarbstoff(e) der Formeln nach Anspruch 1 0,005 bis 5 Gew.-% des Gesamtgewichts der Zusammensetzung ausmachen.

6. Zusammensetzung nach einem der vorhergehenden Ansprüche, **dadurch gekennzeichnet, dass** der anionische grenzflächenaktive Stoff (ii) vom Typ Acylisethionat (ii)₁ und Acyltaurat (ii)₂ der folgenden allgemeinen Struktur entspricht:
R¹-CH₂-CH₂-SO₃- M⁺ (V)
wobei R¹ eine Gruppe R²COO oder R²CONR³ bedeutet,
worin R² eine geradkettige oder verzweigte, gesättigte oder ungesättigte aliphatische Gruppe mit 8 bis 30 Kohlenstoffatomen ist und R³ eine Wasserstoffatom oder eine C₁₋₄-Alkylgruppe bedeutet; und M H, Ammonium, Na oder K oder den Rest eines organischen Amins bedeutet.

7. Zusammensetzung nach einem der vorhergehenden Ansprüche, **dadurch gekennzeichnet, dass** der oder die anionische(n) grenzflächenaktive(n) Stoff(e) 0,05 bis 30 Gew.-% des Gesamtgewichts der Farbmittelzusammensetzung ausmachen.

8. Zusammensetzung nach Anspruch 7, **dadurch gekennzeichnet, dass** der oder die anionische(n) grenzflächenaktive(n) Stoff(e) 0,1 bis 15 Gew.-% des Gesamtgewichts der Farbmittelzusammensetzung ausmachen.

9. Zusammensetzung nach einem der vorhergehenden Ansprüche, **dadurch gekennzeichnet, dass** das zum Färben geeignete Medium (oder der Träger) aus Wasser oder einem Gemisch von Wasser und mindestens einem organischen Lösungsmittel besteht.

10. Zusammensetzung nach einem der vorhergehenden Ansprüche, **dadurch gekennzeichnet, dass** sie einen pH-Wert im Bereich von 2 bis 11 und vorzugsweise 5 bis 10 aufweist.

11. Zusammensetzung nach einem der vorhergehenden Ansprüche, **dadurch gekennzeichnet, dass** sie zum oxidativen Färben vorgesehen ist und eine oder mehrere Oxidationsbasen enthält, die unter den p-Phenylendiaminen, Bisphenylalkylendiaminen, p-Aminophenolen, o-Aminophenolen und heterocyclischen Basen ausgewählt sind.

12. Zusammensetzung nach Anspruch 22, **dadurch gekennzeichnet, dass** die Oxidationsbase(n) 0,0005 bis 12 Gew.-% des Gesamtgewichts der Farbmittelzusammensetzung ausmachen.

13. Zusammensetzung nach Anspruch 12, **dadurch gekennzeichnet, dass** die Oxidationsbase(n) 0,005 bis 6 Gew.-% des Gesamtgewichts der Farbmittelzusammensetzung ausmachen.

14. Zusammensetzung nach einem der Ansprüche 11 bis 13, **dadurch gekennzeichnet, dass** sie einen oder mehrere Kuppler enthält, die unter den m-Phenylendiaminen, m-Aminophenolen, m-Dihydroxybenzolen und heterocyclischen Kupplern ausgewählt sind.

15. Zusammensetzung nach Anspruch 14, **dadurch gekennzeichnet, dass** der oder die Kuppler 0,0001 bis 10 Gew.-% des Gesamtgewichts der Farbmittelzusammensetzung ausmachen.

16. Zusammensetzung nach Anspruch 15, **dadurch gekennzeichnet, dass** der oder die Kuppler 0,005 bis 5 Gew.-% des Gesamtgewichts der Farbmittelzusammensetzung ausmachen.

17. Zusammensetzung nach einem der vorhergehenden Ansprüche, **dadurch gekennzeichnet, dass** sie zum oxidativen Färben oder für die aufhellende Direktfärbung vorgesehen ist und mindestens ein Oxidationsmittel enthält.

18. Verfahren zum Färben von Keratinfasern und insbesondere menschlichen Keratinfasern, wie dem Haar, **dadurch gekennzeichnet, dass** auf die Fasern mindestens eine Farbmittelzusammensetzung nach einem der Ansprüche 1 bis 17 während einer Zeitspanne, die zur Bildung der gewünschten Färbung ausreichend ist, aufgebracht wird und dann gespült, gegebenenfalls mit Haarwaschmittel gewaschen, nochmals gespült und getrocknet wird.

19. Verfahren zum Färben von Keratinfasern und insbesondere menschlichen Keratinfasern, wie dem Haar, **dadurch gekennzeichnet, dass** auf die Fasern mindestens eine Farbmittelzusammensetzung nach einem der Ansprüche 1 bis 17 während einer Zeitspanne, die zur Bildung der gewünschten Färbung ausreichend ist, aufgebracht wird, ohne dass nochmals gespült wird.

20. Verfahren zum Färben von Keratinfasern und insbesondere menschlichen Keratinfasern, wie dem Haar, **dadurch gekennzeichnet, dass** es einen vorbereitenden Schritt umfasst, der darin besteht, einerseits eine Zusammensetzung (A1), die in einem zum Färben geeigneten Medium mindestens einen in den vorhergehenden Ansprüchen definierten Direktfarbstoff (i) und mindestens eine Oxidationsbase enthält, und andererseits eine Zusammensetzung (B1) getrennt voneinander aufzubewahren, die in einem zum Färben geeigneten Medium mindestens ein Oxidationsmittel enthält, und diese bei der Anwendung zu vermischen, bevor das Gemisch auf die Fasern aufgebracht wird, wobei die Zusammensetzung (A1) oder die Zusammensetzung (B1) den in den vorhergehenden Ansprüchen definierten anionischen grenzflächenaktiven Stoff (ii) enthält.

21. Verfahren zum Färben von Keratinfasern und insbesondere menschlichen Keratinfasern, wie dem Haar, **dadurch gekennzeichnet, dass** es einen vorbereitenden Schritt umfasst, der darin besteht, einerseits eine Zusammensetzung (A2), die in einem zum Färben geeigneten Medium mindestens einen in den vorhergehenden Ansprüchen definierten Direktfarbstoff (i) enthält, und andererseits eine Zusammensetzung (B2) getrennt voneinander aufzubewahren, die in einem zum Färben geeigneten Medium mindestens ein Oxidationsmittel enthält, und diese bei der Anwendung zu vermischen, bevor das Gemisch auf die Fasern aufgebracht wird, wobei die Zusammensetzung (A2) oder die Zusammensetzung (B2) den in den vorhergehenden Ansprüchen definierten anionischen grenzflächenaktiven Stoff (ii) enthält.

22. Vorrichtung mit mehreren Abteilungen oder "Kit" zum Färben mit mehreren Abteilungen, **dadurch gekennzeichnet, dass** eine erste Abteilung die Zusammensetzung (A1) oder (A2) nach Anspruch 20 oder 21 und eine andere Abteilung die Zusammensetzung (B1) oder (B2) nach Anspruch 20 oder 21 enthält.

## Claims

1. Composition for dyeing keratinous fibres and in particular human keratinous fibres such as hair, containing in an appropriate dyeing medium, **(i)** at least compound chosen from those of the following formulae: 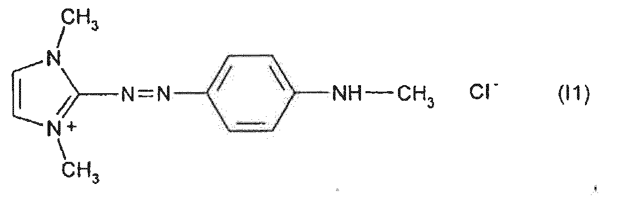 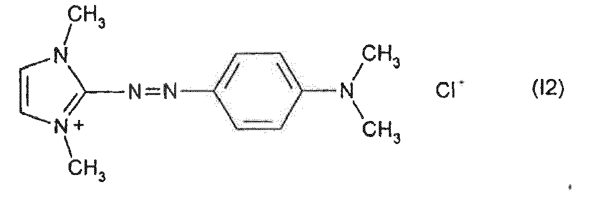 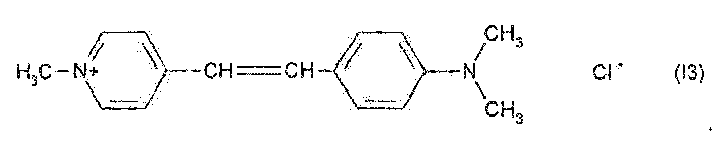 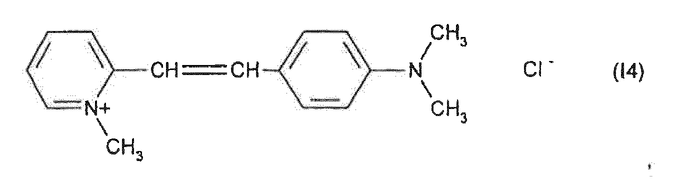 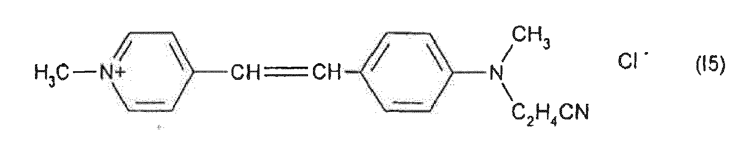 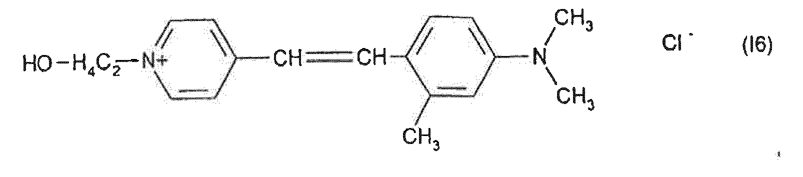 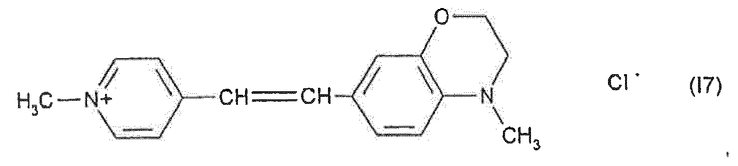 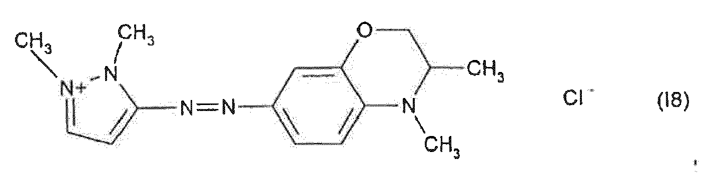 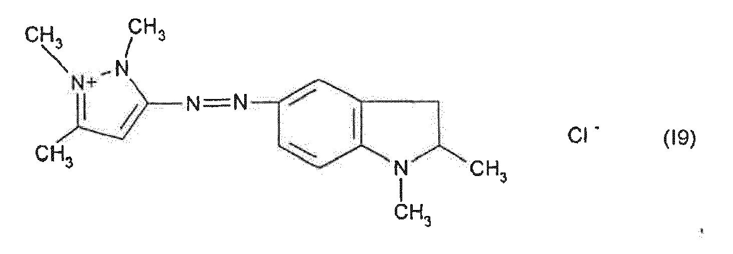 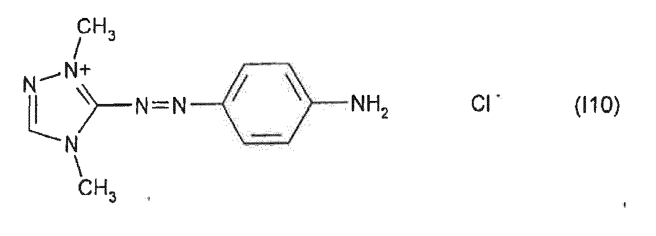 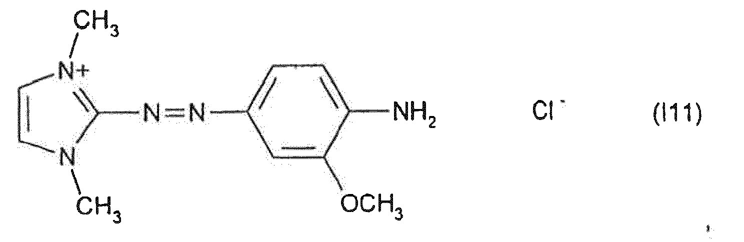 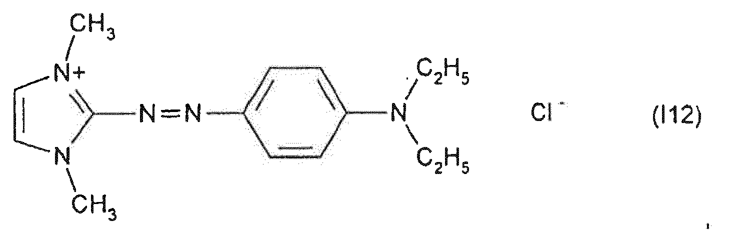 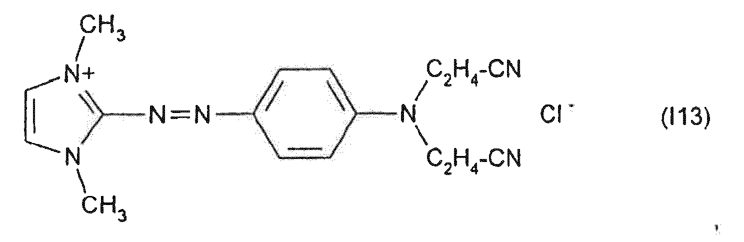 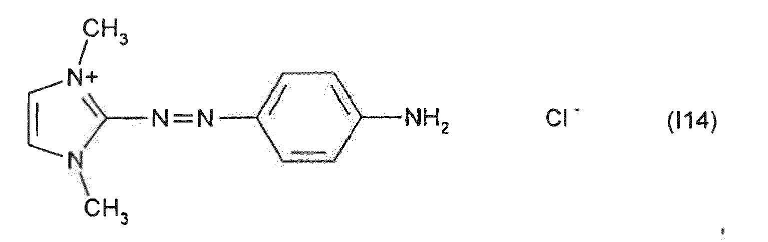 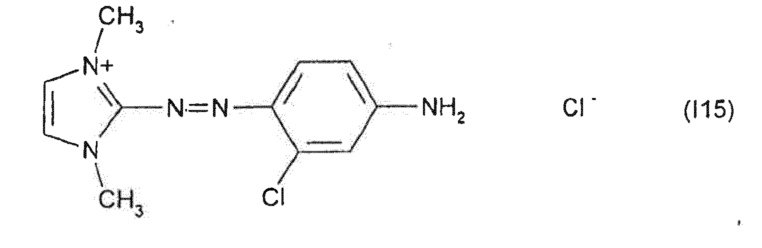 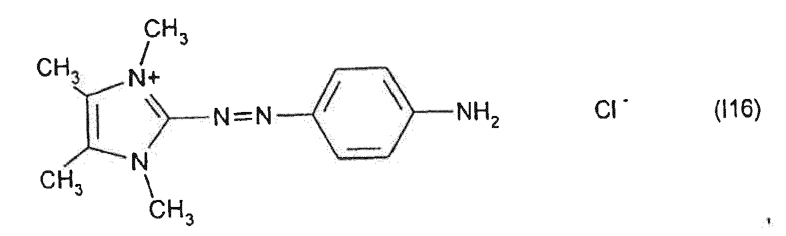 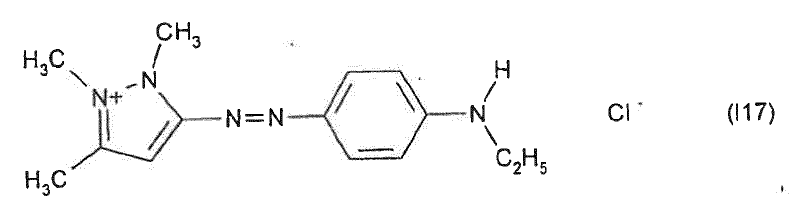 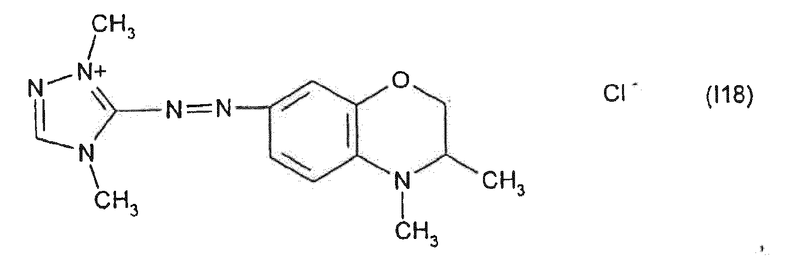 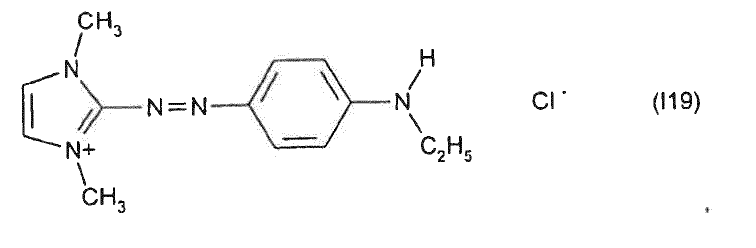 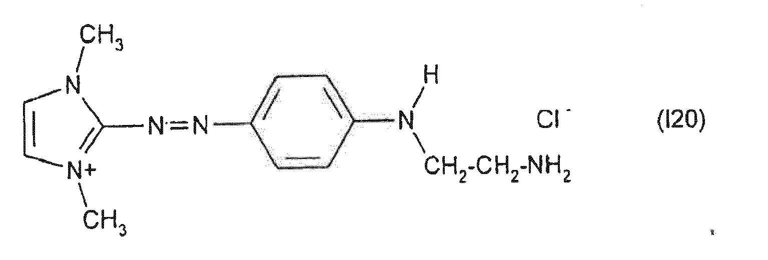 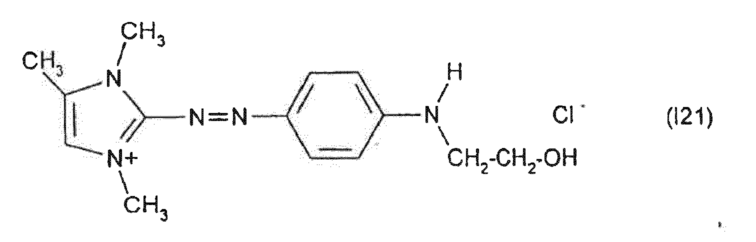 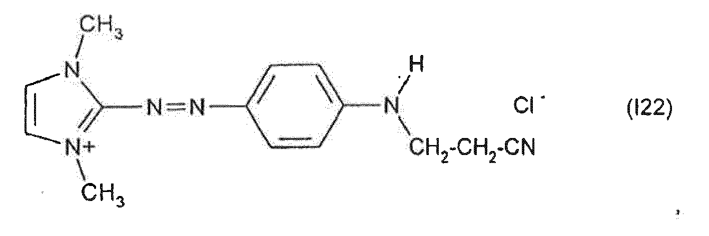 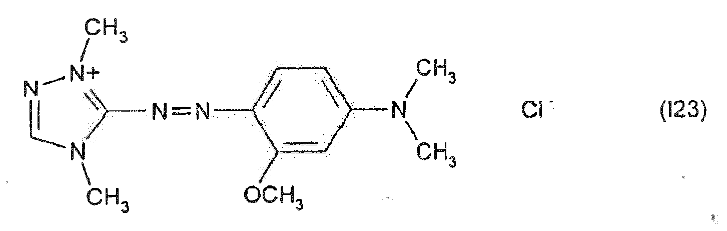 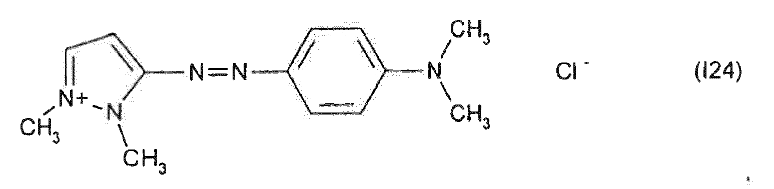 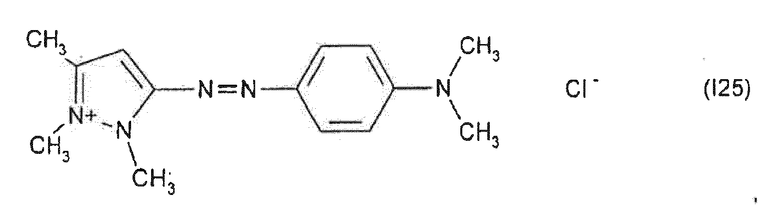 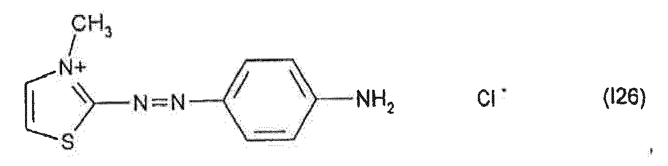 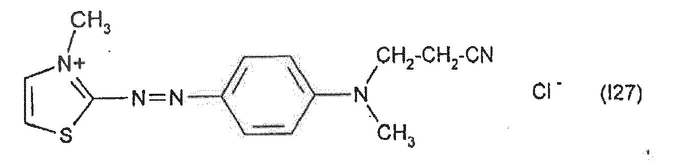 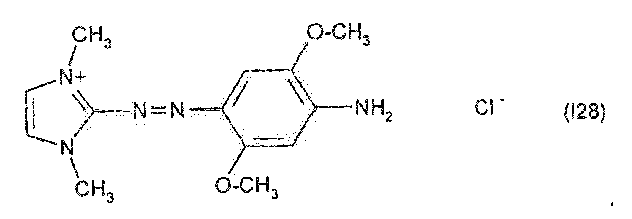 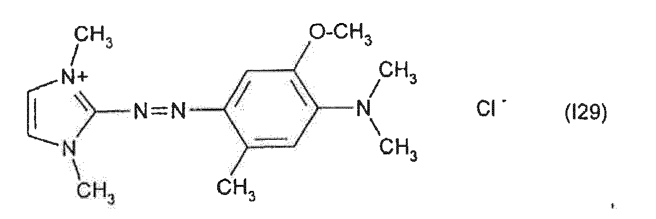 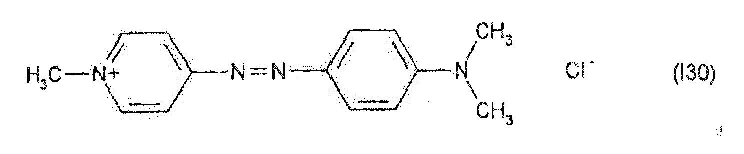 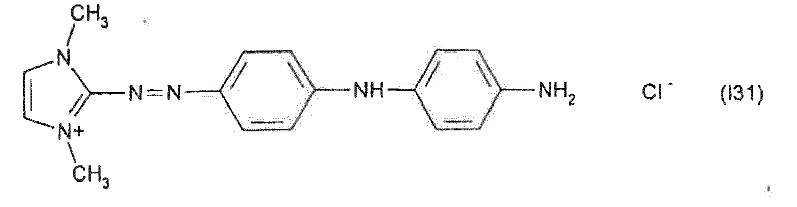 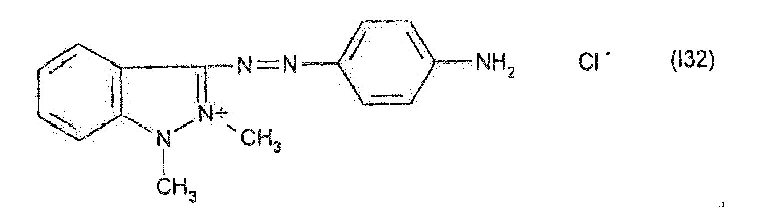 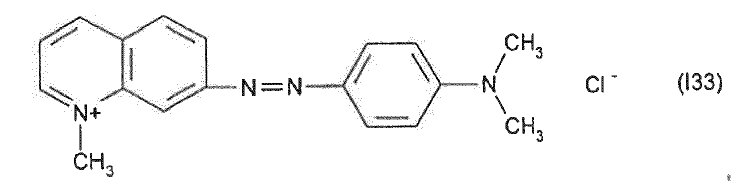 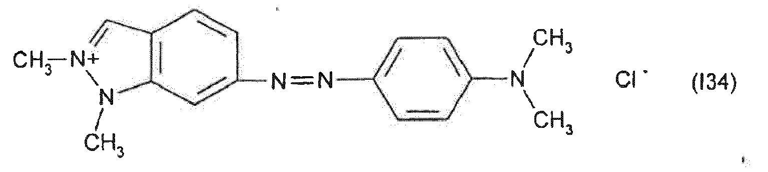 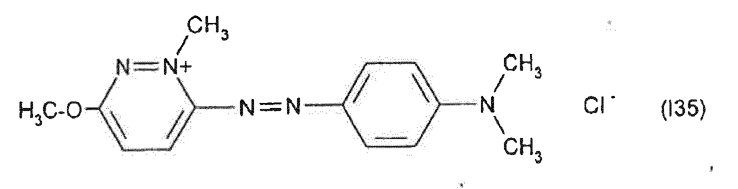 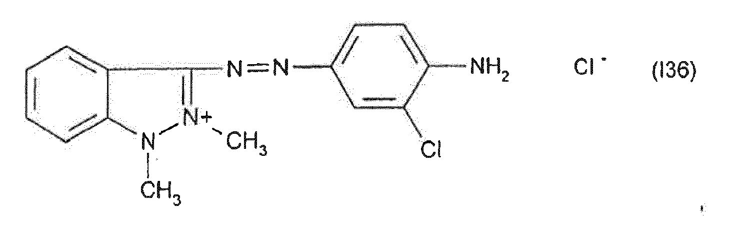 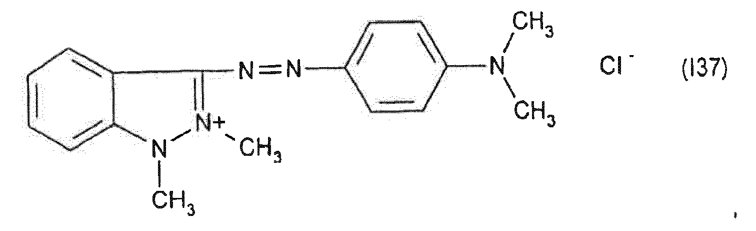 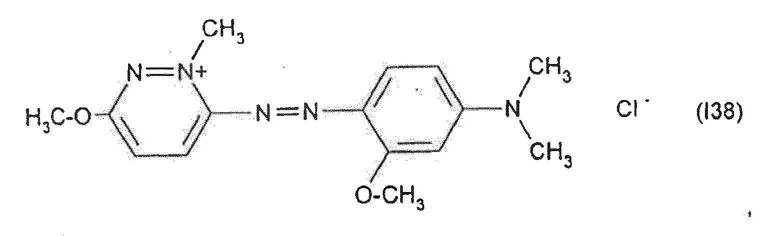 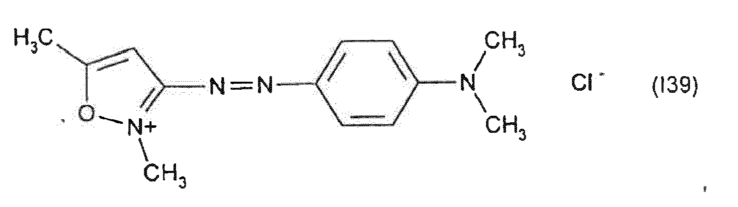 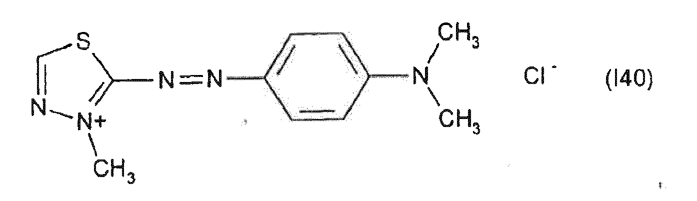 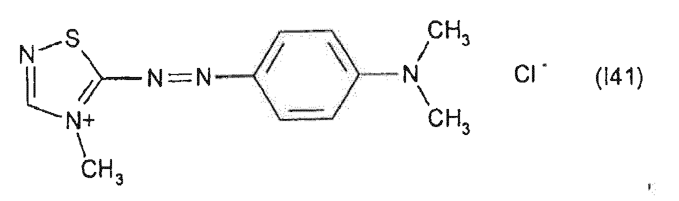 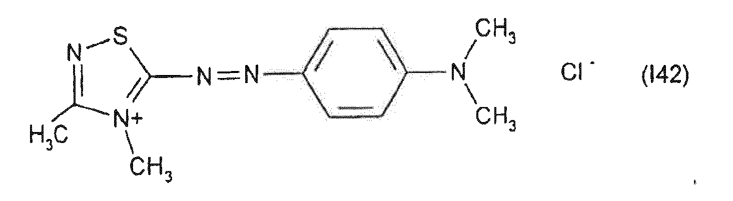 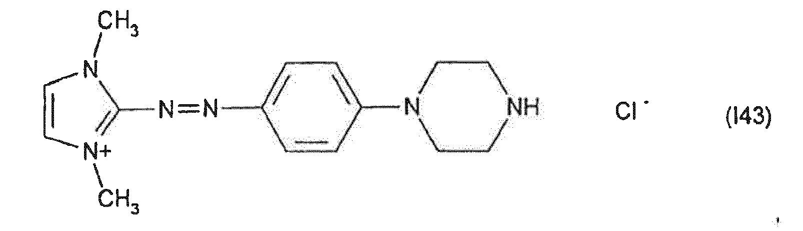 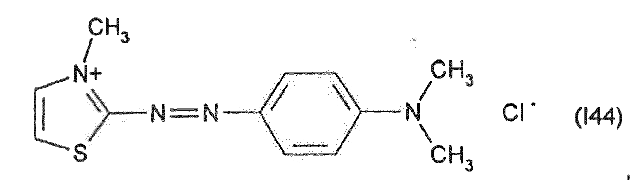 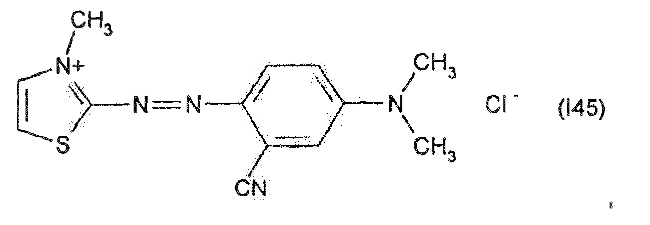 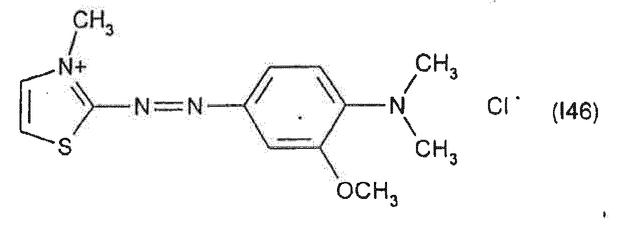 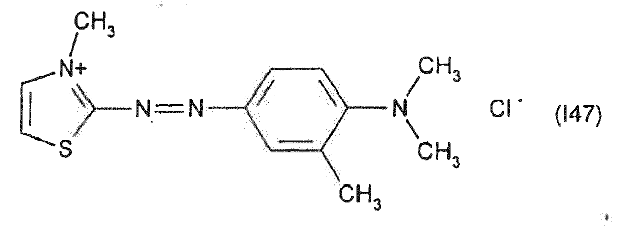 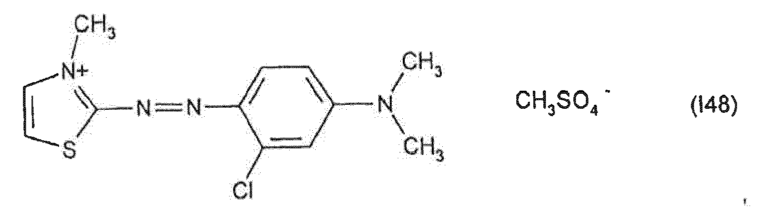 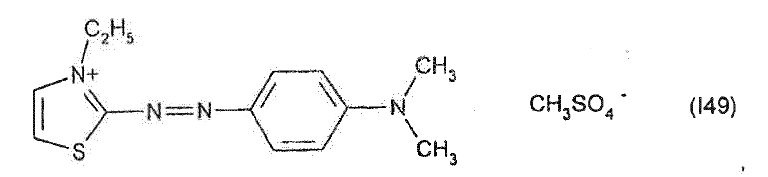 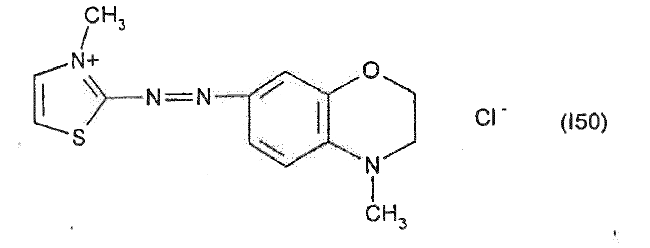 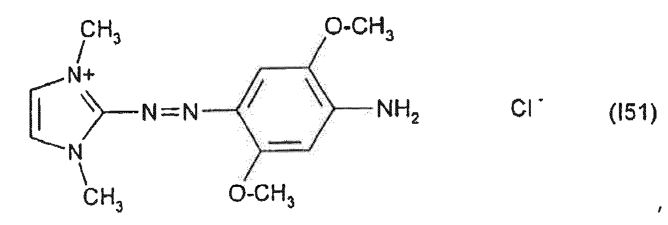 and 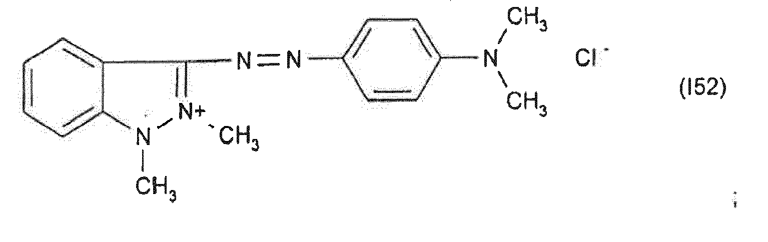 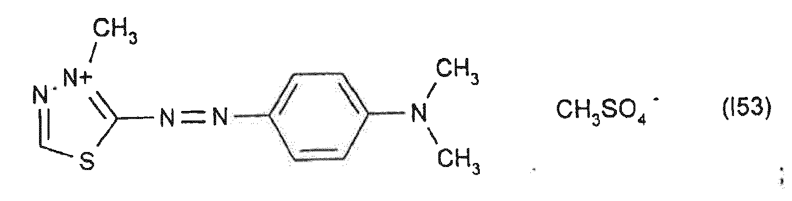 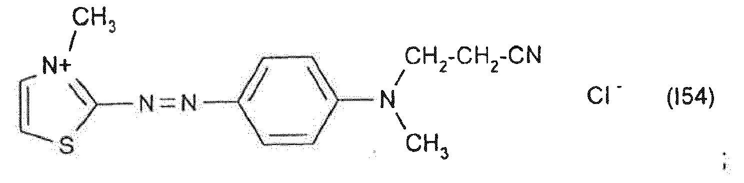 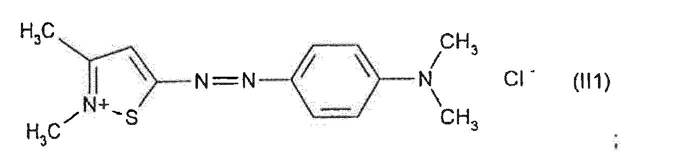 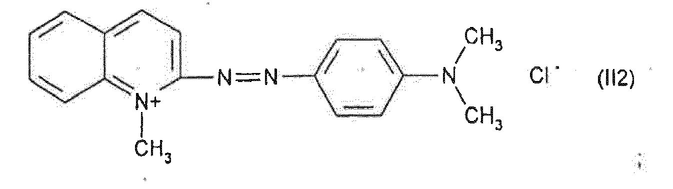 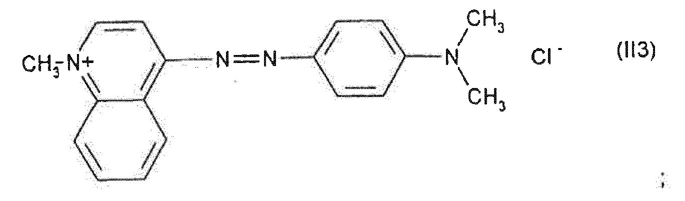 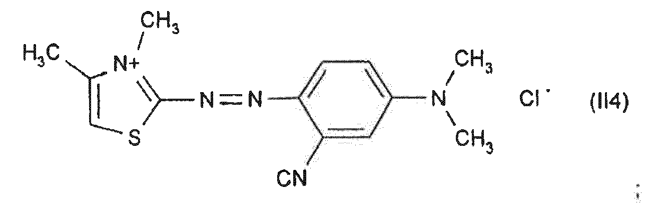 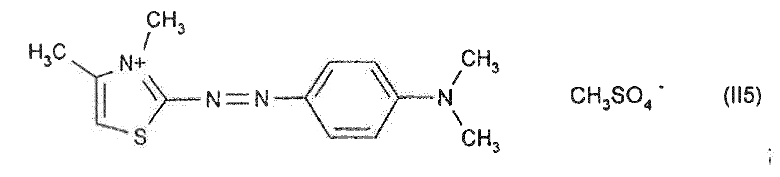 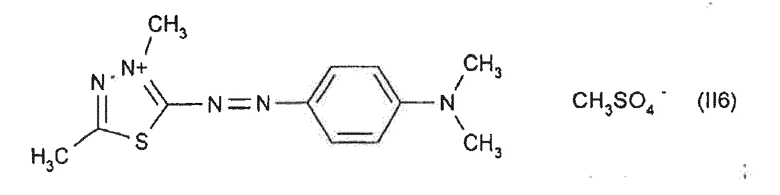 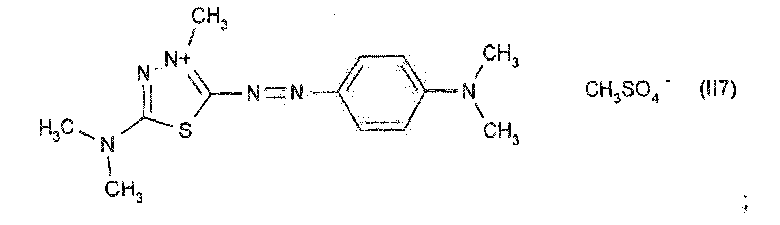 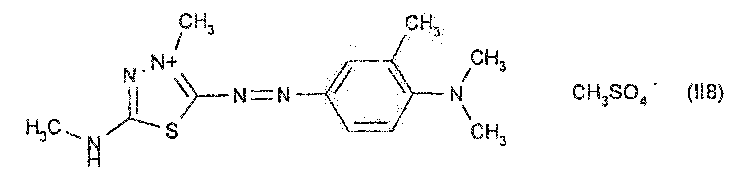 and 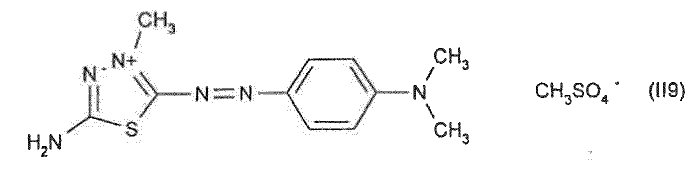 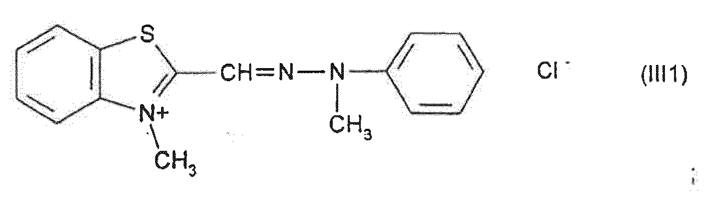 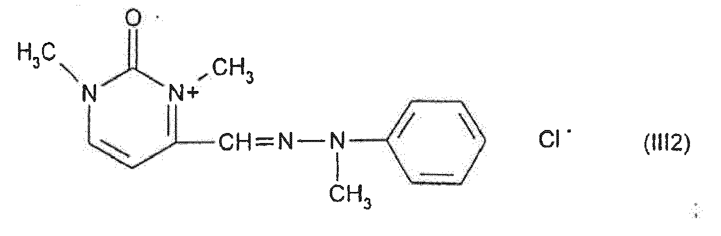 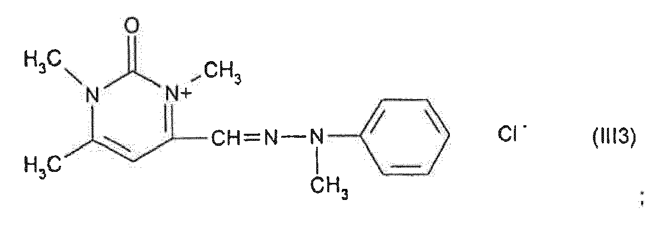 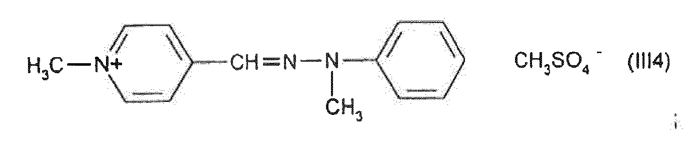 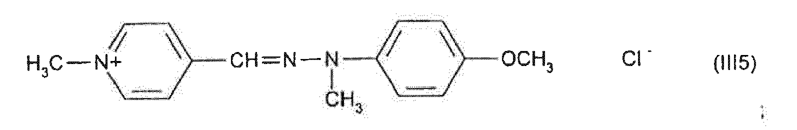 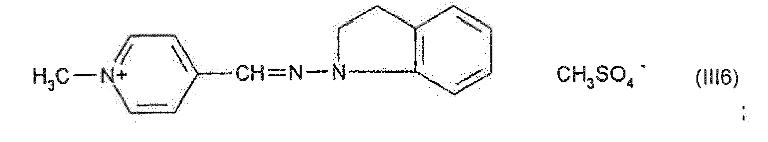 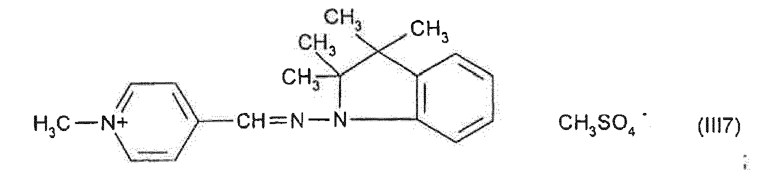 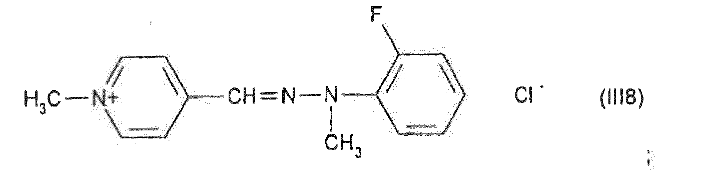 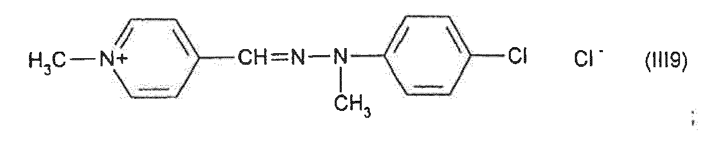 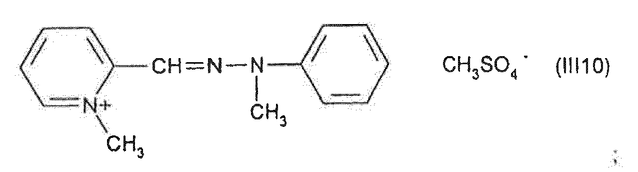 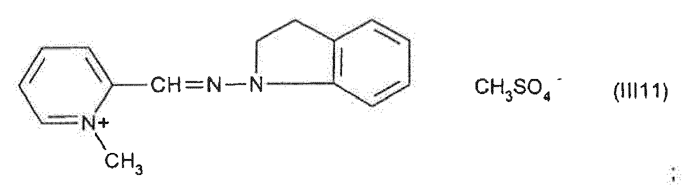 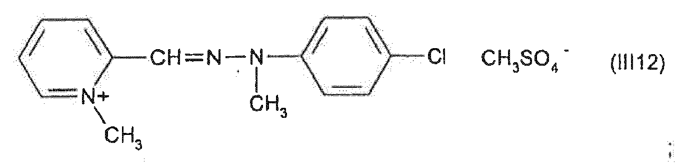 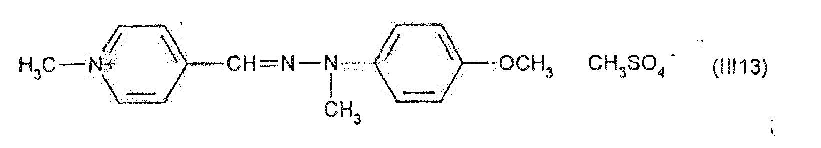 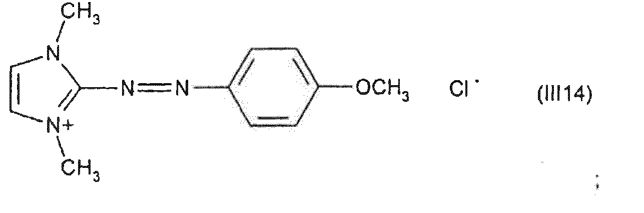 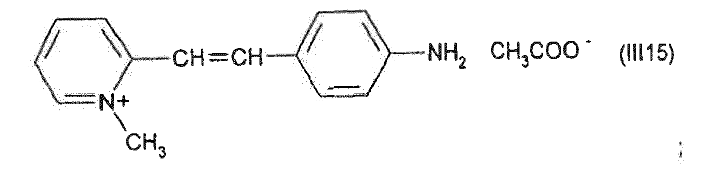 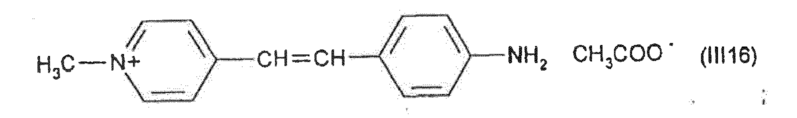 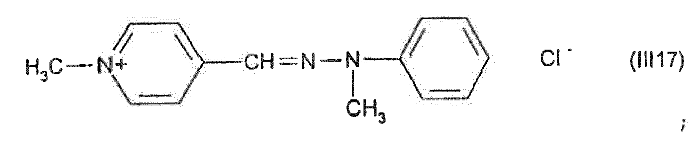 and 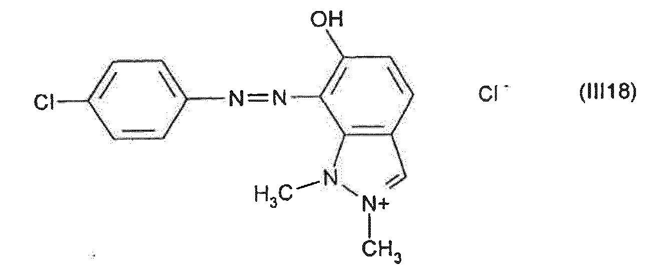 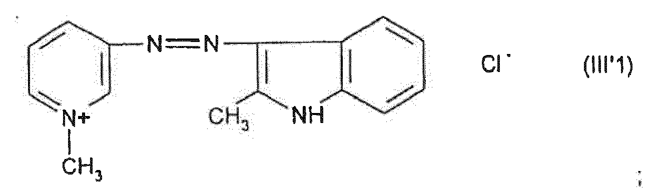 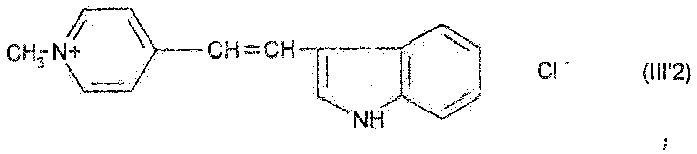 and 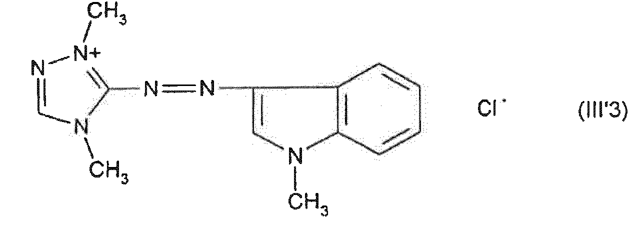 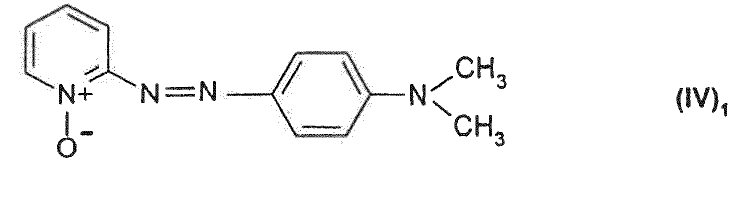 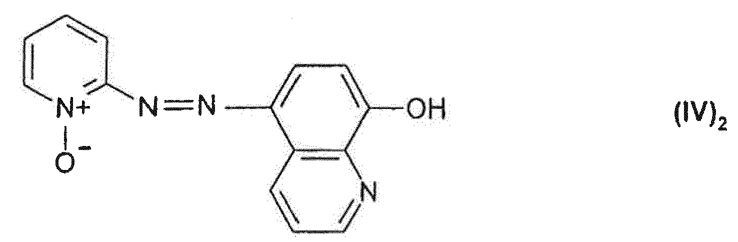 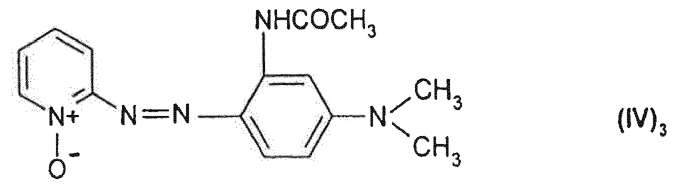 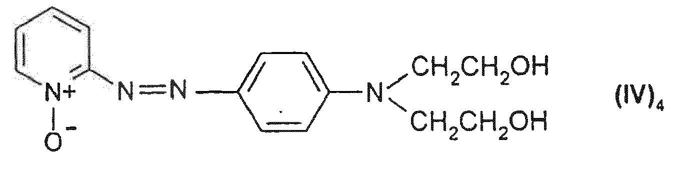 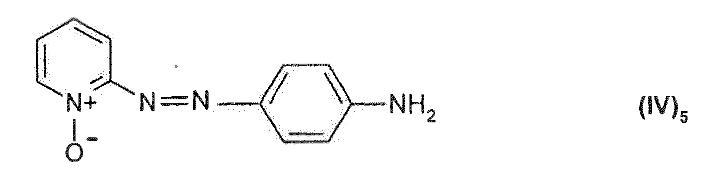 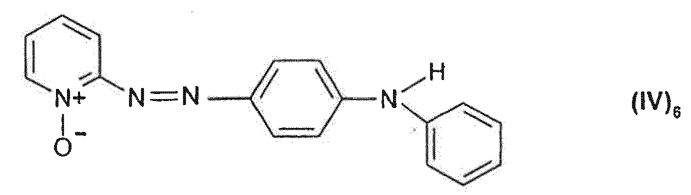 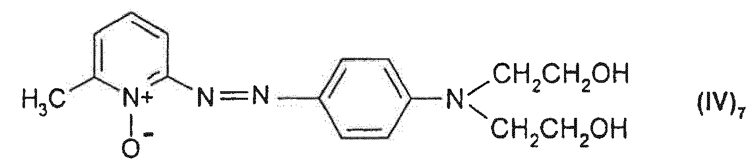 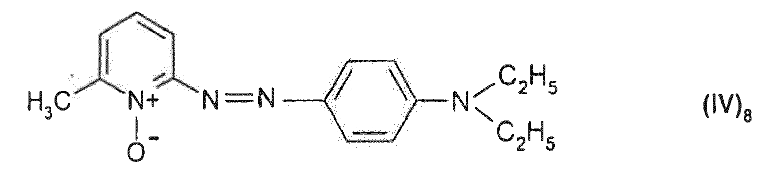 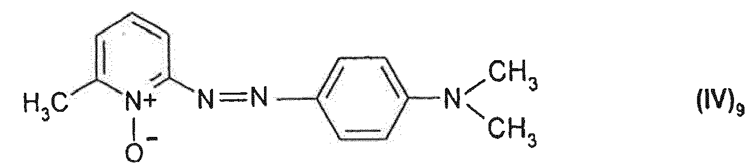 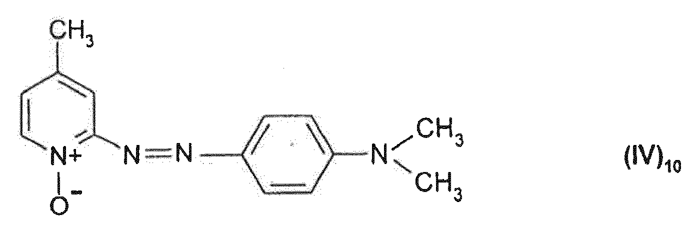 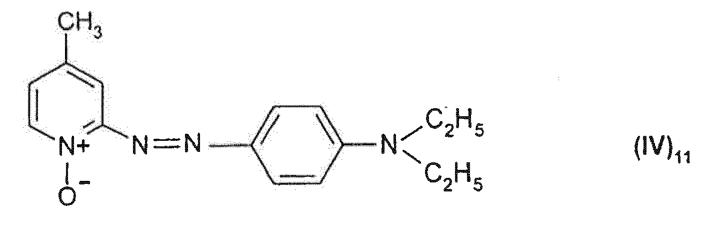 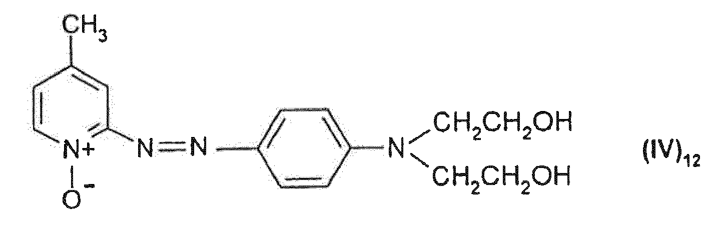 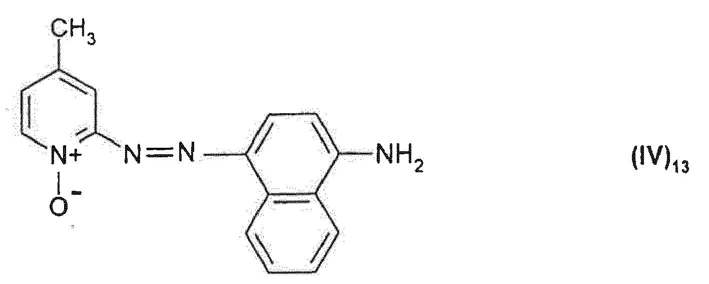 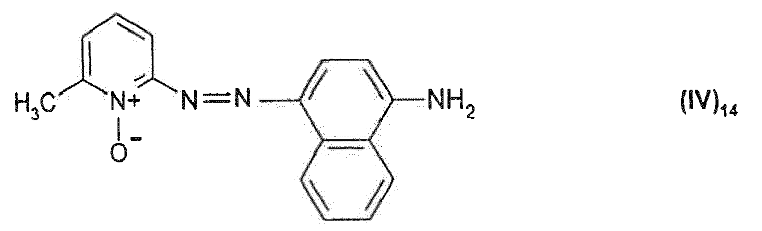 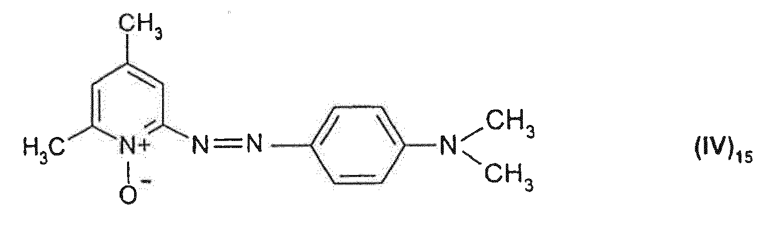 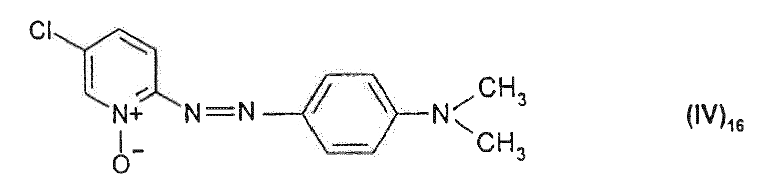 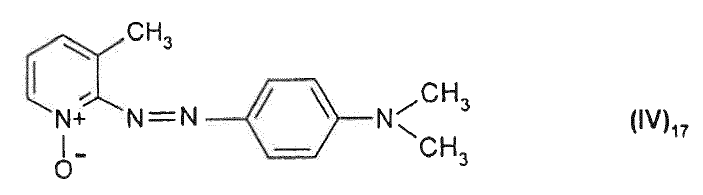 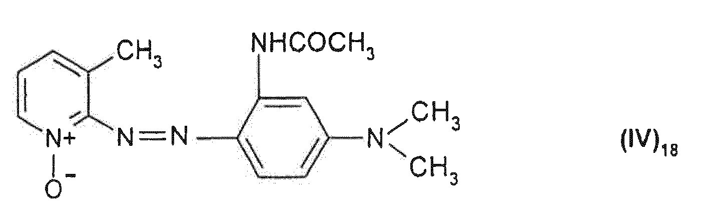 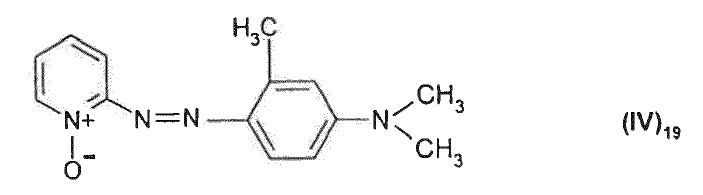 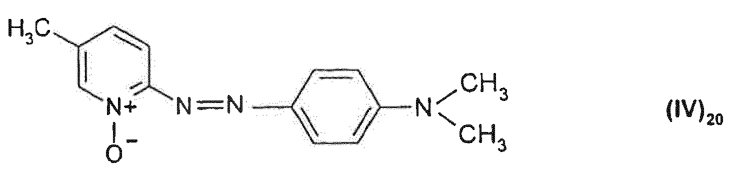 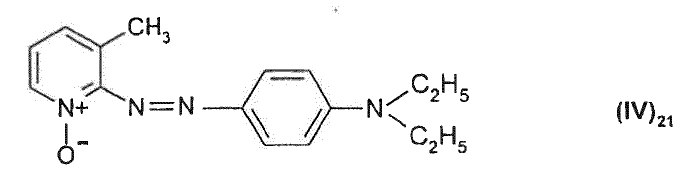 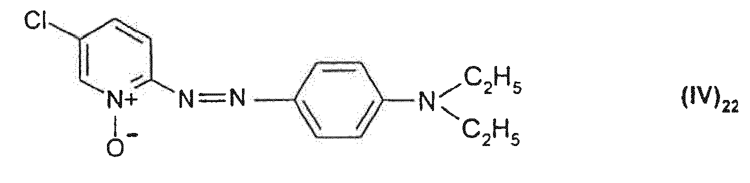 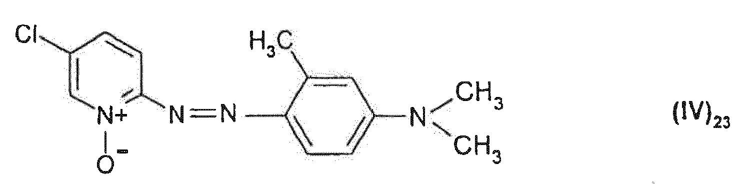 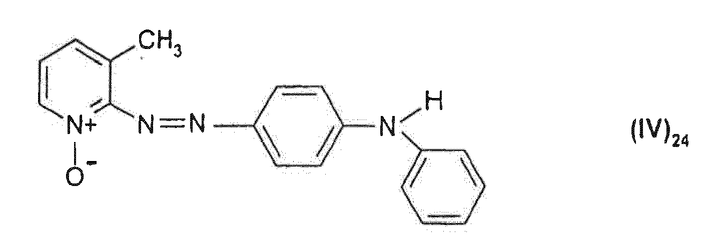 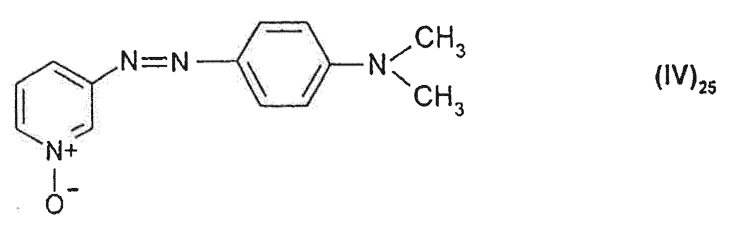 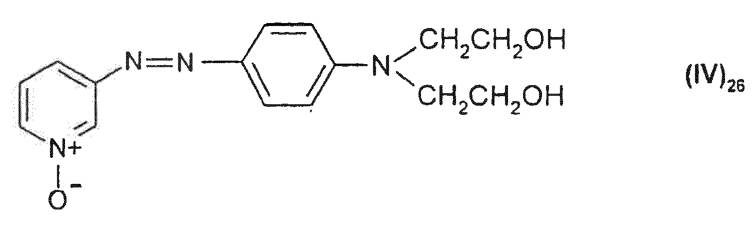 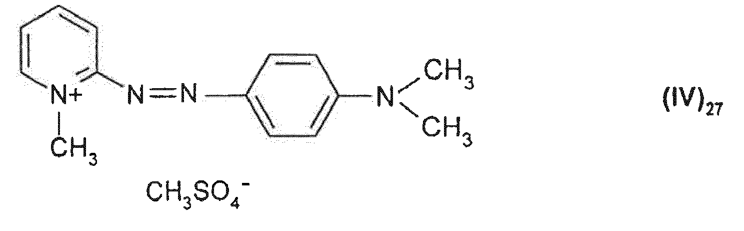 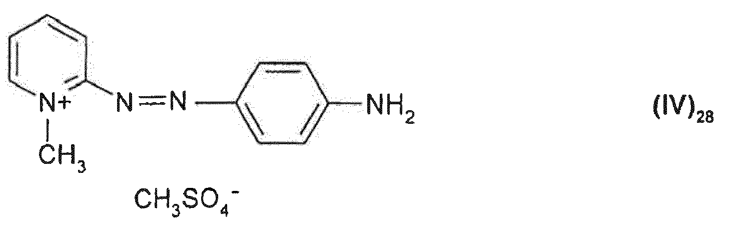 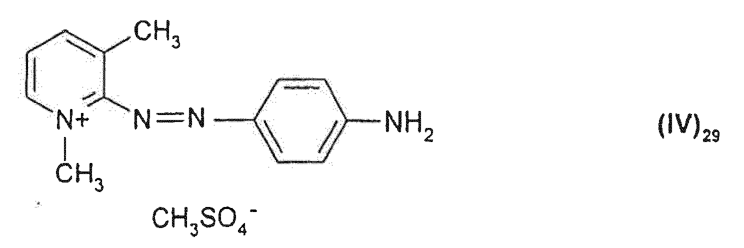 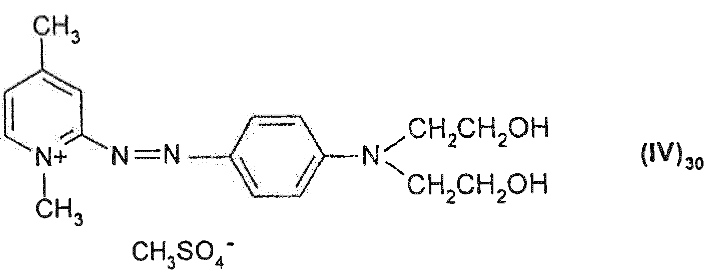 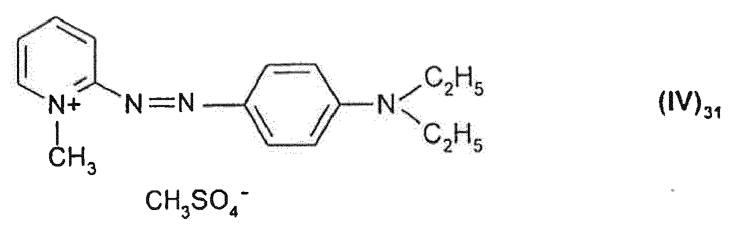 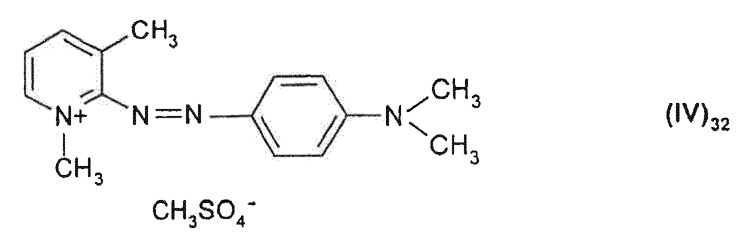 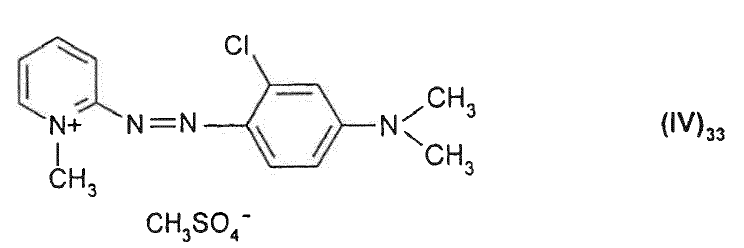 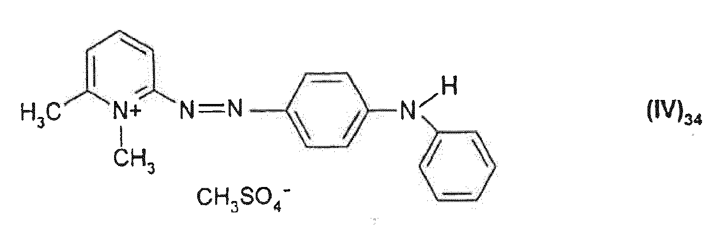 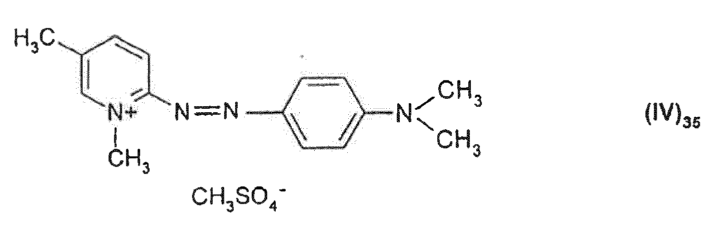 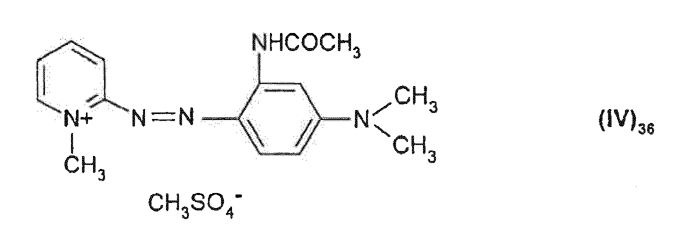 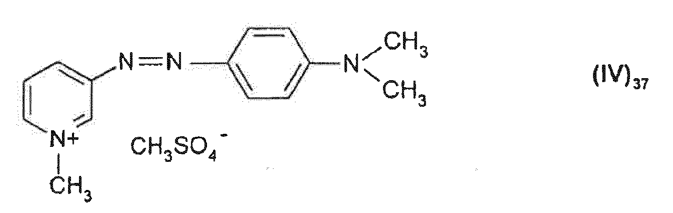 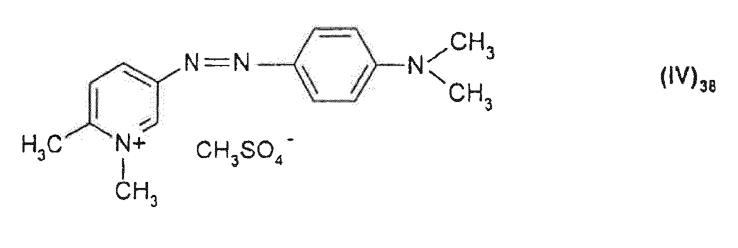 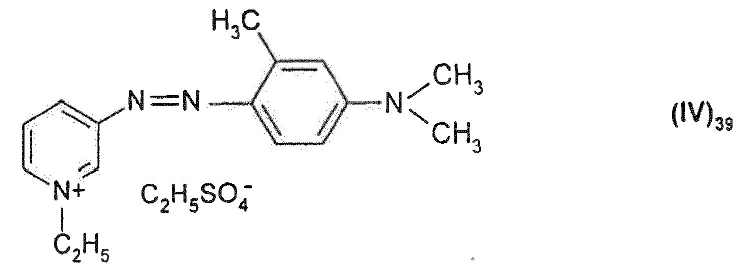 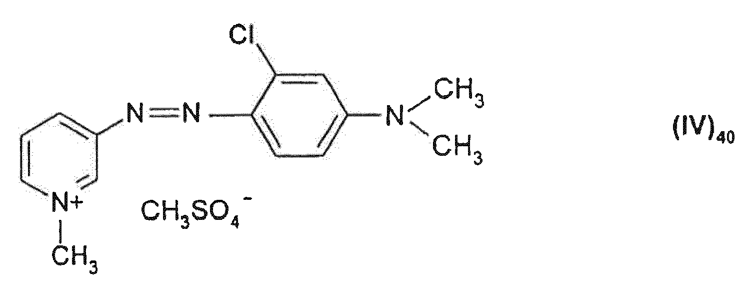 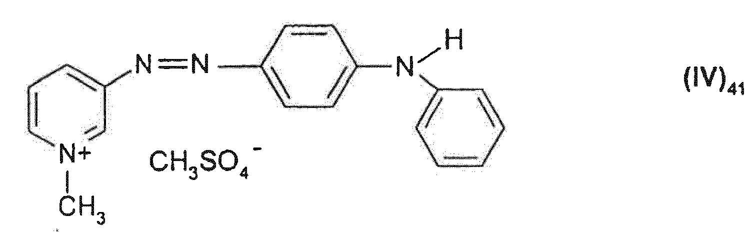 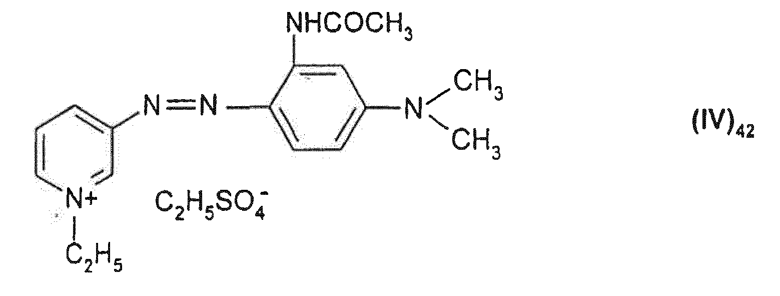 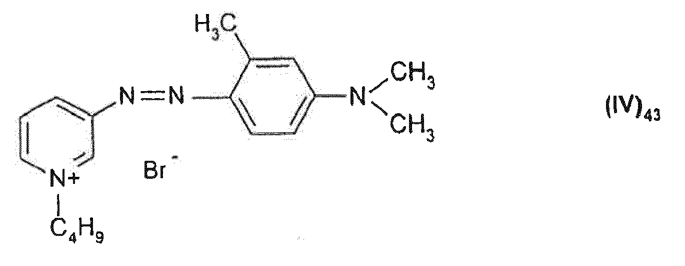 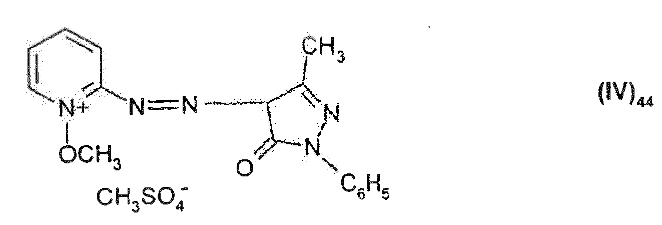 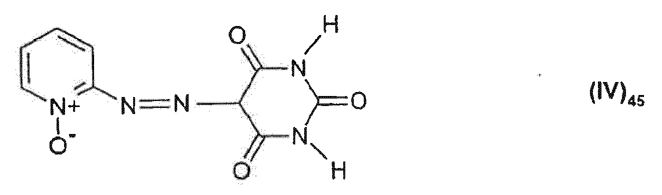 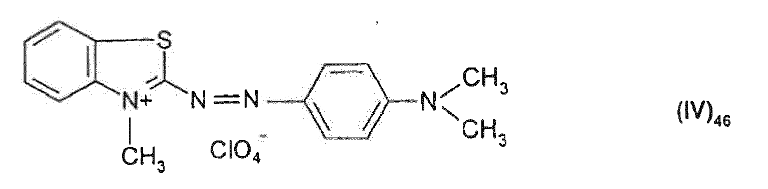 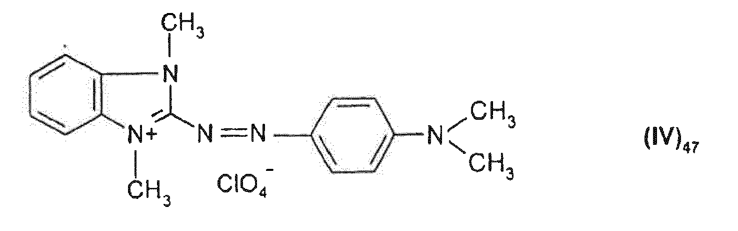 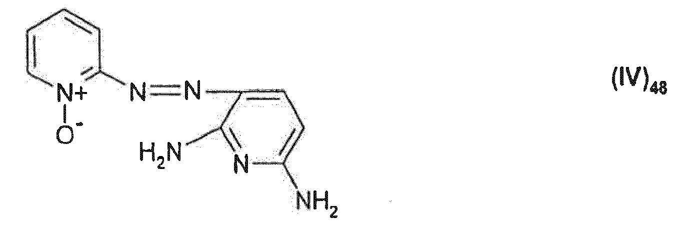 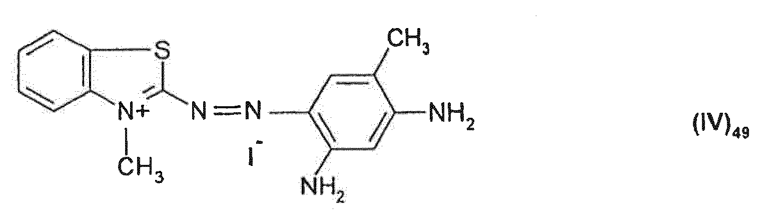 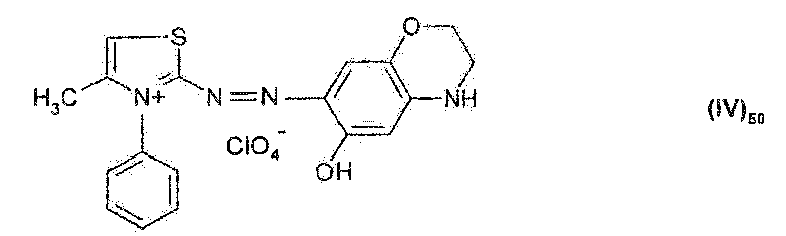 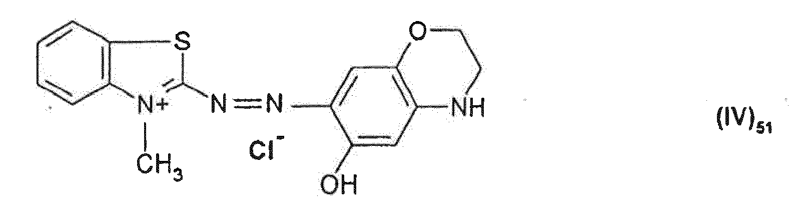 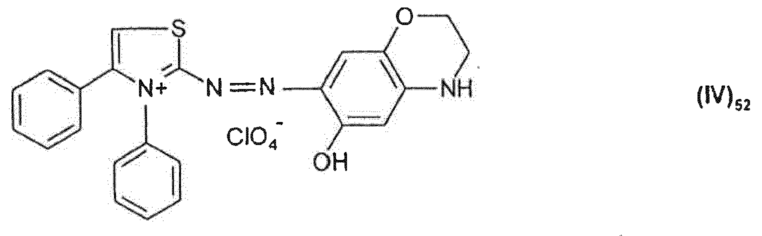 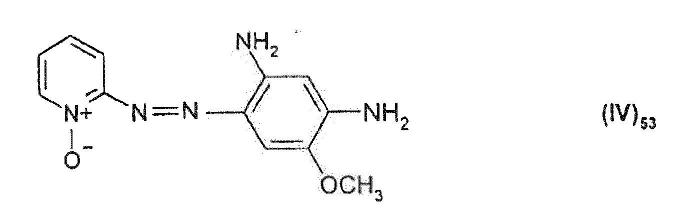 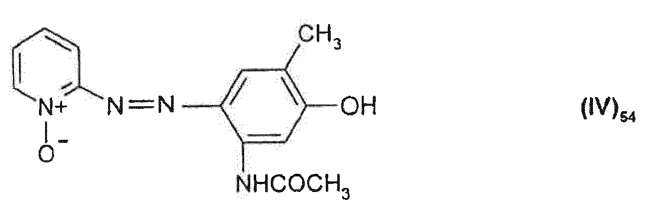 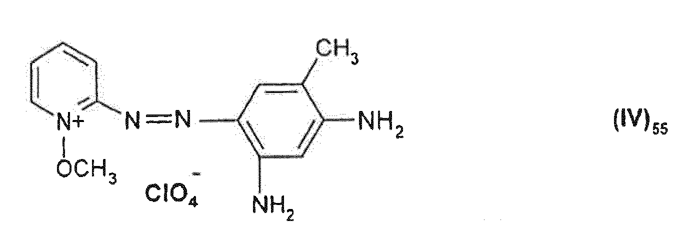 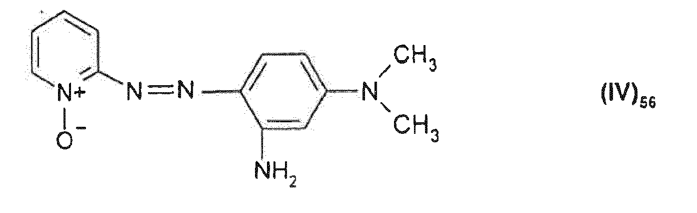 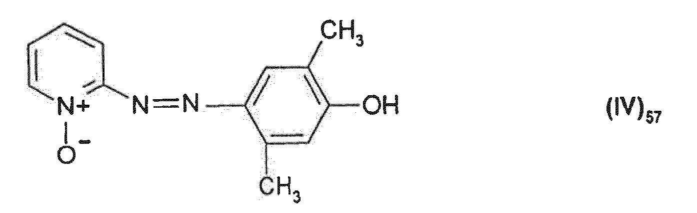 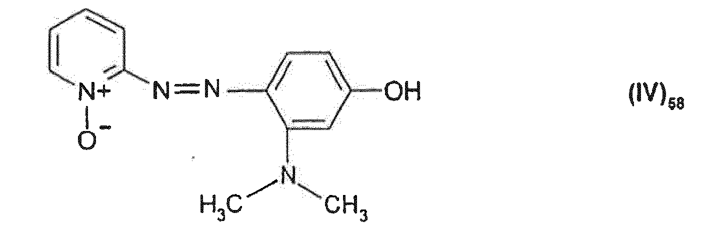 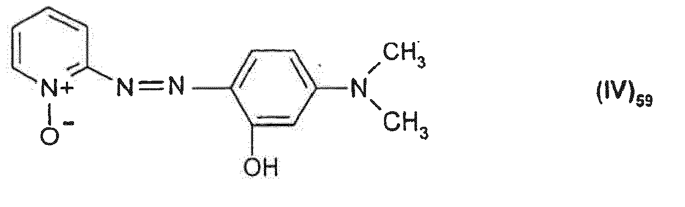 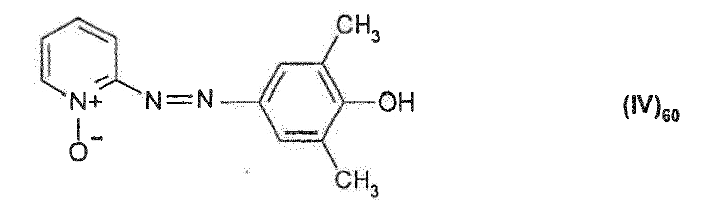 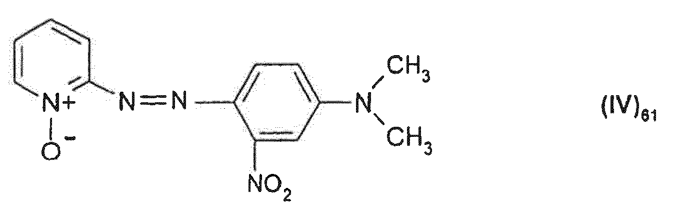 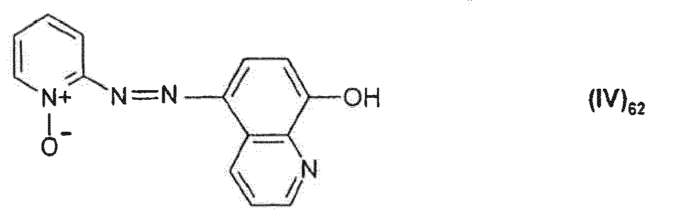 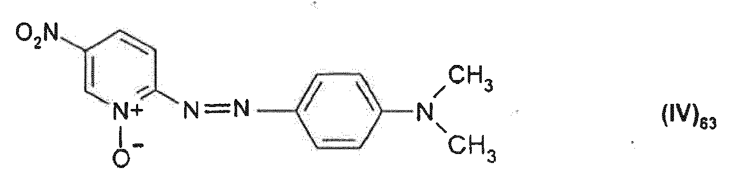 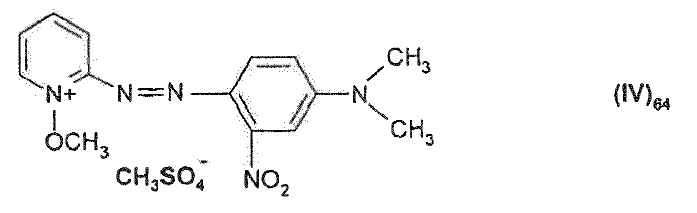 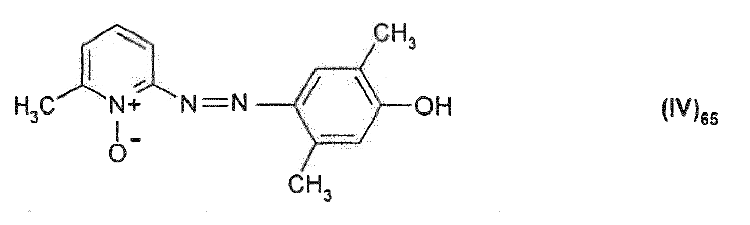 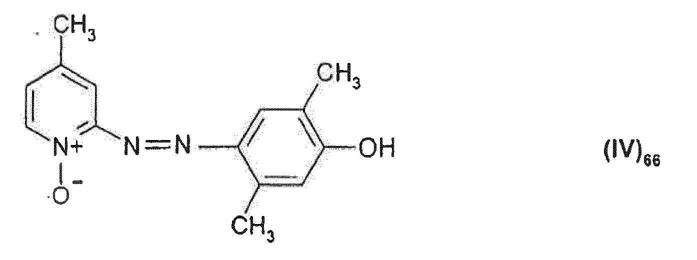 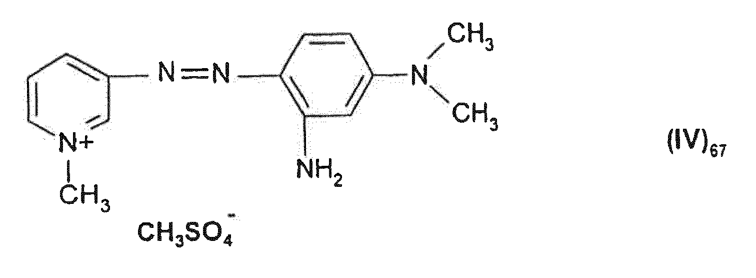 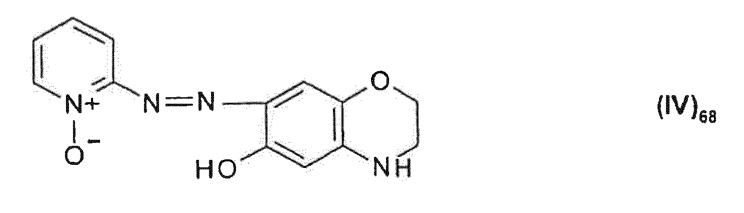 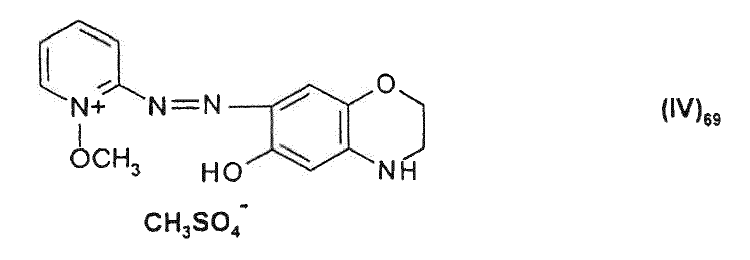 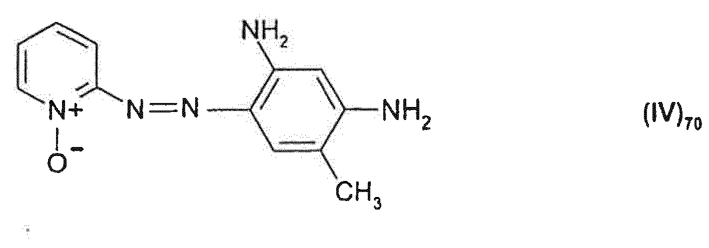 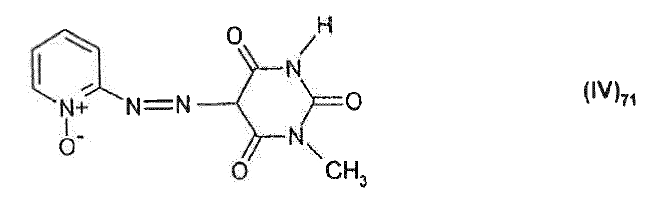 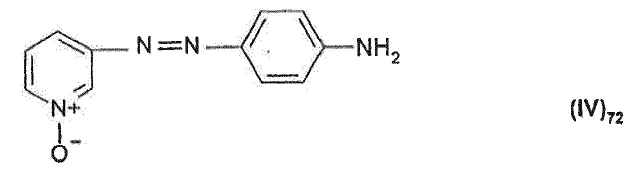 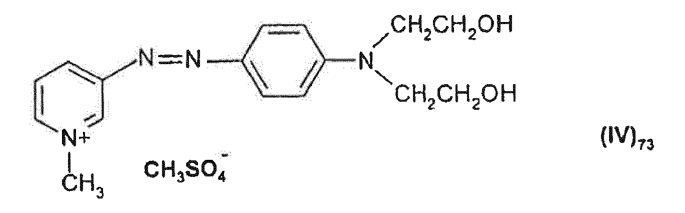 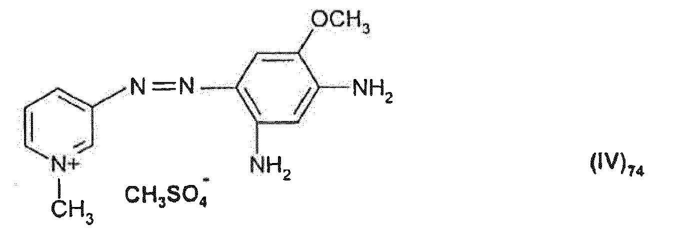 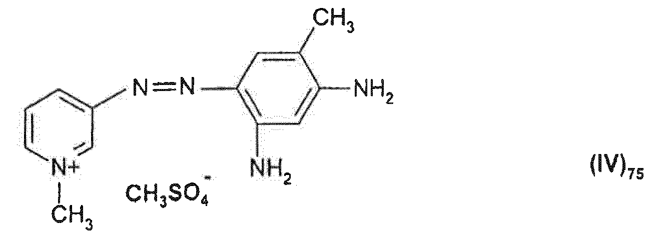 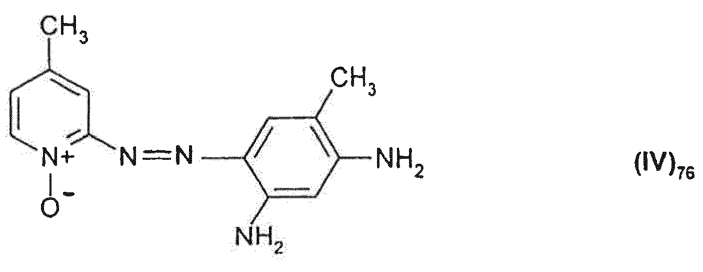 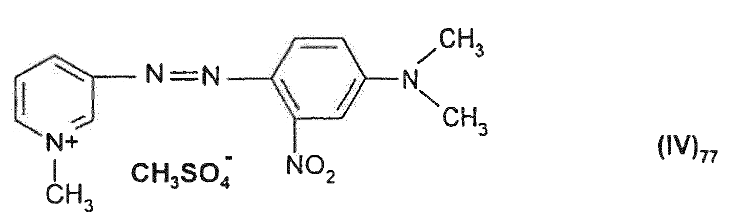 the said composition being **characterized in that** it contains, in addition,
**(ii)** at least one anionic surfactant chosen from the group comprising:
**(ii)**₁ - acyl isethionates;
**(ii)**₂ - acyl taurates;
**(ii)**₁₀ - mixtures thereof.

2. Composition according to Claim 1, **characterized in that** the cationic direct dyes correspond to the structures (I1), (I2), (I14), and (I31).

3. Composition according to Claim 1, **characterized in that** the cationic direct dyes of formula (III) are chosen from the compounds corresponding to the structures (III4), (III5) and (III13).

4. Composition according to any one of the preceding claims, **characterized in that** the cationic direct dye(s) of formulae according to Claim 1 represent from 0.001 to 10% by weight of the total weight of the composition.

5. Composition according to Claim 4, **characterized in that** the cationic direct dye(s) of formulae according to Claim 1 represent from 0.005 to 5% by weight of the total weight of the composition.

6. Composition according to any one of the preceding claims, **characterized in that** the anionic surfactant (ii) of the acyl isethionate **(ii)**₁ and acyl taurate **(ii)**₂ type corresponds to the general formula:
R¹-CH₂-CH₂-SO₃⁻ M⁺ **(V)**
in which:
R¹ denotes an R²COO group or an R²CONR³ group with R² denoting a saturated or unsaturated, linear or branched C₈-C₃₀ aliphatic group and R³ denoting a hydrogen atom or a C₁-C₄ alkyl radical, and
where M denotes H, ammonium, Na or K or an organic amine residue.

7. Composition according to any one of the preceding claims, **characterized in that** the anionic surfactant (s) represent from 0.05 to 30% by weight of the total weight of the composition.

8. Composition according to Claim 7, **characterized in that** the anionic surfactant(s) represent from 0.1 to 15% by weight of the total weight of the composition.

9. Composition according to any one of the preceding claims, **characterized in that** the appropriate dyeing medium (or carrier) consists of water or of a mixture of water and of at least one organic solvent.

10. Composition according to any one of the preceding claims, **characterized in that** it has a pH of between 2 and 11, and preferably between 5 and 10.

11. Composition according to any one of the preceding claims, **characterized in that** it is intended for oxidation dyeing and **in that** it contains one or more oxidation bases chosen from the paraphenylenediamines, the bis-phenylalkylenediamines, the para-aminophenols, the ortho-aminophenols and the heterocyclic bases.

12. Composition according to Claim 11, **characterized in that** the oxidation base(s) represent 0.0005 to 12% by weight of the total weight of the dyeing composition.

13. Composition according to Claim 12, **characterized in that** the oxidation base(s) represent 0.005 to 6% by weight of the total weight of the dyeing composition.

14. Composition according to any one of Claims 11 to 13, **characterized in that** it contains one or more couplers chosen from the meta-phenylenediamines, the meta-aminophenols, the meta-diphenols and the heterocyclic couplers.

15. Composition according to Claim 14, **characterized in that** the coupler(s) represent from 0.0001 to 10% by weight of the total weight of the dyeing composition.

16. Composition according to Claim 15, **characterized in that** the coupler(s) represent from 0.005 to 5% by weight of the total weight of the dyeing composition.

17. Composition according to any one of the preceding claims, **characterized in that** it is intended for direct lightening dyeing or oxidation dyeing and **in that** it then contains at least one oxidizing agent.

18. Method of dyeing keratinous fibres and in particular human keratinous fibres such as hair, **characterized in that** at least one dyeing composition as defined in any one of Claims 1 to 17 is applied to the fibres for a sufficient time to develop the desired colour, after which they are rinsed, optionally washed with shampoo, rinsed again and dried.

19. Method of dyeing keratinous fibres and in particular human keratinous fibres such as hair, **characterized in that** at least one dyeing composition as defined in any one of Claims 1 to 17 is applied to the fibres for a sufficient time to develop the desired colour, with no final rinsing.

20. Method of dyeing keratinous fibres and in particular human keratinous fibres such as hair, **characterized in that** it comprises a preliminary stage consisting of storing in a separate form, on the one hand, a composition (A1) comprising, in an appropriate dyeing medium, at least one cationic direct dye (i) as defined in the preceding claims and at least one oxidation base and, on the other hand, a composition (B1) containing, in an appropriate dyeing medium, at least one oxidizing agent, and then mixing them at the time of use before applying this mixture to the keratinous fibres, the composition (A1) or the composition (B1) containing the anionic surfactant (ii) as defined in the preceding claims.

21. Method of dyeing keratinous fibres and in particular human keratinous fibres such as hair, **characterized in that** it comprises a preliminary stage consisting of storing in a separate form, on the one hand, a composition (A2) comprising, in an appropriate dyeing medium, at least one cationic direct dye (i) as defined in the preceding claims and, on the other hand, a composition (B2) containing, in an appropriate dyeing medium, at least one oxidizing agent, and then mixing them at the time of use before applying this mixture to the keratinous fibres, the composition (A2) or the composition (B2) containing the anionic surfactant (ii) as defined in the preceding claims.

22. Multicompartment device or multicompartment dyeing "kit", **characterized in that** a first compartment contains composition (A1) or (A2) as defined in Claim 20 or 21 and a second compartment contains composition (B1) or (B2) as defined in Claim 20 or 21.
